# EUROPEAN PATENT APPLICATION

(11) **EP 4 470 533 A1**
(43) Date of publication of application: **04.12.2024**
(21) Application number: 23747014.1
(22) Date of filing: 26.01.2023
(51) Int. Cl.: A61K 31/165

(54) **NOVEL B0AT1 INHIBITOR**

(30) Priority: 27.01.2022 JP 2022011030
(71) Applicant: Mitsubishi Tanabe Pharma Corporation, Osaka-shi, Osaka 541-8505 (JP)
(72) Inventor: TODOROKI, Hidenori, Osaka-shi, Osaka 541-8505 (JP); SAITO, Takafumi, Osaka-shi, Osaka 541-8505 (JP); SHIMOOKA, Kazunari, Osaka-shi, Osaka 541-8505 (JP); YAMADA, Takahiro, Osaka-shi, Osaka 541-8505 (JP); FUKUNAGA, Kenji, Osaka-shi, Osaka 541-8505 (JP); KANNO, Rentaro, Osaka-shi, Osaka 541-8505 (JP); IMAZU, Takuya, Osaka-shi, Osaka 541-8505 (JP)
(74) Representative: Ter Meer Steinmeister & Partner
(86) International application number: PCT/JP2023/002387
(87) International publication number: WO 2023/145804

(57) **Abstract**

The present invention aims to provide a novel B0AT1 inhibitor. A compound represented by the following formula (I) : 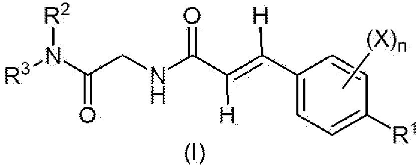 wherein each symbol is as defined in the SPECIFICATION, or a salt thereof. Also, the present invention provides a B0AT1 inhibitor containing the aforementioned compound, and a drug containing the aforementioned compound for the prophylaxis and/or treatment of amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric academia, and the like.

## Description

### [Technical Field]

The present invention relates to a novel cinnamoylglycinamide compound having an inhibitory action against neutral amino acid transporter B0AT1 and useful for the prophylaxis and/or treatment of amino acid metabolism disorders in which BOAT1 is involved.

### [Background Art]

SLC6A19, the causative gene for Hartnup disease, is localized to 5p15.33 and consists of 12 exons. Its gene product, the neutral amino acid transporter B0AT1, consists of 634 amino acids and has 12 transmembrane sites (Non-Patent Documents 1, 2). B0AT1 is a major transporter of neutral amino acids in the small intestine and kidney and is responsible for the absorption of glycine, leucine, phenylalanine, etc. in the small intestine and reabsorption thereof in the kidney (Non-Patent Documents 3 - 5). B0AT1 knockout mice are known to have elevated urinary amino acid levels (Non-Patent Document 6), and clinically, B0AT1 dysfunction also results in severe neutral amino aciduria, known as Hartnup disease (Non-Patent Document 7). Therefore, it has been thought that inhibition of B0AT1 might improve (alleviate) various diseases or conditions involving neutral amino acids, which are transport substrates.

In addition, specific examples of the therapeutic effects on diseases by inhibiting B0AT1 function have also been reported. For example, the pathology of model mouse of phenylketonuria is improved by causing congenital deficiency in B0AT1 or by inducing acquired inhibition of expression through administration of a nucleic acid compound that binds to mRNA (Non-Patent Document 4).

With this background, compounds that inhibit B0AT1 have attracted attention as drugs for the prophylaxis and/or treatment of various diseases or conditions involving neutral amino acids as transport substrates, specifically, amino acid metabolism disorders.

Examples of the amino acid metabolism disorder include designated intractable diseases such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric acidemia and the like. As a treatment method therefor, continuous dietary therapy is mainly known. In addition to dietary therapy, administration of drugs that increase coenzymes and enzymes, and treatment methods based on injection of enzymes are also known, but none of these are fundamental treatments (e.g., Non-Patent Document 8, etc.).

Hence, there is a need to develop agents to prevent and/or treat diseases, specifically, amino acid metabolism disorders that can be alleviated by normalizing neutral amino acid metabolism through inhibition of B0AT1.

So far, as compounds with B0AT1 inhibitory activity are known, for example, nucleic acids having sequences that partially or completely match the RNA of B0AT1 (Non-Patent Document 4), nimesulide and its derivatives (Non-Patent Documents 9 and 10), which are existing marketed products, synromide (Non-Patent Document 11), which is a publicly known compound, and basic compounds found in library screening (Non-Patent Documents 12, 13), and the like have been known. However, it has never been reported that the cinnamoylglycinamide compound of the present invention exhibits B0AT1 inhibitory activity.

### [Document List]

### [Non-Patent Document]

[Non-Patent Document 1] Nat. Genet., 2004; 36: p.999-1002
[Non-Patent Document 2] Nat. Genet., 2004; 36: p.1003-1007
[Non-Patent Document 3] The Journal of Biological Chemistry 2004; 279: p.24467-24476
[Non-Patent Document 4] JCI Insight., 2018; 3(14): p.e121762
[Non-Patent Document 5] IUBMB Life, 2009; 61(6): p.591-599
[Non-Patent Document 6] MOLECULAR METABOLISM, 2015: p.406-417
[Non-Patent Document 7] Biochem. J., 2005; 389: p.745-751
[Non-Patent Document 8] Japan Intractable Diseases Information Center, phenylketonuria (designated intractable diseases 240) (http://www.nanbyou.or.jp/entry/4747)
[Non-Patent Document 9] Biochemical Phamracology, 2014; 89: p.422-430
[Non-Patent Document 10] Bioorganic and Medicinal chemistry letters, 2021; 53: p.128421
[Non-Patent Document 11] SLAS Discovery, 2019; 24(2): p.111-120 [Non-Patent Document 12] British Journal of Pharmacology, 2017; 174: p.468-482
[Non-Patent Document 13] Frontiers in Pharmacology, 2020; 11: p.140

### [Summary of Invention]

### [Technical Problem]

The present invention aims to provide a medicament that exhibits a superior B0AT1 inhibitory action and can prevent and/or treat diseases whose symptoms can be alleviated by said action, specifically, amino acid metabolism disorders.

### [Solution to Problem]

The present inventors have conducted intensive studies in an attempt to solve the above-mentioned problems and found that a compound represented by the following formula (I): 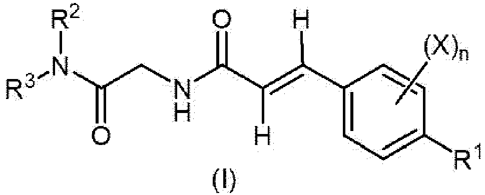 wherein
R¹ is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X in the number of n are each independently a fluorine atom or a chlorine atom;
n is an integer of 0 to 2; and
R² is a C₁₋₆ alkyl group optionally substituted by substituent(s) selected from substituent group a, and R³ is a C₁₋₆ alkyl group optionally substituted by substituent(s) selected from substituent group a or a C₃₋₆ cycloalkyl group optionally substituted by substituent(s) selected from substituent group b, or R² and R³ are bonded to each other to form, together with a nitrogen atom bonded thereto, a nitrogen-containing non-aromatic heterocyclic group optionally substituted by substituent(s) selected from substituent group b
   (substituent group a):
      halogen atom;
      hydroxy group;
      cyano group;
      carboxy group;
      C₁₋₆ alkoxy group optionally substituted by halogen atom(s);
      C₁₋₆ alkylsulfonyl group optionally substituted by halogen atom(s);
      C₁₋₆ alkyl-carbonyl group;
      C₁₋₆ alkoxy-carbonyl group;
      carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
      di-C₁₋₆ alkylamino group;
      C₃₋₈ cycloalkyl group optionally substituted by 1 to 3 substituents selected from substituent group c;
      C₆₋₁₄ aryl group optionally substituted by 1 to 3 substituents selected from substituent group c;
      nitrogen-containing aromatic heterocyclic group optionally substituted by 1 to 3 substituents selected from substituent group c; and
      non-aromatic heterocyclic group optionally substituted by 1 to 3 substituents selected from substituent group b
   (substituent group b):
      halogen atom;
      hydroxy group;
      cyano group;
      carboxy group;
      oxo group;
      thioxo group;
      amino group optionally substituted by 1 or 2 substituents selected from the group consisting of a C₁₋₆ alkyl group, a C₁₋₆ alkyl-carbonyl group, and a C₁₋₆ alkoxy-carbonyl group;
      C₁₋₆ alkyl group optionally substituted by substituent(s) selected from the group consisting of a halogen atom, a hydroxy group, and a C₁₋₆ alkoxy group;
      C₁₋₆ alkoxy group optionally substituted by halogen atom(s);
      C₁₋₆ alkylsulfonyl group optionally substituted by halogen atom(s);
      C₁₋₆ alkyl-carbonyl group optionally substituted by halogen atom(s);
      C₁₋₆ alkoxy-carbonyl group;
      carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
      aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
      trisubstituted silyl group;
      trisubstituted silyloxy group;
      C₃₋₈ cycloalkyl group optionally substituted by 1 to 3 substituents selected from substituent group c;
      C₆₋₁₄ aryl group optionally substituted by 1 to 3 substituents selected from substituent group c; and
      nitrogen-containing aromatic heterocyclic group optionally substituted by 1 to 3 substituents selected from substituent group c
   (substituent group c):
      halogen atom;
      hydroxy group;
      cyano group;
      carboxy group;
      amino group optionally substituted by 1 or 2 substituents selected from the group consisting of a C₁₋₆ alkyl group and a C₁₋₆ alkoxy-carbonyl group;
      C₁₋₆ alkyl group optionally substituted by halogen atom(s);
      C₁₋₆ alkoxy group optionally substituted by halogen atom(s);
      C₁₋₆ alkylsulfonyl group optionally substituted by halogen atom(s);
      C₁₋₆ alkyl-carbonyl group;
      C₁₋₆ alkoxy-carbonyl group;
      carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
      C₆₋₁₄ aryl group optionally substituted by 1 to 3 substituents selected from the group consisting of a halogen atom, a C₁₋₆ alkyl group, and a C₁₋₆ alkoxy group; and
      nitrogen-containing aromatic heterocyclic group optionally substituted by 1 to 3 substituents selected from the group consisting of a halogen atom, a C₁₋₆ alkyl group, and a C₁₋₆ alkoxy group
      (hereinafter sometimes abbreviated as "compound (I)"), or a pharmaceutically acceptable salt thereof has a superior inhibitory action against neutral amino acid transporter B0AT1, which resulted in the completion of the present invention.

That is, the present invention provides the following.
[1] A B0AT1 inhibitor consisting of a compound represented by the formula (I): 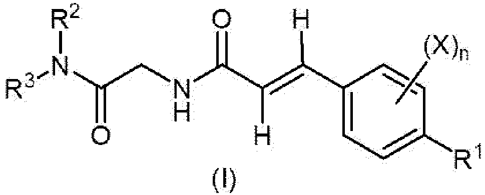 wherein
   R¹ is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
   X in the number of n are each independently a fluorine atom or a chlorine atom;
   n is an integer of 0 to 2; and
   R² is a C₁₋₆ alkyl group optionally substituted by substituent(s) selected from substituent group a, and R³ is a C₁₋₆ alkyl group optionally substituted by substituent(s) selected from substituent group a or a C₃₋₆ cycloalkyl group optionally substituted by substituent(s) selected from substituent group b, or R² and R³ are bonded to each other to form, together with a nitrogen atom bonded thereto, a nitrogen-containing non-aromatic heterocyclic group optionally substituted by substituent(s) selected from substituent group b
      (substituent group a):
         halogen atom;
         hydroxy group;
         cyano group;
         carboxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         di-C₁₋₆ alkylamino group;
         C₃₋₈ cycloalkyl group optionally substituted by 1 to 3 substituents selected from substituent group c;
         C₆₋₁₄ aryl group optionally substituted by 1 to 3 substituents selected from substituent group c;
         nitrogen-containing aromatic heterocyclic group optionally substituted by 1 to 3 substituents selected from substituent group c; and
         non-aromatic heterocyclic group optionally substituted by 1 to 3 substituents selected from substituent group b
      (substituent group b):
         halogen atom;
         hydroxy group;
         cyano group;
         carboxy group;
         oxo group;
         thioxo group;
         amino group optionally substituted by 1 or 2 substituents selected from the group consisting of a C₁₋₆ alkyl group, a C₁₋₆ alkyl-carbonyl group, and a C₁₋₆ alkoxy-carbonyl group;
         C₁₋₆ alkyl group optionally substituted by substituent(s) selected from the group consisting of a halogen atom, a hydroxy group, and a C₁₋₆ alkoxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group optionally substituted by halogen atom(s);
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
         trisubstituted silyl group;
         trisubstituted silyloxy group;
         C₃₋₈ cycloalkyl group optionally substituted by 1 to 3 substituents selected from substituent group c;
         C₆₋₁₄ aryl group optionally substituted by 1 to 3 substituents selected from substituent group c; and
         nitrogen-containing aromatic heterocyclic group optionally substituted by 1 to 3 substituents selected from substituent group c
      (substituent group c):
         halogen atom;
         hydroxy group;
         cyano group;
         carboxy group;
         amino group optionally substituted by 1 or 2 substituents selected from the group consisting of a C₁₋₆ alkyl group and a C₁₋₆ alkoxy-carbonyl group;
         C₁₋₆ alkyl group optionally substituted by halogen atom(s);
         C₁₋₆ alkoxy group optionally substituted by halogen atom(s);
         C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         C₆₋₁₄ aryl group optionally substituted by 1 to 3 substituents selected from the group consisting of a halogen atom, a C₁₋₆ alkyl group, and a C₁₋₆ alkoxy group; and
         nitrogen-containing aromatic heterocyclic group optionally substituted by 1 to 3 substituents selected from the group consisting of a halogen atom, a C₁₋₆ alkyl group, and a C₁₋₆ alkoxy group,
         or a pharmaceutically acceptable salt thereof.
[2] The B0AT1 inhibitor of the above-mentioned [1], wherein, in the formula (I),
   R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group, and
   n is 0.
[3] The B0AT1 inhibitor of the above-mentioned [1] or [2], wherein, in the formula (I), R¹ is a halo C₁₋₄ alkyl group.
[4] The B0AT1 inhibitor of any of the above-mentioned [1] to [3], wherein, in the formula (I),
   R² and R³ are bonded to each other to form, together with a nitrogen atom bonded thereto, a 3- to 10-membered monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b.
[5] The B0AT1 inhibitor of any of the above-mentioned [1] to [3], wherein, in the formula (I), R² and R³ are bonded to each other to form, together with a nitrogen atom bonded thereto, a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b.
[6] The B0AT1 inhibitor of any of the above-mentioned [1] to [3], wherein, in the formula (I),
   R² is a C₁₋₄ alkyl group substituted by 1 to 3 substituents selected from the aforementioned substituent group a, and R³ is a C₁₋₄ alkyl group optionally substituted by 1 to 3 substituents selected from the aforementioned substituent group a.
[7] The B0AT1 inhibitor of any of the above-mentioned [1] to [3], wherein, in the formula (I),
   R² and R³ are bonded to each other to form, together with a nitrogen atom bonded thereto, a group represented by the following formula:

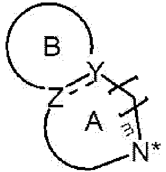

   wherein Y and Z are each independently a carbon atom or a nitrogen atom;

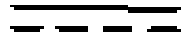

   is a single bond or a double bond; ring A is a 5- to 8-membered non-aromatic heterocycle; ring B is a 5- or 6-membered, non-aromatic heterocycle or aromatic heterocycle, or a benzene ring; m is an integer of 0 to 3; and * is a binding site with the carbonyl group.
[8] A pharmaceutical composition comprising the B0AT1 inhibitor of any of the above-mentioned [1] to [7], and a pharmaceutically acceptable carrier, for preventing and/or treating diseases whose symptoms can be alleviated by a B0AT1 inhibitory action.
[9] The pharmaceutical composition of the above-mentioned [8], wherein the disease whose symptoms can be alleviated by the B0AT1 inhibitory action is an amino acid metabolism disorder.
[10] The pharmaceutical composition of the above-mentioned [9], wherein the amino acid metabolism disorder is phenylketonuria, hypertyrosinemia (types 1-3), hypermethioninemia, maple syrup urine disease, homocystinuria, nonketotic hyperglycinemia, propionic acidemia, methylmalonic academia, or isovaleric acidemia.
[11] The pharmaceutical composition of the above-mentioned [9], wherein the amino acid metabolism disorder is phenylketonuria.
[12] A compound represented by the formula (I'):

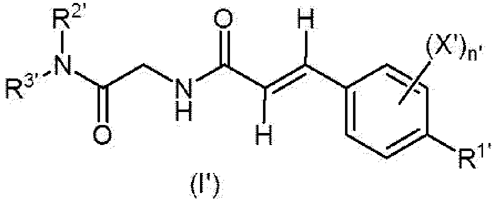

   wherein
   R¹' is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group; X' in the number of n' are each independently a fluorine atom or a chlorine atom;
   n' is an integer of 0 to 2; and
   R²' is a C₁₋₄ alkyl group substituted by substituent(s) selected from substituent group a, and
   R³' is a C₁₋₄ alkyl group optionally substituted by substituent(s) selected from substituent group a or a C₃₋₆ cycloalkyl group optionally substituted by substituent(s) selected from substituent group b, or
   R²' and R³' are bonded to each other to form, together with a nitrogen atom bonded thereto, a nitrogen-containing non-aromatic heterocyclic group optionally substituted by substituent(s) selected from substituent group b
      (substituent group a):
         halogen atom;
         hydroxy group;
         cyano group;
         carboxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from
         the group consisting of a hydroxy group, a di-C₁₋₆ alkylamino group, and a C₁₋₆ alkoxy group;
         di-C₁₋₆ alkylamino group;
         C₃₋₈ cycloalkyl group optionally substituted by 1 to 3 substituents selected from substituent group c;
         C₆₋₁₄ aryl group optionally substituted by 1 to 3 substituents selected from substituent group c;
         nitrogen-containing aromatic heterocyclic group optionally substituted by 1 to 3 substituents selected from substituent group c; and
         non-aromatic heterocyclic group optionally substituted by 1 to 3 substituents selected from substituent group b
      (substituent group b):
         halogen atom;
         hydroxy group;
         cyano group;
         carboxy group;
         oxo group;
         thioxo group;
         amino group optionally substituted by 1 or 2 substituents selected from the group consisting of a C₁₋₆ alkyl group, a C₁₋₆ alkyl-carbonyl group, and a C₁₋₆ alkoxy-carbonyl group;
         C₁₋₆ alkyl group optionally substituted by substituent(s) selected from the group consisting of a halogen atom, a hydroxy group, and a C₁₋₆ alkoxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group optionally substituted by halogen atom(s);
         C₁₋₆ alkoxy-carbonyl group;
         carbamoyl group optionally substituted by 1 or 2 C₁₋₆ alkyl groups optionally substituted by substituent(s) selected from
         the group consisting of a hydroxy group, a di-C₁₋₆ alkylamino group, and a C₁₋₆ alkoxy group;
         aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
         trisubstituted silyl group;
         trisubstituted silyloxy group;
         C₃₋₈ cycloalkyl group optionally substituted by 1 to 3 substituents selected from substituent group c;
         C₆₋₁₄ aryl group optionally substituted by 1 to 3 substituents selected from substituent group c; and
         nitrogen-containing aromatic heterocyclic group optionally substituted by 1 to 3 substituents selected from substituent group c
      (substituent group c):
         halogen atom;
         hydroxy group;
         cyano group;
         carboxy group;
         amino group optionally substituted by 1 or 2 substituents selected from the group consisting of a C₁₋₆ alkyl group and a C₁₋₆ alkoxy-carbonyl group;
         C₁₋₆ alkyl group optionally substituted by halogen atom(s);
         C₁₋₆ alkoxy group optionally substituted by halogen atom(s);
         C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         C₆₋₁₄ aryl group optionally substituted by 1 to 3 substituents selected from the group consisting of a halogen atom, a C₁₋₆ alkyl group, and a C₁₋₆ alkoxy group; and
         nitrogen-containing aromatic heterocyclic group optionally substituted by 1 to 3 substituents selected from the group consisting of a halogen atom, a C₁₋₆ alkyl group, and a C₁₋₆ alkoxy group.]
         (hereinafter sometimes abbreviated as "compound (I')") or a salt thereof, excluding compounds represented by the following formulas: 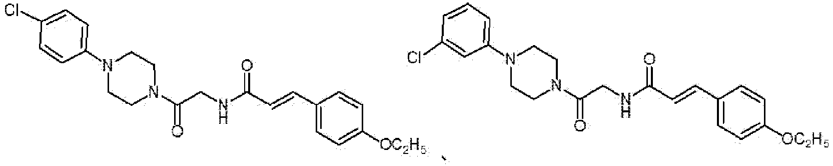 and 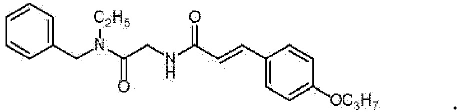
[13] The compound of the above-mentioned [12], wherein, in the formula (I'),
   R¹' is a halo C₁₋₄ alkyl group, and
   n' is 0, or a salt thereof.
[14] The compound of the above-mentioned [12] or [13], wherein, in the formula (I'),
   R²' and R³' are bonded to each other to form, together with a nitrogen atom bonded thereto, a 3- to 10-membered monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b, or a salt thereof.
[15] The compound of the above-mentioned [12] or [13], wherein, in the formula (I'), R²' and R³' are bonded to each other to form, together with a nitrogen atom bonded thereto, a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b, or a salt thereof.
[16] A compound represented by the formula (I"):

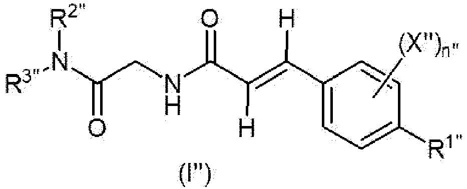

   wherein
   R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
   X'' in the number of n'' are each independently a fluorine atom or a chlorine atom;
   n'' is an integer of 0 to 2; and
   R²" is a C₁₋₄ alkyl group substituted by a group selected from the group consisting of
      (i) a 5- or 6-membered monocyclic nitrogen-containing aromatic heterocyclic group optionally substituted by substituent(s) selected from substituent group c, and
      (ii) a 5- or 6-membered monocyclic nitrogen-containing non-aromatic heterocyclic group optionally substituted by substituent(s) selected from substituent group b, and optionally further substituted by substituent(s) selected from substituent group a, and R³'' is a C₁₋₄ alkyl group substituted by substituent(s) selected from substituent group a, or
   R²" and R³'' are bonded to each other to form, together with a nitrogen atom bonded thereto, a fused nitrogen-containing non-aromatic heterocyclic group (excluding a tetrahydroquinolyl group and a tetrahydroisoquinolyl group) optionally substituted by substituent(s) selected from substituent group b
      (substituent group a):
         halogen atom;
         hydroxy group;
         cyano group;
         carboxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         di-C₁₋₆ alkylamino group;
         C₃₋₈ cycloalkyl group optionally substituted by 1 to 3 substituents selected from substituent group c;
         C₆₋₁₄ aryl group optionally substituted by 1 to 3 substituents selected from substituent group c;
         nitrogen-containing aromatic heterocyclic group optionally substituted by 1 to 3 substituents selected from substituent group c; and
         non-aromatic heterocyclic group optionally substituted by 1 to 3 substituents selected from substituent group b
      (substituent group b):
         halogen atom;
         hydroxy group;
         cyano group;
         carboxy group;
         oxo group;
         thioxo group;
         amino group optionally substituted by 1 or 2 substituents selected from the group consisting of a C₁₋₆ alkyl group, a C₁₋₆ alkyl-carbonyl group, and a C₁₋₆ alkoxy-carbonyl group;
         C₁₋₆ alkyl group optionally substituted by substituent(s) selected from the group consisting of a halogen atom, a hydroxy group, and a C₁₋₆ alkoxy group;
         C₁₋₆ alkoxy group optionally substituted by halogen atom(s);
         C₁₋₆ alkylsulfonyl group optionally substituted by halogen atom(s);
         C₁₋₆ alkyl-carbonyl group optionally substituted by halogen atom(s);
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
         trisubstituted silyl group;
         trisubstituted silyloxy group;
         C₃₋₈ cycloalkyl group optionally substituted by 1 to 3 substituents selected from substituent group c;
         C₆₋₁₄ aryl group optionally substituted by 1 to 3 substituents selected from substituent group c; and
         nitrogen-containing aromatic heterocyclic group optionally substituted by 1 to 3 substituents selected from substituent group c
      (substituent group c):
         halogen atom;
         hydroxy group;
         cyano group;
         carboxy group;
         amino group optionally substituted by 1 or 2 substituents selected from the group consisting of a C₁₋₆ alkyl group and a C₁₋₆ alkoxy-carbonyl group;
         C₁₋₆ alkyl group optionally substituted by halogen atom(s);
         C₁₋₆ alkoxy group optionally substituted by halogen atom(s); C₁₋₆ alkylsulfonyl group optionally substituted by halogen atom(s);
         C₁₋₆ alkyl-carbonyl group;
         C₁₋₆ alkoxy-carbonyl group;
         carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
         C₆₋₁₄ aryl group optionally substituted by 1 to 3 substituents selected from the group consisting of a halogen atom, a C₁₋₆ alkyl group, and a C₁₋₆ alkoxy group; and
         nitrogen-containing aromatic heterocyclic group optionally substituted by 1 to 3 substituents selected from the group consisting of a halogen atom, a C₁₋₆ alkyl group, and a C₁₋₆ alkoxy group
         (hereinafter sometimes abbreviated as "compound (I")") or a salt thereof.
[17] The compound of the above-mentioned [16], wherein, in the formula (I"),
   R²" and R³'' are bonded to each other to form, together with a nitrogen atom bonded thereto, a fused nitrogen-containing non-aromatic heterocyclic group represented by the following formula:

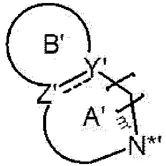

   wherein Y' and Z' are each independently a carbon atom or a nitrogen atom;

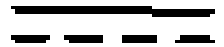

   is a single bond or a double bond; ring A' is a 5- to 8-membered non-aromatic heterocycle; ring B' is a 5- or 6-membered, non-aromatic heterocycle or aromatic heterocycle; m' is an integer of 0 to 3; and *' is a binding site with the carbonyl group,
   and optionally substituted by 1 to 3 substituents selected from the aforementioned substituent group b, or a salt thereof.
[18] A pharmaceutical composition comprising the compound of any of the above-mentioned [12] to [17] or a salt thereof, and a pharmaceutically acceptable carrier.
[19] The pharmaceutical composition of the above-mentioned [18], which is for use in the treatment and/or prophylaxis of an amino acid metabolism disorder.
[20] The pharmaceutical composition of the above-mentioned [18], which is for use in the treatment and/or prophylaxis of a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[21] The pharmaceutical composition of the above-mentioned [18], which is for use in the treatment and/or prophylaxis of phenylketonuria.
[22] The pharmaceutical composition of any of the above-mentioned [8] to [11] and [18] to [21], which is administered in combination with other drug.
[23] The pharmaceutical composition of the above-mentioned [22], wherein the other drug is a B0AT1 inhibitor.
[24] The pharmaceutical composition of the above-mentioned [22], wherein the other drug is a vitamin, a drug for alleviating various symptoms of amino acid metabolism disorders, an antidepressant, an antianxiety drug, or a therapeutic drug for ADHD.
[25] The pharmaceutical composition of any of the above-mentioned [22] to [24], wherein the pharmaceutical composition of any of the above-mentioned [8] to [11] and [18] to [21] and other drug are administered separately.
[26] The pharmaceutical composition of any of the above-mentioned [22] to [24], wherein the pharmaceutical composition of any of the above-mentioned [8] to [11] and [18] to [21], and other drug are administered simultaneously or sequentially.
[27] The pharmaceutical composition of any of the above-mentioned [22] to [26], which is for use in the treatment and/or prophylaxis of an amino acid metabolism disorder.
[28] The pharmaceutical composition of any of the above-mentioned [22] to [26], which is for use in the treatment and/or prophylaxis of a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[29] A pharmaceutical composition comprising the pharmaceutical composition of any of the above-mentioned [8] to [11] and [18] to [21] and other drug.
[30] The pharmaceutical composition of the above-mentioned [29], which is for use in the treatment and/or prophylaxis of an amino acid metabolism disorder.
[31] The pharmaceutical composition of the above-mentioned [29], which is for use in the treatment and/or prophylaxis of a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[32] A method for preventing or treating an amino acid metabolism disorder in a mammal, comprising administering an effective amount of the B0AT1 inhibitor of any of the above-mentioned [1] to [7] to the mammal.
[33] A method for preventing or treating a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia in a mammal, comprising administering an effective amount of the B0AT1 inhibitor of any of the above-mentioned [1] to [7] to the mammal.
[34] A method for preventing or treating an amino acid metabolism disorder in a mammal, comprising administering an effective amount of the compound of any of the above-mentioned [12] to [17] or a salt thereof to the mammal.
[35] A method for preventing or treating a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia, comprising administering an effective amount of the compound of any of the above-mentioned [12] to [17] or a salt thereof to the mammal.
[36] A method for preventing or treating an amino acid metabolism disorder in a mammal, comprising administering an effective amount of the pharmaceutical composition of any of the above-mentioned [8] to [11] and [18] to [21], and an effective amount of other drug simultaneously or sequentially to the mammal.
[37] A method for preventing or treating a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia in a mammal, comprising administering an effective amount of the pharmaceutical composition of any of the above-mentioned [8] to [11] and [18] to [21], and an effective amount of other drug simultaneously or sequentially to the mammal.
[38] Use of the B0AT1 inhibitor of any of the above-mentioned [1] - [7] in manufacturing a therapeutic or prophylactic agent for an amino acid metabolism disorder.
[39] Use of the B0AT1 inhibitor of any of the above-mentioned [1] - [7] in manufacturing a therapeutic or prophylactic agent for a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[40] Use of the compound of any of the above-mentioned [12] to [17] or a salt thereof in manufacturing a therapeutic or prophylactic agent for an amino acid metabolism disorder.
[41] Use of the compound of any of the above-mentioned [12] to [17] or a salt thereof in manufacturing a therapeutic or prophylactic agent for a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[42] The compound of any of the above-mentioned [12] to [17] or a salt thereof for use in the treatment and/or prophylaxis of an amino acid metabolism disorder.
[43] The compound of any of the above-mentioned [12] to [17] or a salt thereof for use in the treatment and/or prophylaxis of a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.
[44] A method for producing the compound of any of the above-mentioned [12] to [17] or a salt thereof.
[45] A prodrug of the compound of any of the above-mentioned [12] to [17] or a salt thereof.

### [Advantageous Effects of Invention]

Compound (I) of the present invention or a pharmaceutically acceptable salt thereof has a superior inhibitory activity against B0AT1. Therefore, a pharmaceutical composition containing said compound is useful for the treatment and/or prophylaxis of diseases whose symptoms can be alleviated by a B0AT1 inhibitory action. Examples of the disease include amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric acidemia, and the like. Since these amino acid metabolism disorders are designated intractable diseases that require long-term medical treatments including a very strict dietary therapy (amino acid-restricted diet) for life, the compound (I) of the present invention or a pharmaceutically acceptable salt thereof can provide a novel and effective prophylactic and/or therapeutic drug.

### [Description of Embodiments]

The definitions of the terms and symbols used in the present specification are explained below.

In the present specification, the "halogen atom" means a fluorine atom, a chlorine atom, a bromine atom, or an iodine atom.

In the present specification, the "alkyl (group)" means a linear or branched chain monovalent group with one or more carbon atoms, excluding one hydrogen atom from any carbon atom of an alkane. While the number of carbon atoms is not particularly limited, it is a C₁₋₂₀ alkyl group, preferably a C₁₋₆ alkyl group.

In the present specification, the "C₁₋₂₀ alkyl (group)" means an alkyl group having 1 to 20 carbon atoms and, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, tridecyl, eicosyl, and the like can be mentioned.

In the present specification, the "C₁₋₆ alkyl (group)" means an alkyl group having 1 to 6 carbon atoms and, for example, methyl, ethyl, propyl, isopropyl, butyl, isobutyl, sec-butyl, tert-butyl, pentyl, isopentyl, neopentyl, sec-pentyl (pentan-2-yl), 3-pentyl (pentan-3-yl), tert-pentyl (1,1-dimethylpropyl), hexyl, isohexyl, 1,1-dimethylbutyl, 2,2-dimethylbutyl, 3,3-dimethylbutyl, 2-ethylbutyl, and the like can be mentioned.

In the present specification, the "C₁₋₄ alkyl (group)" means an alkyl group having 1 to 4 carbon atoms and, for example, methyl, ethyl, propyl, isopropyl, butyl, isobutyl, sec-butyl, tert-butyl, and the like can be mentioned.

In the present specification, the "haloalkyl (group)" means the aforementioned alkyl group in which one or more (preferably, 1 to 6, more preferably, 1 to 3) hydrogen atoms are substituted by halogen. Specifically, for example, fluoromethyl, difluoromethyl, trifluoromethyl, 2-chloroethyl, 2-bromoethyl, 2-iodoethyl, 2-fluoroethyl, 2,2-difluoroethyl, 2,2,2-trifluoroethyl, pentafluoroethyl, 2,2,3,3-tetrafluoropropyl, 3,3,3-trifluoropropyl, 4,4,4-trifluorobutyl, 5,5,5-trifluoropentyl, 6,6,6-trifluorohexyl, and the like can be mentioned. Among these, "halo C₁₋₆ alkyl" is preferred, and "halo C₁₋₄ alkyl" is more preferred.

In the present specification, the "cycloalkyl (group)" means a cyclic alkyl group. While the number of carbon atoms is not particularly limited, it is preferably a C₃₋₈ cycloalkyl group.

In the present specification, the "C₃₋₈ cycloalkyl (group)" means a cyclic alkyl group having 3 to 8 carbon atoms and, for example, cyclopropyl, cyclobutyl, cyclopentyl, cyclohexyl, cycloheptyl, cyclooctyl, and the like can be mentioned. Among these, a C₃₋₆ cycloalkyl group is preferable.

In the present specification, the "C₃₋₈ cycloalkyloxy (group)" means a group resulting from the binding of the aforementioned C₃₋₈ cycloalkyl group to an oxygen atom and specifically, for example, cyclopropyloxy, cyclobutyloxy, cyclopentyloxy, cyclohexyloxy, cycloheptyloxy, cyclooctyloxy, and the like can be mentioned.

In the present specification, the "C₃₋₈ cycloalkylsulfanyl (group)" means a group resulting from the binding of the aforementioned C₃₋₈ cycloalkyl group to a sulfur atom and specifically, for example, cyclopropylsulfanyl, cyclobutylsulfanyl, cyclopentylsulfanyl, cyclohexylsulfanyl, cycloheptylsulfanyl, cyclooctylsulfanyl, and the like can be mentioned.

In the present specification, the "alkoxy (group)" means a group resulting from the binding of a linear or branched chain alkyl group to an oxygen atom. While the number of carbon atoms is not particularly limited, it is a C₁₋₂₀ alkoxy group, preferably a C₁₋₆ alkoxy group.

In the present specification, the "C₁₋₆ alkoxy (group)" means an alkoxy group having 1 to 6 carbon atoms and, for example, methoxy, ethoxy, propoxy, isopropoxy, butoxy, isobutoxy, sec-butoxy, tert-butoxy, pentyloxy, isopentyloxy, neopentyloxy, hexyloxy, and the like can be mentioned. Among these, a C₁₋₄ alkoxy group is preferred.

In the present specification, the "haloalkoxy (group)" means the aforementioned alkoxy group in which one or more hydrogen atoms are substituted by halogen. Specifically, for example, fluoromethoxy, difluoromethoxy, trifluoromethoxy, 2-chloroethoxy, 2-bromoethoxy, 2-iodoethoxy, 2-fluoroethoxy, 2,2-difluoroethoxy, 2,2,2-trifluoroethoxy, pentafluoroethoxy, 2,2,3,3-tetrafluoropropoxy, 3,3,3-trifluoropropoxy, 4,4,4-trifluorobutoxy, 5,5,5-trifluoropentyloxy, 6,6,6-trifluorohexyloxy, and the like can be mentioned. Among these, "halo C₁₋₆ alkoxy" is preferred, and "halo C₁₋₄ alkoxy" is more preferred.

In the present specification, the "alkylsulfanyl (group)" means a group resulting from the binding of a linear or branched chain alkyl group to a sulfur atom, and a C₁₋₆ alkylsulfanyl group is preferred.

In the present specification, the "C₁₋₆ alkylsulfanyl (group)" means an alkylsulfanyl group having 1 to 6 carbon atoms and, for example, methylsulfanyl, ethylsulfanyl, propylsulfanyl, isopropylsulfanyl, butylsulfanyl, isobutylsulfanyl, sec-butylsulfanyl, tert-butylsulfanyl, pentylsulfanyl, isopentylsulfanyl, neopentylsulfanyl, hexylsulfanyl, and the like can be mentioned.

In the present specification, the "haloalkylsulfanyl (group)" means the aforementioned alkylsulfanyl group in which one or more hydrogen atoms are substituted by halogen. Specifically, for example, fluoromethylsulfanyl, difluoromethylsulfanyl, trifluoromethylsulfanyl, 2-chloroethylsulfanyl, 2-bromoethylsulfanyl, 2-iodoethylsulfanyl, 2-fluoroethylsulfanyl, 2,2-difluoroethylsulfanyl, 2,2,2-trifluoroethylsulfanyl, pentafluoroethylsulfanyl, 2,2,3,3-tetrafluoropropylsulfanyl, 3,3,3-trifluoropropylsulfanyl, 4,4,4-trifluorobutylsulfanyl, 5,5,5-trifluoropentylsulfanyl, 6,6,6-trifluorohexylsulfanyl, and the like can be mentioned. Among these, "halo C₁₋₆ alkylsulfanyl" is preferred.

In the present specification, the "C₁₋₆ alkylsulfonyl (group)" means a group resulting from the binding of the aforementioned "C₁₋₆ alkyl" group to a sulfonyl group (-S(=O)₂-), that is, a linear or branched chain alkylsulfonyl group having 1 to 6 carbon atoms. As the "C₁₋₆ alkylsulfonyl (group)", methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, 1-ethylpropylsulfonyl, hexyl sulfonyl, and the like can be mentioned.

In the present specification, the "alkyl-carbonyl (group)" means a group resulting from the binding of the aforementioned alkyl group to a carbonyl group. While the number of carbon atoms is not particularly limited, it is preferably a C₁₋₆ alkyl-carbonyl group.

In the present specification, the "alkoxy-carbonyl (group)" means a group resulting from the binding of the aforementioned alkoxy group to a carbonyl group. While the number of carbon atoms is not particularly limited, it is preferably a C₁₋₆ alkoxy-carbonyl group.

In the present specification, the "carbamoyl (group) optionally substituted by 1 or 2 C₁₋₆ alkyl groups" means a group in which 1 or 2 hydrogen atom of carbamoyl group (-CONH₂) are each independently optionally substituted by the aforementioned C₁₋₆ alkyl group, and mono- or di-C₁₋₆ alkylcarbamoyl group is preferred.

In the present specification, the "aryl (group)" means an aromatic monocyclic or polycyclic (fused) hydrocarbon group, and specifically, for example, C₆₋₁₄ aryl groups such as phenyl, 1-naphthyl, 2-naphthyl, biphenylyl, 2-anthryl, fluorenyl and the like can be mentioned. Among these, a C₆₋₁₀ aryl group is preferred.

In the present specification, as the "C₆₋₁₀ aryl (group)", phenyl, 1-naphthyl, and 2-naphthyl can be mentioned. Among these, phenyl is preferred.

In the present specification, the "aryl-carbonyl (group)" means a group resulting from the binding of the aforementioned aryl group to a carbonyl group. While the number of carbon atoms is not particularly limited, it is preferably a C₆₋₁₄ aryl-carbonyl group.

In the present specification, the "C₆₋₁₄ aryloxy (group)" means a group resulting from the binding of the aforementioned C₆₋₁₄ aryl group to an oxygen atom, and specifically, for example, phenyloxy, 1-naphthyloxy, 2-naphthyloxy, biphenylyloxy, 2-anthryloxy, and the like can be mentioned. Among these, a phenyloxy group is particularly preferred.

In the present specification, the "C₆₋₁₄ arylsulfonyl (group)" means a group resulting from the binding of the aforementioned C₆₋₁₄ aryl group to a sulfur atom of a sulfonyl group (-S(=O)₂-) and, for example, phenylsulfonyl, 1-naphthylsulfonyl, 2-naphthylsulfonyl, and the like can be mentioned.

In the present specification, the "C₆₋₁₄ arylsulfonyloxy (group)" means a group resulting from the binding of the aforementioned C₆₋₁₄ aryl group to a sulfur atom of a sulfonyloxy group (-S(=O)₂-O-) and, for example, phenylsulfonyloxy, 1-naphthylsulfonyloxy, 2-naphthylsulfonyloxy, and the like can be mentioned.

In the present specification, the "C₇₋₁₈ aralkyl (group)" means a group resulting from the binding of the aforementioned C₆₋₁₄ aryl group to the aforementioned C₁₋₄ alkyl group and specifically, for example, benzyl, phenethyl, naphthylmethyl, biphenylylmethyl, and the like can be mentioned. Among these, a C₇₋₁₀ aralkyl group is preferred, and a benzyl group is particularly preferred.

In the present specification, the "C₇₋₁₈ aralkyloxy (group)" means a group resulting from the binding of the aforementioned C₇₋₁₈ aralkyl group to an oxygen atom, and specifically, for example, benzyloxy, phenethyloxy, naphthylmethyloxy, biphenylylmethyloxy, and the like can be mentioned. Among these, a benzyloxy group is particularly preferred.

In the present specification, the "acyl (group)" means a formyl group, a linear or branched chain alkyl-carbonyl group, or an aryl-carbonyl group. While the number of carbon atoms is not particularly limited, it is preferably a formyl group, a C₁₋₆ alkyl-carbonyl group, or a C₆₋₁₄ aryl-carbonyl group. Specific preferable examples of the acyl (group) include formyl, acetyl, propionyl, butyryl, isobutyryl, pentanoyl, tert-butylcarbonyl (pivaloyl), hexanoyl, heptanoyl, benzoyl, 1-naphthoyl, 2-naphthoyl, and the like.

In the present specification, the "acyloxy (group)" means a group resulting from the binding of the aforementioned acyl group to an oxygen atom, and it is preferably a C₁₋₆ alkyl-carbonyloxy group or a C₆₋₁₄ aryl-carbonyloxy group.

In the present specification, the "amino (group) optionally substituted by 1 or 2 substituents selected from the group consisting of a C₁₋₆ alkyl group and a C₁₋₆ alkoxy-carbonyl group " means an unsubstituted amino group, or a group in which 1 or 2 hydrogen atoms of the amino group is substituted by the aforementioned C₁₋₆ alkyl group and/or C₁₋₆ alkoxy-carbonyl group.

In the present specification, the "heterocycle (group)" is (i) an aromatic heterocyclic group, (ii) a non-aromatic heterocyclic group, (iii) a 6- to 10-membered bridged non-aromatic heterocyclic group, and (iv) a 6- to 12-membered spirocyclic non-aromatic heterocyclic group, each of which contains 1 to 4 hetero atoms selected from a nitrogen atom, a sulfur atom, and an oxygen atom as ring-constituting atom besides carbon atom.

In the present specification, as the "aromatic heterocyclic group", a 5- to 14-membered aromatic heterocyclic group containing 1 to 4 hetero atoms selected from a nitrogen atom, a sulfur atom, and an oxygen atom as ring-constituting atoms besides carbon atom can be mentioned.

Preferred examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and
8- to 14-membered fused aromatic heterocyclic groups (fused polycyclic (preferably bi- or tri-cyclic) aromatic heterocyclic groups) such as benzothiophenyl, benzofuranyl, benzoimidazolyl, benzoxazolyl, benzisoxazolyl, benzothiazolyl, benzisothiazolyl, benzotriazolyl, imidazopyridyl, thienopyridyl, furopyridyl, pyrrolopyridyl, pyrazolopyridyl, oxazolopyridyl, thiazolopyridyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acrydinyl, phenazinyl, phenothiazinyl, phenoxazinyl and the like.

In the present specification, as the "non-aromatic heterocyclic group", a 3- to 14-membered non-aromatic heterocyclic group containing 1 to 4 hetero atoms selected from a nitrogen atom, a sulfur atom, and an oxygen atom as ring-constituting atoms besides carbon atom can be mentioned.

Preferred examples of the "non-aromatic heterocyclic group" include 3- to 10-membered monocyclic non-aromatic heterocyclic groups (preferably 5- to 8-membered monocyclic non-aromatic heterocyclic groups) such as aziridinyl, oxiranyl, thiiranyl, azetidinyl, oxetanyl, thietanyl, tetrahydrothienyl, tetrahydrofuryl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidyl, piperazinyl, tetrahydropyridyl, dihydropyridyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl, and the like; and 9- to 14-membered fused non-aromatic heterocyclic groups (fused polycyclic (preferably bicyclic) non-aromatic heterocyclic groups) such as tetrahydrotriazolopyridyl, tetrahydrotriazolopyrazinyl, tetrahydropyrazolopyrazinyl, dihydropyrazolopyrrolyl, tetrahydropyrazolopyridyl, tetrahydroimidazolopyridyl, dihydroimidazolopyridyl, dihydroimidazolopyrrolyl tetrahydroimidazolopyrazinyl, dihydropyrimidopyrrolyl, dihydropyridopyrrolyl, tetrahydropyridopyridyl, tetrahydrooxazolopyrazinyl, tetrahydropyridopyridyl, tetrahydrotriazolodiazepinyl, octahydro-1,4-oxazinopyrazinyl, dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolidinyl, indolinyl, isoindolinyl, tetrahydrothieno[2,3-c]pyridyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolynyl, tetrahydroacridinyl, tetrahydrophenazinyl, tetrahydrothioxanthenyl, octahydroisoquinolyl, and the like.

More preferred examples of the "non-aromatic heterocyclic group" include pyrrolidinyl, piperidyl, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, 1,2,3,4-tetrahydro-2,7-naphthyridinyl, and the like. Among these, fused non-aromatic heterocyclic groups such as 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, 1,2,3,4-tetrahydro-2,7-naphthyridinyl, and the like are particularly preferred.

In the present specification, preferred examples of the "6- to 10-membered bridged non-aromatic heterocyclic group" include 3,8-diazabicyclo[3.2.1]octyl, 2,5-diazabicyclo[2.2.2]octyl, 7-azabicyclo[2.2.1]heptanyl, quinuclidinyl, and the like.

In the present specification, preferred examples of the "6- to 12-membered spirocyclic non-aromatic heterocyclic group" include 2,8-diazaspiro[4.5]decyl, 2,7-diazaspiro[3.5]nonyl, 2,6-diazaspiro[3.3]heptyl, and the like.

In the present specification, the "nitrogen-containing aromatic heterocyclic group" or "nitrogen-containing non-aromatic heterocyclic group" means the aforementioned aromatic heterocyclic group or non-aromatic heterocyclic group that has at least one nitrogen atom as a ring-constituting atom.

In the present specification, the "aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c" means a group in which one hydrogen atom of the aminosulfonyl group (-S(=O)₂-NH₂) is substituted by the aforementioned C₁₋₆ alkyl group, C₃₋₈ cycloalkyl group, or non-aromatic heterocyclic group, each of which is optionally substituted by 1 to 3 substituents selected from substituent group c.

In the present specification, the "trisubstituted silyl (group)" means a silyl group substituted by the same or different 3 substituents (e.g., C₁₋₆ alkyl group, C₆₋₁₀ aryl group, etc.). Examples thereof include trialkylsilyl groups such as trimethylsilyl group, triethylsilyl group, triisopropylsilyl group, tert-butyldimethylsilyl group, and the like (preferably, tri-C₁₋₆ alkylsilyl group), tert-butyldiphenylsilyl group, triphenylsilyl group, and the like.

In the present specification, the "trisubstituted silyloxy(group)" means a group in which a trisubstituted silyl group is bonded to an oxygen atom. Examples thereof include trialkylsilyloxy groups such as trimethylsilyloxy group, triethylsilyloxy group, triisopropylsilyloxy group, tert-butyldimethylsilyloxy group and the like (preferably, tri C₁₋₆ alkylsilyloxy group), tert-butyldiphenylsilyloxy group, triphenylsilyloxy group, and the like.

In the present specification, being "optionally substituted" means unsubstituted or substituted by a specific substituent at any substitutable position (any hydrogen atom is replaced with a substituent). The "substituent" is not particularly limited and may be a substituent selected from the group consisting of the following (substituent group a), (substituent group a'), (substituent group a''), (substituent group b), (substituent group c), and (substituent group d). When the substituent group is not particularly specified, it means being optionally substituted by one or more substituents selected from the following (substituent group d). While the number of the substituents is not particularly limited as long as it is a substitutable number, it is generally 1 to 5, preferably 1 to 3. When plural substituents are present, the respective substituents may be the same or different.
(substituent group a):
   halogen atom;
   hydroxy group;
   cyano group;
   carboxy group;
   C₁₋₆ alkoxy group optionally substituted by halogen atom(s);
   C₁₋₆ alkylsulfonyl group optionally substituted by halogen atom(s) ;
   C₁₋₆ alkyl-carbonyl group;
   C₁₋₆ alkoxy-carbonyl group;
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
   di-C₁₋₆ alkylamino group;
   C₃₋₈ cycloalkyl group optionally substituted by 1 to 3 substituents selected from substituent group c;
   C₆₋₁₄ aryl group optionally substituted by 1 to 3 substituents selected from substituent group c;
   nitrogen-containing aromatic heterocyclic group optionally substituted by 1 to 3 substituents selected from substituent group c (preferably, 5- or 6-membered nitrogen-containing aromatic heterocyclic group); and
   non-aromatic heterocyclic group optionally substituted by 1 to 3 substituents selected from substituent group b (preferably, 5- or 6-membered non-aromatic heterocyclic group).
(substituent group a'):
   hydroxy group;
   carboxy group;
   C₁₋₆ alkoxy group optionally substituted by halogen atom(s) (e.g., methoxy group, ethoxy group);
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group (e.g., methylcarbamoyl group, dimethylcarbamoyl group);
   C₁₋₆ alkylsulfonyl group optionally substituted by halogen atom(s) (e.g., methylsulfonyl group);
   5- or 6-membered nitrogen-containing aromatic heterocyclic group optionally substituted by 1 to 3 substituents selected from substituent group c (e.g., pyridazinyl group, pyrazolyl group, thiazolyl group, pyridyl group, triazolyl group, pyrimidinyl group, oxadiazolyl group, imidazolyl group); and 5- or 6-membered non-aromatic heterocyclic group optionally substituted by 1 to 3 substituents selected from substituent group b (e.g., tetrahydropyranyl group, tetrahydrofuryl group)
(substituent group a''):
   hydroxy group;
   carboxy group;
   C₁₋₆ alkoxy group optionally substituted by halogen atom(s) (e.g., methoxy group, ethoxy group);
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group (e.g., methylcarbamoyl group, dimethylcarbamoyl group); and
   C₁₋₆ alkylsulfonyl group optionally substituted by halogen atom(s) (e.g., methylsulfonyl group).
(substituent group b):
   halogen atom;
   hydroxy group;
   cyano group;
   carboxy group;
   oxo group;
   thioxo group;
   amino group optionally substituted by 1 or 2 substituents selected from the group consisting of a C₁₋₆ alkyl group, a C₁₋₆ alkyl-carbonyl group, and a C₁₋₆ alkoxy-carbonyl group;
   C₁₋₆ alkyl group optionally substituted by substituent(s) selected from the group consisting of a halogen atom, a hydroxy group, and a C₁₋₆ alkoxy group;
   C₁₋₆ alkoxy group optionally substituted by halogen atom(s);
   C₁₋₆ alkylsulfonyl group optionally substituted by halogen atom(s) ;
   C₁₋₆ alkyl-carbonyl group optionally substituted by halogen atom(s) ;
   C₁₋₆ alkoxy-carbonyl group;
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
   aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
   trisubstituted silyl group;
   trisubstituted silyloxy group;
   C₃₋₈ cycloalkyl group optionally substituted by 1 to 3 substituents selected from substituent group c;
   C₆₋₁₄ aryl group optionally substituted by 1 to 3 substituents selected from substituent group c; and
   nitrogen-containing aromatic heterocyclic group optionally substituted by 1 to 3 substituents selected from substituent group c (preferably, 5- or 6-membered nitrogen-containing aromatic heterocyclic group).
(substituent group c):
   halogen atom;
   hydroxy group;
   cyano group;
   carboxy group;
   amino group optionally substituted by 1 or 2 substituents selected from the group consisting of a C₁₋₆ alkyl group and a C₁₋₆ alkoxy-carbonyl group;
   C₁₋₆ alkyl group optionally substituted by halogen atom(s);
   C₁₋₆ alkoxy group optionally substituted by halogen atom(s);
   C₁₋₆ alkylsulfonyl group optionally substituted by halogen atom(s) ;
   C₁₋₆ alkyl-carbonyl group;
   C₁₋₆ alkoxy-carbonyl group;
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
   C₆₋₁₄ aryl group optionally substituted by 1 to 3 substituents selected from the group consisting of a halogen atom, a C₁₋₆ alkyl group, and a C₁₋₆ alkoxy group; and
   nitrogen-containing aromatic heterocyclic group optionally substituted by 1 to 3 substituents selected from the group consisting of a halogen atom, a C₁₋₆ alkyl group, and a C₁₋₆ alkoxy group (preferably, 5- or 6-membered nitrogen-containing aromatic heterocyclic group).
(substituent group d):
   halogen atom;
   hydroxy group;
   carboxy group;
   nitro group;
   cyano group;
   amino group optionally substituted by 1 or 2 substituents selected from the group consisting of a C₁₋₆ alkyl group and a C₁₋₆ alkoxy-carbonyl group;
   C₁₋₆ alkyl group optionally substituted by 1 to 3 substituents selected from substituent group a;
   C₁₋₆ alkoxy group optionally substituted by 1 to 3 substituents selected from substituent group a;
   C₁₋₆ alkoxy-carbonyl group;
   C₁₋₆ alkylsulfonyl group optionally substituted by 1 to 3 substituents selected from substituent group a;
   C₃₋₈ cycloalkyl group optionally substituted by 1 to 3 substituents selected from substituent group b;
   acyl group optionally substituted by 1 to 3 substituents selected from substituent group a;
   acyloxy group optionally substituted by 1 to 3 substituents selected from substituent group a;
   C₆₋₁₄ aryl group optionally substituted by 1 to 3 substituents selected from substituent group b;
   C₆₋₁₄ aryloxy group optionally substituted by 1 to 3 substituents selected from substituent group b;
   C₆₋₁₄ arylsulfonyl group optionally substituted by 1 to 3 substituents selected from substituent group b;
   C₆₋₁₄ arylsulfonyloxy group optionally substituted by 1 to 3 substituents selected from substituent group b;
   C₇₋₁₈ aralkyl group optionally substituted by 1 to 3 substituents selected from substituent group b;
   C₇₋₁₈ aralkyloxy group optionally substituted by 1 to 3 substituents selected from substituent group b;
   carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
   carbamoyloxy group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
   oxo group;
   thioxo group;
   mono- or di-C₁₋₆ alkylamino group;
   azido group;
   trisubstituted silyl group;
   trisubstituted silyloxy group;
   aromatic heterocyclic group optionally substituted by 1 to 3 substituents selected from substituent group b; and non-aromatic heterocyclic group optionally substituted by 1 to 3 substituents selected from substituent group b.

When the (substituent group d) is any substituent of the "optionally substituted C₁₋₆ alkyl group" or the "optionally substituted C₁₋₆ alkoxy group", it means a substituent other than the "C₁₋₆ alkyl group optionally substituted by substituent (s) selected from substituent group a" and the "C₇₋₁₈ aralkyl group optionally substituted by substituent(s) selected from substituent group b".

In the present specification, the "pharmaceutically acceptable salt thereof" means a salt that can be used as a medicament. When the compound (I) of the present invention has an acidic group or a basic group, it shows a basic salt or an acidic salt that can be obtained by reacting with a base or an acid. A water-soluble salt is preferred.

Examples of the pharmaceutically acceptable "basic salt" of the compound (I) of the present invention include alkali metal salts such as sodium salt, potassium salt, lithium salt, and the like; alkaline earth metal salts such as magnesium salt, calcium salt, and the like; ammonium salts such as ammonium salt, tetramethylammonium salt, and the like; salts with organic base such as N-methylmorpholinium salt, triethylamine salt, tributylamine salt, diisopropylethylamine salt, dicyclohexylamine salt, N-methylpiperidinium salt, pyridinium salt, 4-pyrrolidinopyridinium salt, picolinium salt, and the like, and the like. It is preferably an alkali metal salt.

Examples of the pharmaceutically acceptable "acidic salt" of the compound (I) of the present invention include inorganic acid salts such as hydrohalides (e.g., hydrofluoride, hydrochloride, hydrobromide, hydroiodide, and the like), nitride, perchloride, sulfate, phosphate and the like; organic acid salts such as lower alkanesulfonates (e.g., methanesulfonate, trifluoromethanesulfonate, ethanesulfonate, and the like), arylsulfonates (e.g., benzenesulfonate, p-toluenesulfonate, and the like), acetate, malate, fumarate, succinate, citrate, ascorbate, tartrate, oxalate, maleate and the like, and the like. A hydrohalide (particularly, hydrochloride) is preferred.

In the present specification, the "salt thereof" means all salts including the aforementioned "pharmaceutically acceptable salt thereof".

In the present specification, the "prophylaxis" includes prevention of disease onset, delay of disease onset, and prevention of pathogenesis. The "prophylactically effective amount" refers to a dose of the active ingredient sufficient to achieve the prophylactic purposes.

In the present specification, the "treatment" includes curing a disease, improving the pathology of a disease (e.g., one or more symptoms), and inhibiting the progression of the disease (severity of the disease). The "therapeutically effective amount" is a dose of the active ingredient sufficient to achieve the therapeutic purpose. Thus, the "improvement" is a concept encompassed by the "treatment".

In the present specification, the "subject" means an object to whom a medicament (pharmaceutical composition) containing an effective amount of an active ingredient necessary to prevent and/or treat (or ameliorate) a disease or a disease condition is administered. The "subject" may be a human or non-human animal (particularly mammal (e.g., mouse, rat, guinea pig, hamster, rabbit, cat, dog, bovine, sheep, monkey etc.)).

In the present specification, the "B0AT1 inhibitor" means an agent containing a compound that has the ability to inhibit B0AT1, a transporter responsible for the reabsorption of neutral amino acids such as phenylalanine in the kidney. In particular, the compound (I) of the present invention, or a pharmaceutically acceptable salt thereof, exhibits excellent inhibitory activity against B0AT1. The B0AT1 inhibitory activity can be measured, for example, by the method described in Non-Patent Document 11 (SLAS Discovery, 2019; 24(2): p.111-120) and the method described in the Experimental Examples described below.

In the present specification, the "diseases for which symptoms can be alleviated by B0AT1 inhibition" means diseases caused by an increase in the level of neutral amino acids in the blood due to a mutation in a gene related to an amino acid metabolic pathway or the like. Specific examples of the disease include amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric academia, and the like.

In the present specification, the configurations of double bonds in the formula (I) (including the formula (I'), the formula (I") and the chemical formulas of the respective Example compounds are all E-configurations regardless of the presence or absence of description in the chemical formulas.

(Compounds of the present invention (compound (I), compound (I'), and compound (I")))
Each group in the aforementioned formula (I) of compound (I) is explained below.

R¹ is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group.

R¹ is preferably a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group, more preferably, a halo C₁₋₄ alkyl group.

X in the number of n are each independently a fluorine atom or a chlorine atom.

X is preferably a chlorine atom.

n is an integer of 0 to 2.

n is preferably 0 or 1, more preferably, 0.

R² is a C₁₋₆ alkyl group optionally substituted by substituent(s) selected from the aforementioned substituent group a.

R² is preferably a C₁₋₄ alkyl group substituted by 1 to 3 substituents selected from the aforementioned substituent group a,
more preferably, a C₁₋₄ alkyl group substituted by 1 to 3 substituents selected from the aforementioned substituent group a',
further preferably, a C₁₋₄ alkyl group substituted by 1 to 3 substituents selected from the aforementioned substituent group a".

R³ is preferably a C₁₋₆ alkyl group optionally substituted by substituent(s) selected from substituent group a or a C₃₋₆ cycloalkyl group optionally substituted by substituent(s) selected from substituent group b.

R³ is preferably a C₁₋₄ alkyl group optionally substituted by 1 to 3 substituents selected from the aforementioned substituent group a,
more preferably, a C₁₋₄ alkyl group optionally substituted by 1 to 3 substituents selected from the aforementioned substituent group a',
further preferably, a C₁₋₄ alkyl group optionally substituted by 1 to 3 substituents selected from the aforementioned substituent group a".

R² and R³ are optionally bonded to each other to form, together with a nitrogen atom bonded to R² and R³, a nitrogen-containing non-aromatic heterocyclic group optionally substituted by substituent(s) selected from substituent group b.

In this case, R² and R³ are preferably bonded to each other to form, together with a nitrogen atom bonded thereto, a 3- to 10-membered (preferably, 5- to 8-membered) monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b.

More preferably, it is a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b, and further preferably, pyrrolidinyl, piperidyl, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl, each of which is optionally substituted by 1 to 3 substituents selected from the aforementioned substituent group b, and particularly preferably, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl, each of which is optionally substituted by 1 to 3 substituents selected from the aforementioned substituent group b.

In a preferred embodiment of the fused nitrogen-containing non-aromatic heterocyclic group, R² and R³ are bonded to each other to form, together with a nitrogen atom bonded thereto, a fused nitrogen-containing non-aromatic heterocyclic group represented by the following formula:

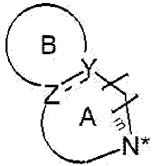

wherein Y and Z are each independently a carbon atom or a nitrogen atom;

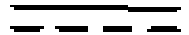

is a single bond or a double bond; ring A is a 5- to 8-membered non-aromatic heterocycle; ring B is a 5- or 6-membered, non-aromatic heterocycle or aromatic heterocycle, or a benzene ring; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula:

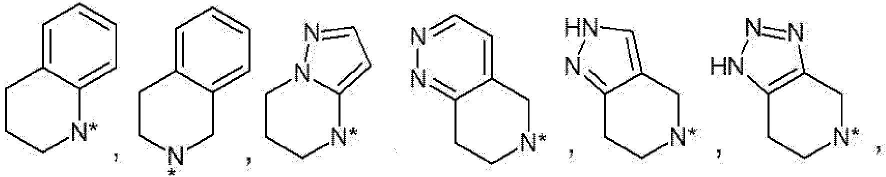

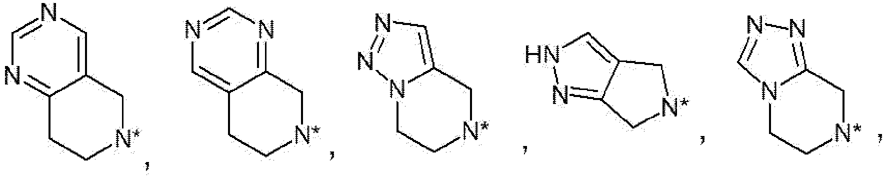

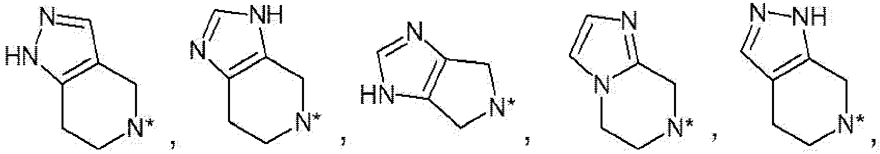

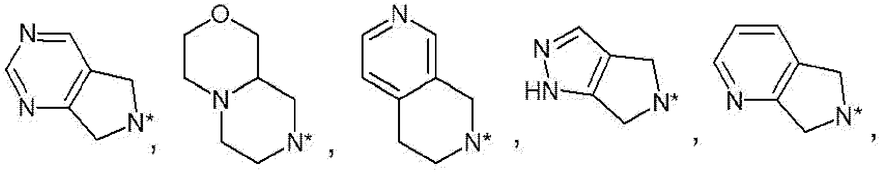

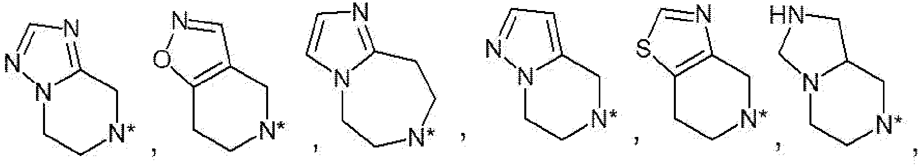

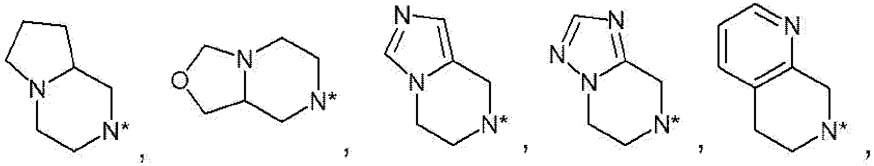

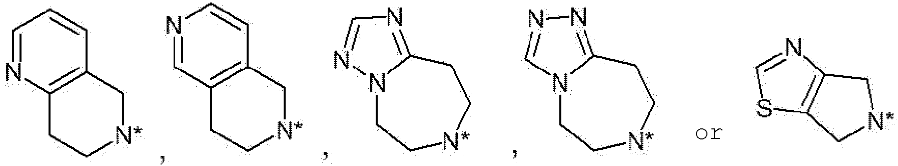

wherein * is a binding site with the carbonyl group, which is optionally substituted by 1 to 3 substituents selected from the aforementioned substituent group b).

In another preferred embodiment, R² and R³ are bonded to each other to form, together with a nitrogen atom bonded thereto, a fused nitrogen-containing non-aromatic heterocyclic group represented by the following formula:

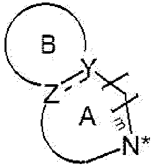

wherein Y and Z are each independently a carbon atom or a nitrogen atom;

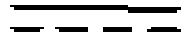

is a single bond or a double bond; ring A is a 5- to 8-membered non-aromatic heterocycle; ring B is a 5- or 6-membered, non-aromatic heterocycle or aromatic heterocycle; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula: 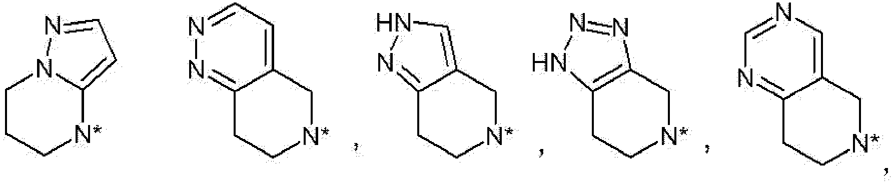 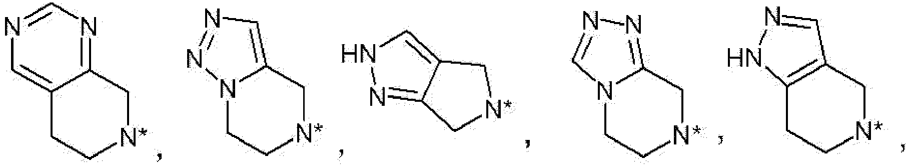 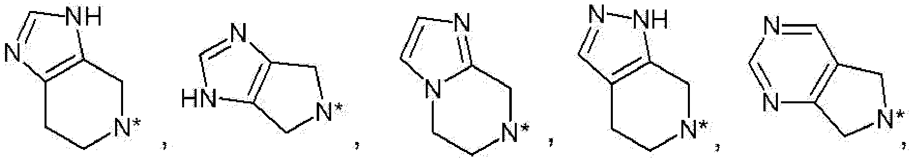 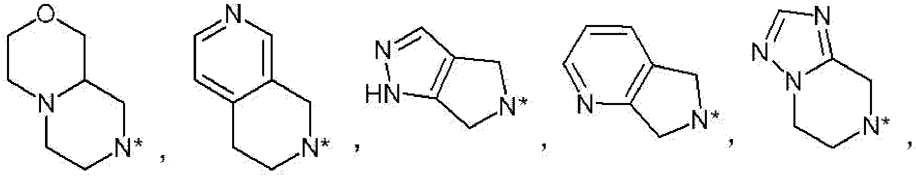 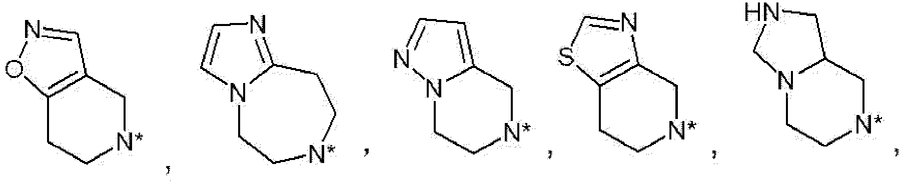 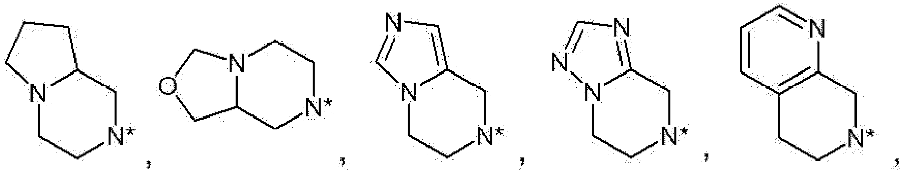 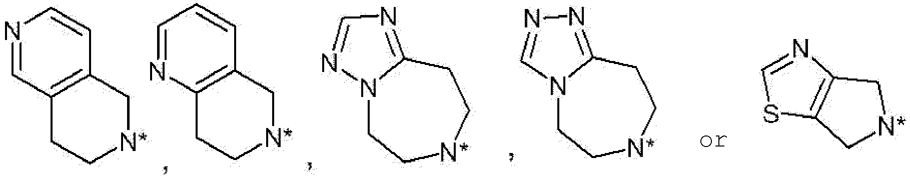 wherein * is a binding site with the carbonyl group, which is optionally substituted by 1 to 3 substituents selected from the aforementioned substituent group b).

As compound (I), the following compounds are preferred.

### [Compound (IA)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1);
R² is a C₁₋₄ alkyl group substituted by 1 to 3 substituents selected from the aforementioned substituent group a; and
R³ is a C₁₋₄ alkyl group optionally substituted by 1 to 3 substituents selected from the aforementioned substituent group a,
or a pharmaceutically acceptable salt thereof.

### [Compound (IB)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
n is 0;
R² is a C₁₋₄ alkyl group substituted by 1 to 3 substituents selected from the aforementioned substituent group a'; and
R³ is a C₁₋₄ alkyl group optionally substituted by 1 to 3 substituents selected from the aforementioned substituent group a' ,
or a pharmaceutically acceptable salt thereof.

### [Compound (IC)]

Compound (I) wherein
R¹ is a halo C₁₋₄ alkyl group;
n is 0;
R² is a C₁₋₄ alkyl group substituted by 1 to 3 substituents selected from the aforementioned substituent group a" ; and
R³ is a C₁₋₄ alkyl group optionally substituted by 1 to 3 substituents selected from the aforementioned substituent group a",
or a pharmaceutically acceptable salt thereof.

### [Compound (ID)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1); and
R² and R³ are bonded to each other to form, together with a nitrogen atom bonded thereto, a 3- to 10-membered (preferably, 5- to 8-membered) monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group (e.g., pyrrolidinyl group, piperidyl group, piperazinyl group, morpholinyl group, thiomorpholinyl group, 3,8-diazabicyclo[3.2.1]octyl group, diazepanyl group, 5,6,7,8-tetrahydro-[1,2,4]triazolo[4,3-a]pyrazinyl group, 5,6,7,8-tetrahydro-[1,2,4]triazolo[1,5-a]pyrazinyl group, 4,5,6,7-tetrahydropyrazolo[1,5-a]pyrazinyl group, 1,2,3,4-tetrahydroisoquinolyl group, 5,6,7,8-tetrahydro-1,6-naphthyridinyl group, 1,2,3,4-tetrahydro-2,6-naphthyridinyl group, 1,2,3,4-tetrahydro-2,7-naphthyridinyl group, 2,6-diazaspiro[3.3]heptyl group, etc.), each of which is optionally substituted by 1 to 3 substituents selected from the aforementioned substituent group b,
or a pharmaceutically acceptable salt thereof.

### [Compound (IE)]

Compound (I) wherein
R¹ is a halo C₁₋₄ alkyl group;
n is 0; and
R² and R³ are bonded to each other to form, together with a nitrogen atom bonded thereto, a 3- to 10-membered (preferably, 5- to 8-membered) monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group (e.g., pyrrolidinyl group, piperidyl group, piperazinyl group, morpholinyl group, thiomorpholinyl group, 3,8-diazabicyclo[3.2.1]octyl group, diazepanyl group, 5,6,7,8-tetrahydro-[1,2,4]triazolo[4,3-a]pyrazinyl group, 5,6,7,8-tetrahydro-[1,2,4]triazolo[1,5-a]pyrazinyl group, 4,5,6,7-tetrahydropyrazolo[1,5-a]pyrazinyl group, 1,2,3,4-tetrahydroisoquinolyl group, 5,6,7,8-tetrahydro-1,6-naphthyridinyl group, 1,2,3,4-tetrahydro-2,6-naphthyridinyl group, 1,2,3,4-tetrahydro-2,7-naphthyridinyl group, 2,6-diazaspiro[3.3]heptyl group, etc.), each of which is optionally substituted by 1 to 3 substituents selected from the aforementioned substituent group b,
or a pharmaceutically acceptable salt thereof.

### [Compound (IF)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1); and
R² and R³ are bonded to each other to form, together with a nitrogen atom bonded thereto, a fused nitrogen-containing non-aromatic heterocyclic group optionally substituted by 1 to 3 substituents selected from the aforementioned substituent group b,
or a pharmaceutically acceptable salt thereof.

### [Compound (IG)]

Compound (I) wherein
R¹ is a halo C₁₋₄ alkyl group;
n is 0; and
R² and R³ are bonded to each other to form, together with a nitrogen atom bonded thereto, a 9- to 14-membered fused nitrogen-containing non-aromatic heterocyclic group optionally substituted by 1 to 3 substituents selected from the aforementioned substituent group b,
or a pharmaceutically acceptable salt thereof.

### [Compound (IH)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1); and
R² and R³ are bonded to each other to form, together with a nitrogen atom bonded thereto, a 9- to 14-membered fused nitrogen-containing non-aromatic heterocyclic group represented by the following formula:

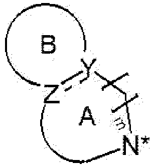

   wherein Y and Z are each independently a carbon atom or a nitrogen atom;

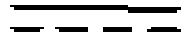

   is a single bond or a double bond; ring A is a 5- to 8-membered non-aromatic heterocycle; ring B is a 5- or 6-membered, non-aromatic heterocycle or aromatic heterocycle, or a benzene ring; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula: 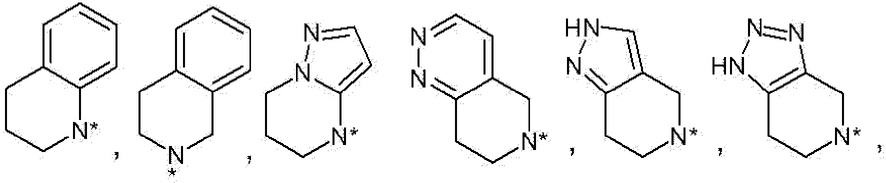 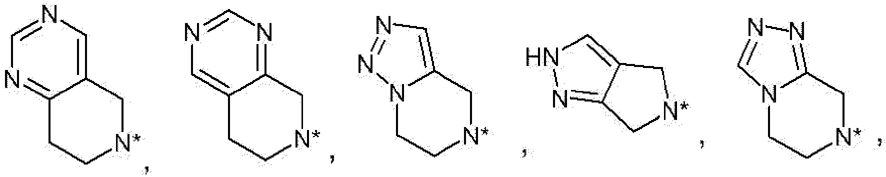 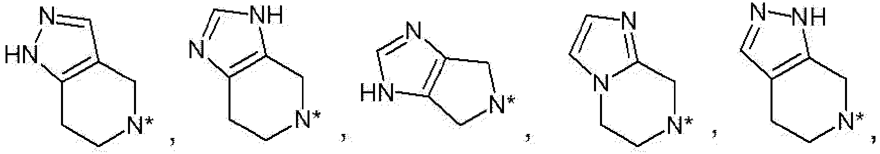 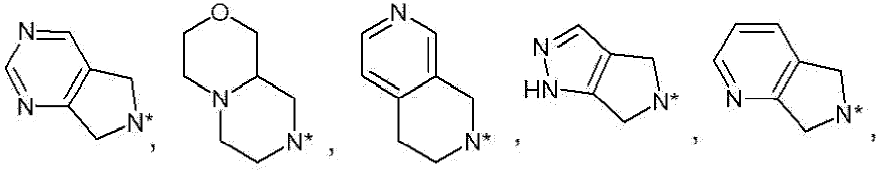 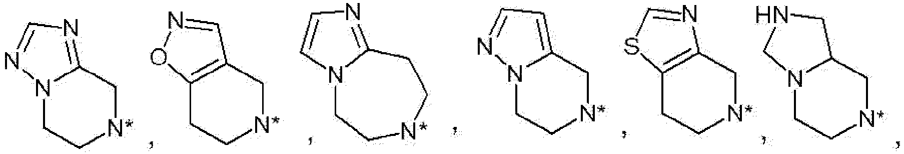 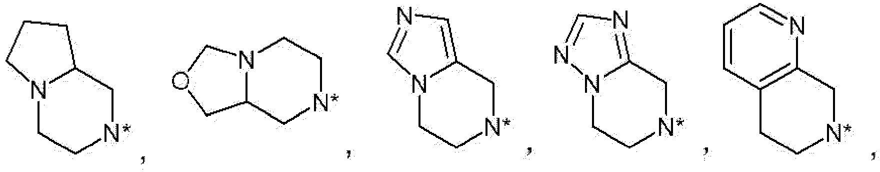 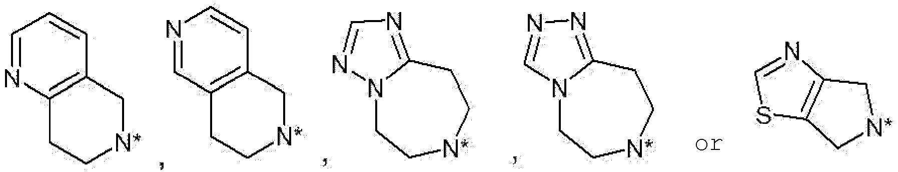 wherein * is a binding site with the carbonyl group, which is optionally substituted by 1 to 3 substituents selected from the aforementioned substituent group b),
   or a pharmaceutically acceptable salt thereof.

### [Compound (IJ)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1); and
R² and R³ are bonded to each other to form, together with a nitrogen atom bonded thereto, a 9- to 14-membered fused nitrogen-containing non-aromatic heterocyclic group represented by the following formula:

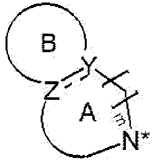

   wherein Y and Z are each independently a carbon atom or a nitrogen atom;

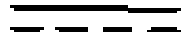

   is a single bond or a double bond; ring A is a 5- to 8-membered non-aromatic heterocycle; ring B is a 5- or 6-membered, non-aromatic heterocycle or aromatic heterocycle; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula: 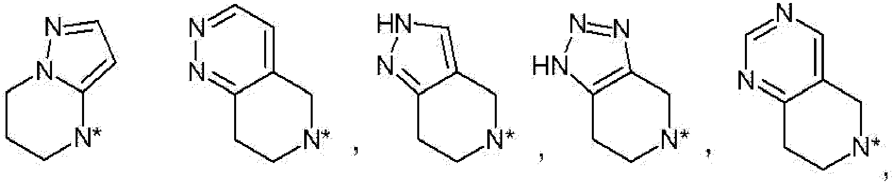 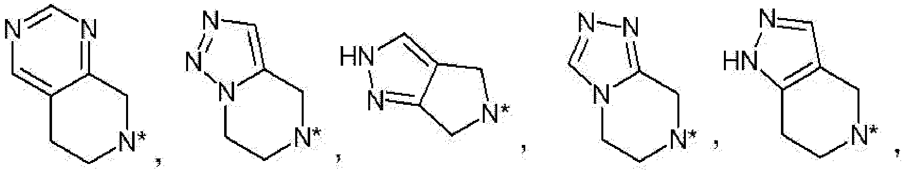 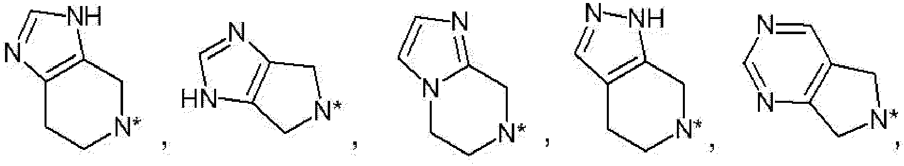 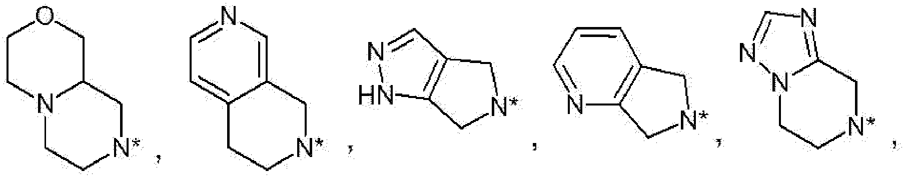 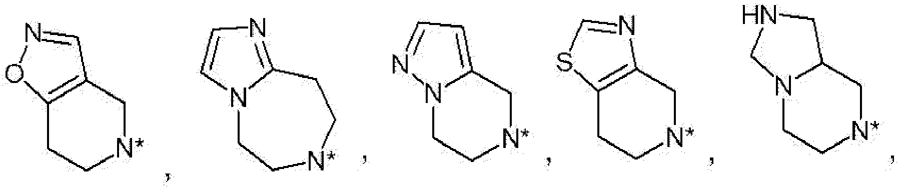 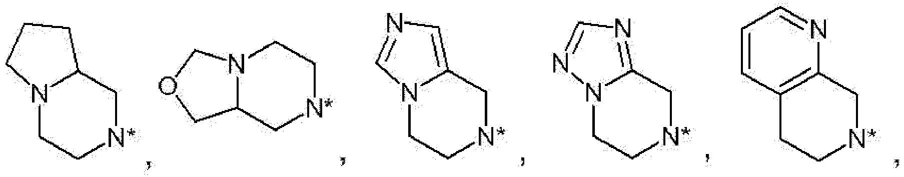 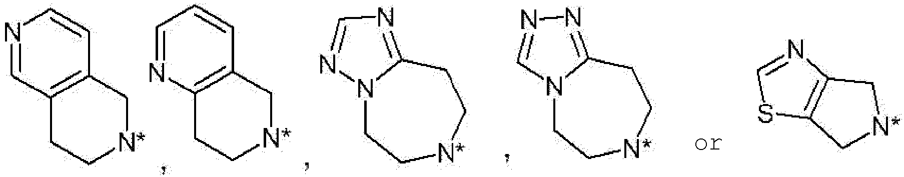 wherein * is a binding site with the carbonyl group, which is optionally substituted by 1 to 3 substituents selected from the aforementioned substituent group b),
   or a pharmaceutically acceptable salt thereof.

### [Compound (IK)]

Compound (I) wherein
R¹ is a halo C₁₋₄ alkyl group;
n is 0; and
R² and R³ are bonded to each other to form, together with a nitrogen atom bonded thereto, a fused nitrogen-containing non-aromatic heterocyclic group represented by the following formula:

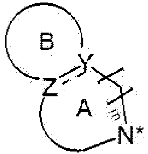

   wherein Y and Z are each independently a carbon atom or a nitrogen atom;

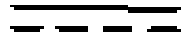

   is a single bond or a double bond; ring A is a 5- to 8-membered non-aromatic heterocycle; ring B is a 5- or 6-membered, non-aromatic heterocycle or aromatic heterocycle, or a benzene ring; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula: 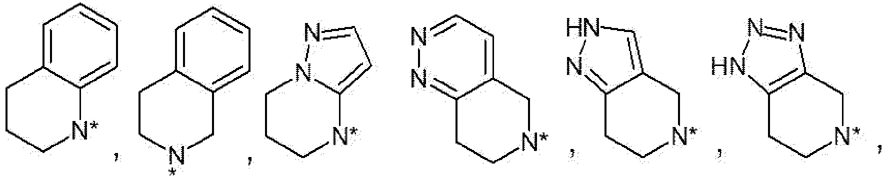 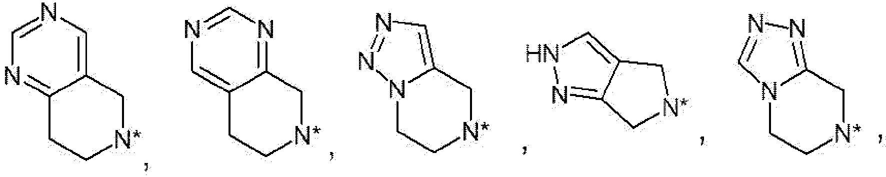 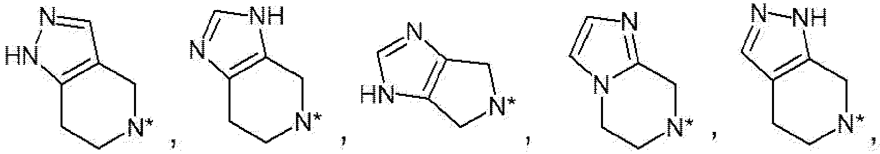 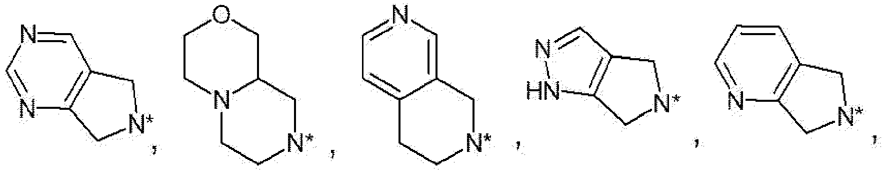 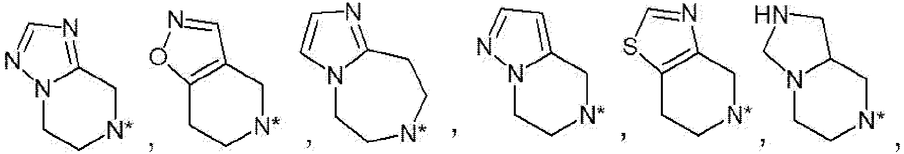 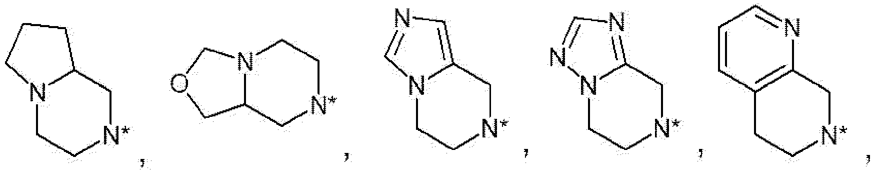 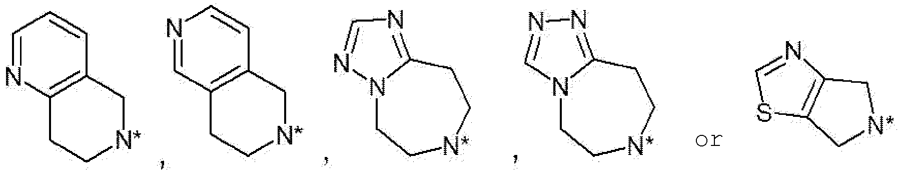 wherein * is a binding site with the carbonyl group, which is optionally substituted by 1 to 3 substituents selected from the aforementioned substituent group b),
   or a pharmaceutically acceptable salt thereof.

### [compound (IL)]

Compound (I) wherein
R¹ is a halo C₁₋₄ alkyl group;
n is 0; and
R² and R³ are bonded to each other to form, together with a nitrogen atom bonded thereto, a fused nitrogen-containing non-aromatic heterocyclic group represented by the following formula:

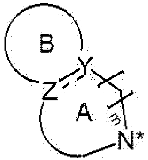

   wherein Y and Z are each independently a carbon atom or a nitrogen atom;

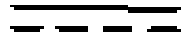

   is a single bond or a double bond; ring A is a 5- to 8-membered non-aromatic heterocycle; ring B is a 5- or 6-membered, non-aromatic heterocycle or aromatic heterocycle; m is an integer of 0 to 3; and * is a binding site with the carbonyl group, which is optionally substituted by 1 to 3 substituents selected from the aforementioned substituent group b (e.g., fused nitrogen-containing non-aromatic heterocyclic group represented by the following formula: 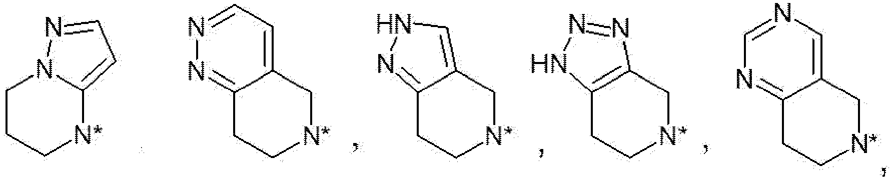 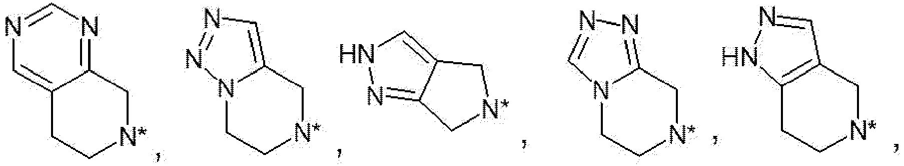 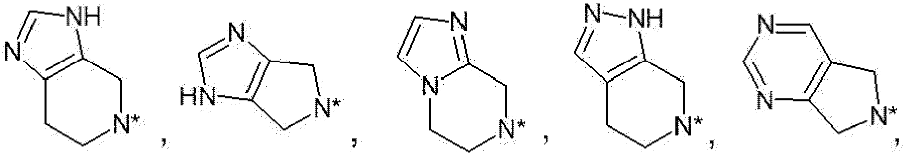 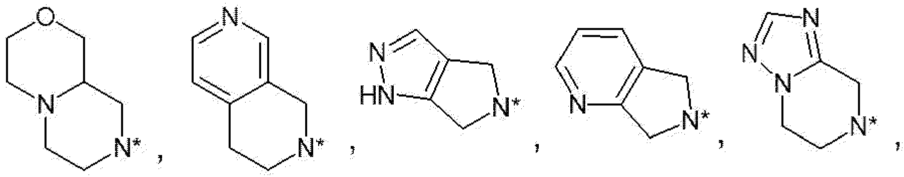 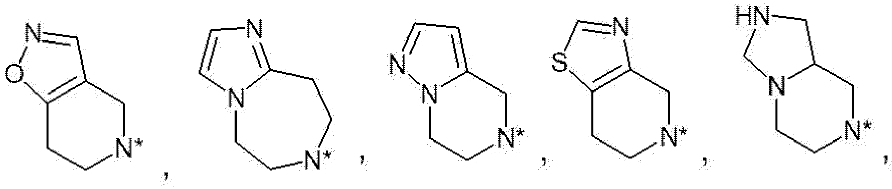 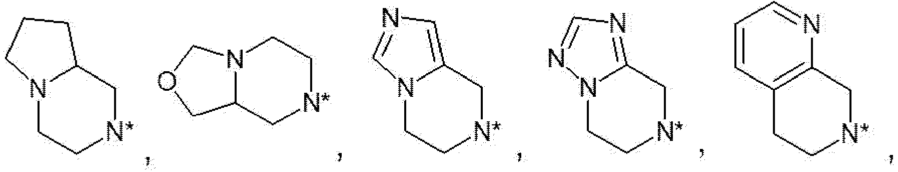 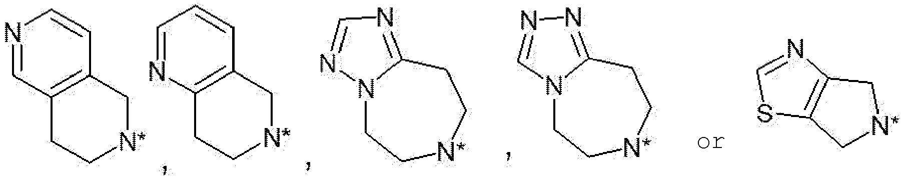 wherein * is a binding site with the carbonyl group, which is optionally substituted by 1 to 3 substituents selected from the aforementioned substituent group b),
   or a pharmaceutically acceptable salt thereof.

### [Compound (IM)]

Compound (I) wherein
R¹ is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X in the number of n are each independently a fluorine atom or a chlorine atom (preferably, a chlorine atom),
n is an integer of 0 to 2 (preferably, 0 or 1); and
R² and R³ are preferably bonded to each other to form, together with a nitrogen atom bonded thereto, a pyrrolidinyl group, a piperidyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, or a 1,2,3,4-tetrahydro-2,7-naphthyridinyl group (preferably, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl), each of which is optionally substituted by 1 to 3 substituents selected from the aforementioned substituent group b),
or a pharmaceutically acceptable salt thereof.

### [Compound (IN)]

Compound (I) wherein
R¹ is a halo C₁₋₄ alkyl group;
n is 0; and
R² and R³ are preferably bonded to each other to form, together with a nitrogen atom bonded thereto, a pyrrolidinyl group, a piperidyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, or a 1,2,3,4-tetrahydro-2,7-naphthyridinyl group (preferably, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl), each of which is optionally substituted by 1 to 3 substituents selected from the aforementioned substituent group b,
or a pharmaceutically acceptable salt thereof.

Among the compounds (I) of the present invention or pharmaceutically acceptable salts thereof, compounds represented by the aforementioned formula (I') and the aforementioned formula (I") (that is, compound (I') and compound (I")) and salts thereof are novel compounds.

Each group in the aforementioned formula (I') is explained below.

R¹' is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group.

R¹' is preferably a halo C₁₋₄ alkyl group.

X' in the number of n' are each independently a fluorine atom or a chlorine atom.

X' is preferably a chlorine atom.

n' is an integer of 0 to 2.

n' is preferably 0 or 1, more preferably, 0.

R²' is a C₁₋₄ alkyl group substituted by substituent(s) selected from the aforementioned substituent group a.

R²' is preferably a C₁₋₄ alkyl group substituted by 1 to 3 substituents selected from the aforementioned substituent group a' .

R³' is a C₁₋₄ alkyl group optionally substituted by substituent(s) selected from the aforementioned substituent group a or a C₃₋₆ cycloalkyl group optionally substituted by substituent(s) selected from the aforementioned substituent group b.

R³' is preferably a C₁₋₄ alkyl group optionally substituted by 1 to 3 substituents selected from the aforementioned substituent group a',
more preferably, a C₁₋₄ alkyl group optionally substituted by 1 to 3 substituents selected from the aforementioned substituent group a".

In another embodiment, R²' and R³' are optionally bonded to each other to form, together with a nitrogen atom bonded to R²' and R³', a nitrogen-containing non-aromatic heterocyclic group optionally substituted by substituent(s) selected from substituent group b.

In such embodiment, R²' and R³' are preferably bonded to each other to form, together with a nitrogen atom bonded thereto, a 3- to 10-membered (preferably, 5- to 8-membered) monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused non-aromatic heterocyclic group, each of which is optionally substituted by 1 to 3 substituents selected from the aforementioned substituent group b.

More preferably, it is a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b, and further preferably, a pyrrolidinyl group, a piperidyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, or a 1,2,3,4-tetrahydro-2,7-naphthyridinyl group, each of which is optionally substituted by 1 to 3 substituents selected from the aforementioned substituent group b, and particularly preferably, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl, each of which is optionally substituted by 1 to 3 substituents selected from the aforementioned substituent group b.

As compound (I'), the following compounds are preferred.

### [Compound (I'A)]

Compound (I') wherein
R¹' is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X' in the number of n' are each independently a fluorine atom or a chlorine atom (preferably, a chlorine atom),
n' is an integer of 0 to 2 (preferably, 0 or 1);
R²' is a C₁₋₄ alkyl group substituted by 1 to 3 substituents selected from the aforementioned substituent group a; and
R³' is a C₁₋₄ alkyl group optionally substituted by 1 to 3 substituents selected from the aforementioned substituent group a,
or a salt thereof (provided that a compound represented by the following formula:

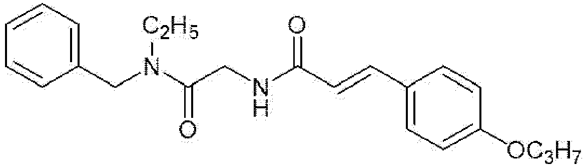

is excluded).

### [Compound (I'B)]

Compound (I') wherein
R¹' is a halo C₁₋₄ alkyl group;
X' in the number of n' are each independently a fluorine atom or a chlorine atom (preferably, a chlorine atom),
n' is an integer of 0 to 2 (preferably, 0 or 1, more preferably, 0);
R²' is a C₁₋₄ alkyl group substituted by 1 to 3 substituents selected from the aforementioned substituent group a'; and
R³' is a C₁₋₄ alkyl group optionally substituted by 1 to 3 substituents selected from the aforementioned substituent group a' ,
or a salt thereof.

### [Compound (I'C)]

Compound (I') wherein
R¹' is a halo C₁₋₄ alkyl group;
n' is 0;
R²' is a C₁₋₄ alkyl group substituted by 1 to 3 substituents selected from the aforementioned substituent group a" ; and
R³' is a C₁₋₄ alkyl group optionally substituted by 1 to 3 substituents selected from the aforementioned substituent group a",
or a salt thereof.

### [Compound (I'D)]

Compound (I') wherein
R¹' is a C₂₋₆ alkyl group, a halo C₁₋₆ alkyl group, a C₃₋₆ cycloalkyl group, a C₂₋₆ alkoxy group, a halo C₂₋₆ alkoxy group, a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or 6-membered nitrogen-containing aromatic heterocyclic group optionally substituted by a halogen atom or a C₁₋₆ alkyl group;
X' in the number of n' are each independently a fluorine atom or a chlorine atom (preferably, a chlorine atom),
n' is an integer of 0 to 2 (preferably, 0 or 1); and
R²' and R³' are optionally bonded to each other to form, together with a nitrogen atom bonded to R²' and R³', a nitrogen-containing non-aromatic heterocyclic group optionally substituted by 1 to 3 substituents selected from substituent group b,
or a salt thereof (provided that compounds represented by the following formulas: 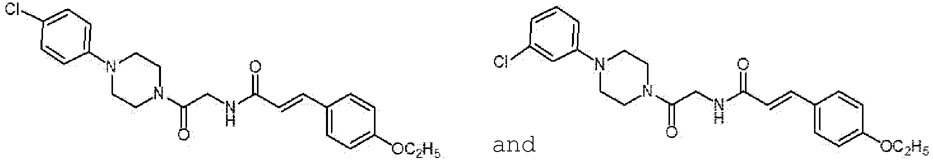 are excluded).

### [Compound (I'E)]

Compound (I') wherein
R¹' is a halo C₁₋₄ alkyl group;
X' in the number of n' are each independently a fluorine atom or a chlorine atom (preferably, a chlorine atom),
n' is an integer of 0 to 2 (preferably, 0 or 1, more preferably, 0);
R²' and R³' are bonded to each other to form, together with a nitrogen atom bonded thereto, a 3 to 10-membered monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b,
or a salt thereof.

### [Compound (I'F)]

Compound (I') wherein
R¹' is a halo C₁₋₄ alkyl group;
X' in the number of n' are each independently a fluorine atom or a chlorine atom (preferably, a chlorine atom),
n' is an integer of 0 to 2 (preferably, 0 or 1, more preferably, 0);
R²' and R³' are bonded to each other to form, together with a nitrogen atom bonded thereto, a 3 to 10-membered monocyclic nitrogen-containing non-aromatic heterocyclic group, 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group (e.g., pyrrolidinyl group, piperidyl group, piperazinyl group, morpholinyl group, thiomorpholinyl group, 3,8-diazabicyclo[3.2.1]octyl group, diazepanyl group, 5,6,7,8-tetrahydro-[1,2,4]triazolo[4,3-a]pyrazinyl group, 5,6,7,8-tetrahydro-[1,2,4]triazolo[1,5-a]pyrazinyl group, 4,5,6,7-tetrahydropyrazolo[1,5-a]pyrazinyl group, 1,2,3,4-tetrahydroisoquinolyl group, 5,6,7,8-tetrahydro-1,6-naphthyridinyl group, 1,2,3,4-tetrahydro-2,6-naphthyridinyl group, 1,2,3,4-tetrahydro-2,7-naphthyridinyl group, 2,6-diazaspiro[3.3]heptyl group, etc.), each of which is optionally substituted by 1 to 3 substituents selected from the aforementioned substituent group b,
or a salt thereof.

### [Compound (I'G)]

Compound (I') wherein
R¹' is a halo C₁₋₄ alkyl group;
X' in the number of n' are each independently a fluorine atom or a chlorine atom (preferably, a chlorine atom),
n' is an integer of 0 to 2 (preferably, 0 or 1, more preferably, 0);
R²' and R³' are bonded to each other to form, together with a nitrogen atom bonded thereto, a pyrrolidinyl group, a piperidyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, or a 1,2,3,4-tetrahydro-2,7-naphthyridinyl group (preferably, 5,6,7,8-tetrahydro-[1,2,4]triazolo[4,3-a]pyrazinyl, 5,6,7,8-tetrahydro-[1,2,4]triazolo[1,5-a]pyrazinyl, 4,5,6,7-tetrahydropyrazolo[1,5-a]pyrazinyl, 1,2,3,4-tetrahydroisoquinolyl, 5,6,7,8-tetrahydro-1,6-naphthyridinyl, 1,2,3,4-tetrahydro-2,6-naphthyridinyl, or 1,2,3,4-tetrahydro-2,7-naphthyridinyl), each of which is optionally substituted by 1 to 3 substituents selected from the aforementioned substituent group b,
or a salt thereof.

Each group in the aforementioned formula (I") is explained below.

R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group.

R¹'' is preferably a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, a pentafluorosulfanyl group, or an optionally substituted C₆₋₁₄ aryl group, more preferably, a halo C₁₋₄ alkyl group.

X'' in the number of n" are each independently a fluorine atom or a chlorine atom.

X" is preferably a chlorine atom.

n" is an integer of 0 to 2.

n" is preferably 0 or 1, more preferably, 0.

R²" is a C₁₋₄ alkyl group substituted by a group selected from the group consisting of
(i) a 5- or 6-membered monocyclic nitrogen-containing aromatic heterocyclic group optionally substituted by substituent(s) selected from the aforementioned substituent group c, and
(ii) a 5- or 6-membered monocyclic nitrogen-containing non-aromatic heterocyclic group optionally substituted by substituent(s) selected from the aforementioned substituent group b, and
is optionally further substituted by substituent(s) selected from the aforementioned substituent group a.

R²" is preferably a C₁₋₄ alkyl group substituted by a 5- or 6-membered monocyclic nitrogen-containing aromatic heterocyclic group optionally substituted by 1 to 3 substituents selected from the aforementioned substituent group c (e.g., pyridazinyl group, pyrazolyl group, thiazolyl group, pyridyl group, triazolyl group, pyrimidinyl group, oxadiazolyl group, imidazolyl group) and optionally further substituted by 1 to 3 substituents selected from the aforementioned substituent group a''.

R³" is a C₁₋₄ alkyl group substituted by substituent(s) selected from the aforementioned substituent group a.

R³" is preferably a C₁₋₄ alkyl group substituted by 1 to 3 substituents selected from the aforementioned substituent group a',
more preferably, a C₁₋₄ alkyl group substituted by 1 to 3 substituents selected from the aforementioned substituent group a' ' .

In another embodiment, R²" and R³" are optionally bonded to each other to form, together with a nitrogen atom bonded to R²" and R³'', a fused nitrogen-containing non-aromatic heterocyclic group (excluding tetrahydroquinolyl groups and tetrahydroisoquinolyl groups) optionally substituted by substituent(s) selected from the aforementioned substituent group b.

In such embodiment, R²" and R³" are preferably bonded to each other to form, together with a nitrogen atom bonded to R²" and R³", a fused nitrogen-containing non-aromatic heterocyclic group represented by the following formula:

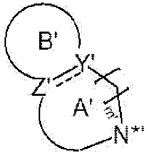

wherein Y' and Z' are each independently a carbon atom or a nitrogen atom;

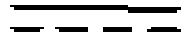

is a single bond or a double bond; ring A' is a 5- to 8-membered non-aromatic heterocycle; ring B' is a 5- or 6-membered, non-aromatic heterocycle or aromatic heterocycle; m' is an integer of 0 to 3 (preferably, 0 to 2); and *' is a binding site with the carbonyl group, which is optionally substituted by 1 to 3 substituents selected from the aforementioned substituent group b,
   more preferably, a fused nitrogen-containing non-aromatic heterocyclic group represented by the following formula: 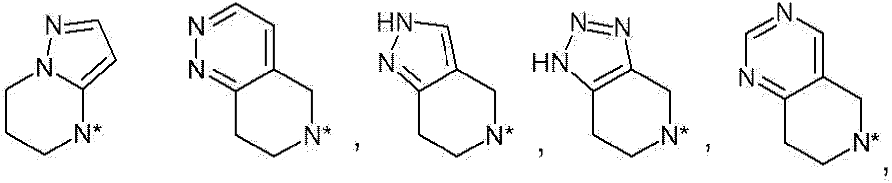 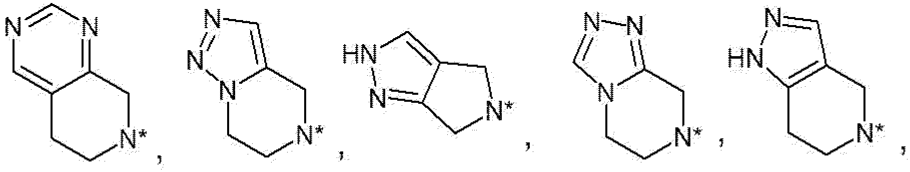 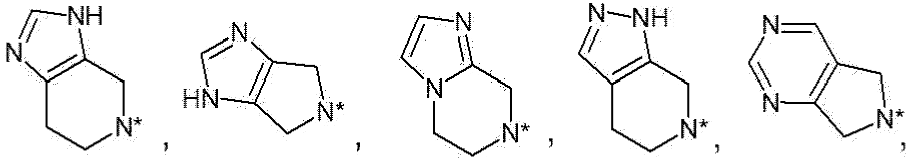 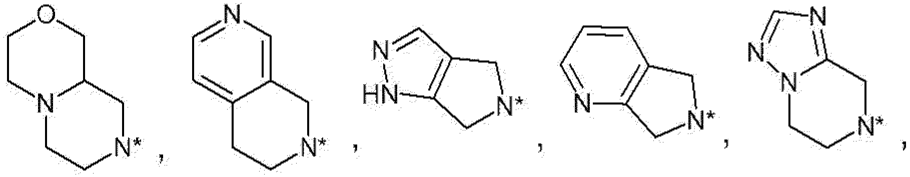 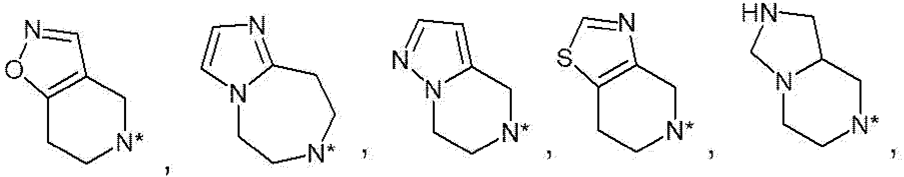 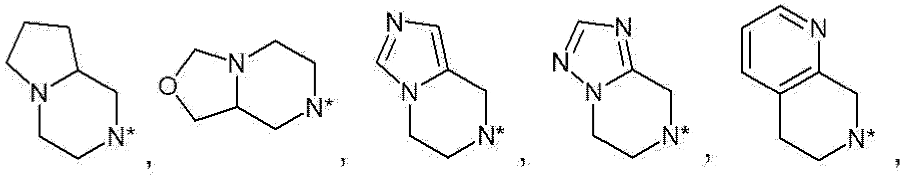 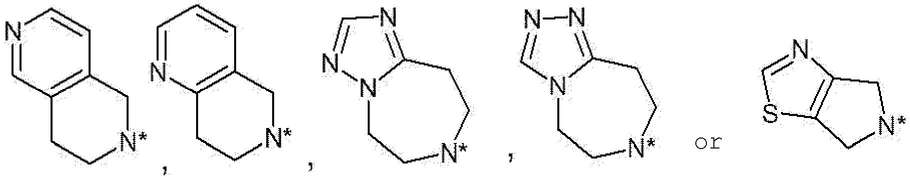 wherein * is a binding site with the carbonyl group, which is optionally substituted by 1 to 3 substituents selected from the aforementioned substituent group b.

As compound (I"), the following compounds are preferred.

### [Compound (I" A)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X'' in the number of n'' are each independently a fluorine atom or a chlorine atom (preferably, a chlorine atom),
n" is an integer of 0 to 2 (preferably, 0 or 1);
R²" is a C₁₋₄ alkyl group substituted by a 5- or 6-membered monocyclic nitrogen-containing aromatic heterocyclic group (e.g., pyridazinyl group, pyrazolyl group, thiazolyl group, pyridyl group, triazolyl group, pyrimidinyl group, oxadiazolyl group, imidazolyl group) optionally substituted by 1 to 3 substituents selected from the aforementioned substituent group c, and further optionally substituted by 1 to 3 substituents selected from the aforementioned substituent group a''; and
R³" is a C₁₋₄ alkyl group substituted by 1 to 3 substituents selected from the aforementioned substituent group a',
or a salt thereof.

### [Compound (I" B)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, a pentafluorosulfanyl group, or an optionally substituted C₆₋₁₄ aryl group;
X'' in the number of n'' are each independently a fluorine atom or a chlorine atom (preferably, a chlorine atom),
n" is an integer of 0 to 2 (preferably, 0 or 1);
R²" is a C₁₋₄ alkyl group substituted by a 5- or 6-membered monocyclic nitrogen-containing aromatic heterocyclic group (e.g., pyridazinyl group, pyrazolyl group, thiazolyl group, pyridyl group, triazolyl group, pyrimidinyl group, oxadiazolyl group, imidazolyl group) optionally substituted by 1 to 3 substituents selected from the aforementioned substituent group c, and further optionally substituted by 1 to 3 substituents selected from the aforementioned substituent group a''; and
R³" is a C₁₋₄ alkyl group substituted by 1 to 3 substituents selected from the aforementioned substituent group a',
or a salt thereof.

### [Compound (I" C)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, a pentafluorosulfanyl group, or an optionally substituted C₆₋₁₄ aryl group;
n'' is 0;
R²" is a C₁₋₄ alkyl group substituted by a 5- or 6-membered monocyclic nitrogen-containing aromatic heterocyclic group (e.g., pyridazinyl group, pyrazolyl group, thiazolyl group, pyridyl group, triazolyl group, pyrimidinyl group, oxadiazolyl group, imidazolyl group) optionally substituted by 1 to 3 substituents selected from the aforementioned substituent group c, and further optionally substituted by 1 to 3 substituents selected from the aforementioned substituent group a"; and
R³" is a C₁₋₄ alkyl group substituted by 1 to 3 substituents selected from the aforementioned substituent group a",
or a salt thereof.

### [Compound (I" D)]

Compound (I") wherein
R¹" is a halo C₁₋₄ alkyl group;
n'' is 0;
R²" is a C₁₋₄ alkyl group substituted by a 5- or 6-membered monocyclic nitrogen-containing aromatic heterocyclic group (e.g., pyridazinyl group, pyrazolyl group, thiazolyl group, pyridyl group, triazolyl group, pyrimidinyl group, oxadiazolyl group, imidazolyl group) optionally substituted by 1 to 3 substituents selected from the aforementioned substituent group c, and further optionally substituted by 1 to 3 substituents selected from the aforementioned substituent group a"; and
R³" is a C₁₋₄ alkyl group substituted by 1 to 3 substituents selected from the aforementioned substituent group a",
or a salt thereof.

### [compound (I" E)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X'' in the number of n" are each independently a fluorine atom or a chlorine atom (preferably, a chlorine atom),
n" is an integer of 0 to 2 (preferably, 0 or 1); and
R²" and R³" are bonded to each other to form, together with a nitrogen atom bonded thereto, a fused nitrogen-containing non-aromatic heterocyclic group (excluding tetrahydroquinolyl groups and tetrahydroisoquinolyl groups),
or a salt thereof.

### [Compound (I" F)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X'' in the number of n" are each independently a fluorine atom or a chlorine atom (preferably, a chlorine atom),
n" is an integer of 0 to 2 (preferably, 0 or 1); and
R²" and R³" are bonded to each other to form, together with a nitrogen atom bonded thereto, a fused nitrogen-containing non-aromatic heterocyclic group represented by the following formula: 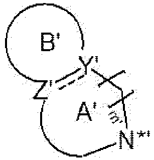
wherein Y' and Z' are each independently a carbon atom or a nitrogen atom;

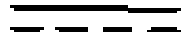

is a single bond or a double bond; ring A' is a 5- to 8-membered non-aromatic heterocycle; ring B' is a 5- or 6-membered, non-aromatic heterocycle or aromatic heterocycle; m' is an integer of 0 to 3 (preferably, 0 to 2); and *' is a binding site with the carbonyl group, which is optionally substituted by 1 to 3 substituents selected from the aforementioned substituent group b,
or a salt thereof.

### [Compound (I" G)]

Compound (I") wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, a pentafluorosulfanyl group, or an optionally substituted C₆₋₁₄ aryl group;
X'' in the number of n'' are each independently a fluorine atom or a chlorine atom (preferably, a chlorine atom),
n" is an integer of 0 to 2 (preferably, 0 or 1); and
R²" and R³" are bonded to each other to form, together with a nitrogen atom bonded thereto, a fused nitrogen-containing non-aromatic heterocyclic group represented by the following formula:

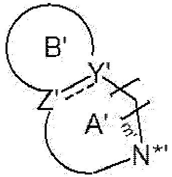

   wherein Y' and Z' are each independently a carbon atom or a nitrogen atom;

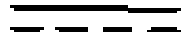

   is a single bond or a double bond; ring A' is a 5- to 8-membered non-aromatic heterocycle; ring B' is a 5- or 6-membered, non-aromatic heterocycle or aromatic heterocycle; m' is an integer of 0 to 3 (preferably, 0 to 2); and *' is a binding site with the carbonyl group, which is optionally substituted by 1 to 3 substituents selected from the aforementioned substituent group b,
   or a salt thereof.

### [Compound (I" H)]

Compound (I") wherein
R¹" is a halo C₁₋₄ alkyl group;
n" is 0; and
R²" and R³" are bonded to each other to form, together with a nitrogen atom bonded thereto, a fused nitrogen-containing non-aromatic heterocyclic group represented by the following formula: 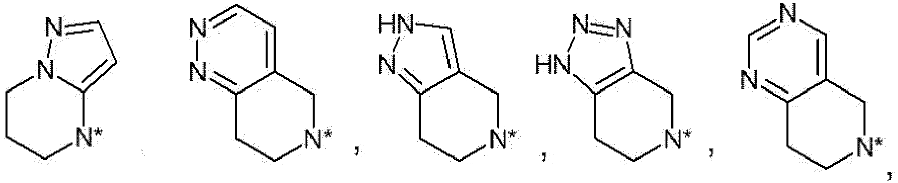 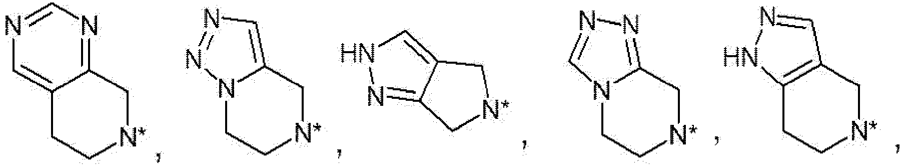 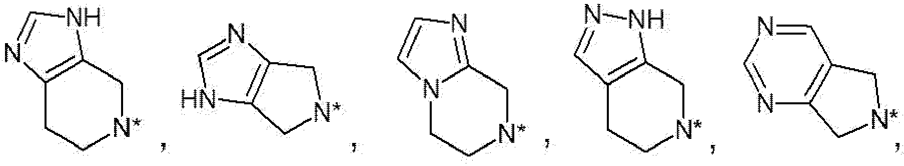 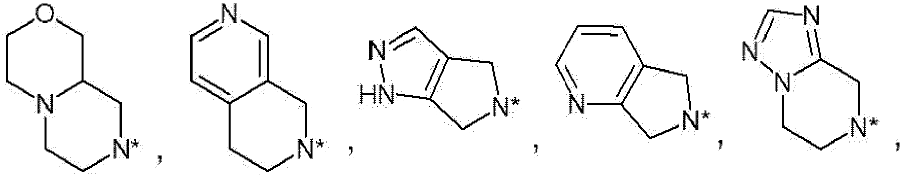 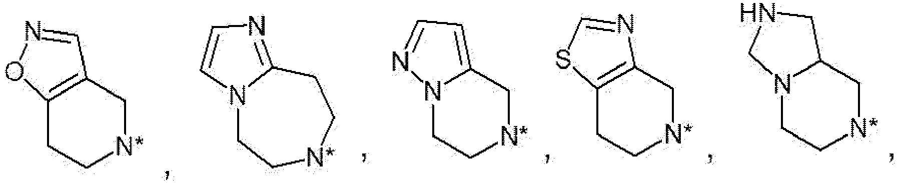 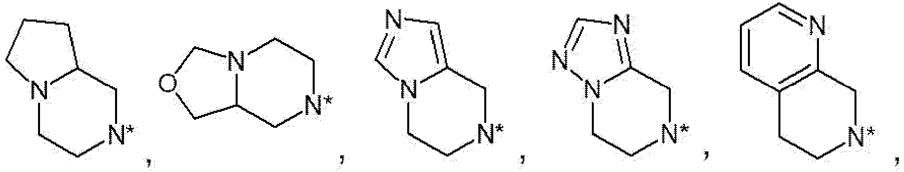 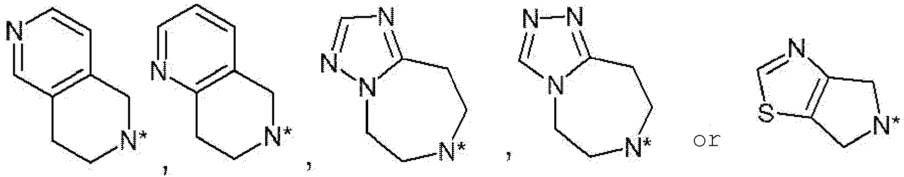
   wherein * is a binding site with the carbonyl group, which is optionally substituted by 1 to 3 substituents selected from the aforementioned substituent group b,
   or a salt thereof.

Specific preferred examples of compound (I) are the compounds of the below-mentioned Examples 1 to 283 (compound (I-1) to compound (I-283)), or pharmaceutically acceptable salts thereof (or a salt thereof).

When compound (I) of the present invention has asymmetric carbon atom(s) in the molecule, it can exist as multiple stereoisomers (i.e., diastereomeric isomers, optical isomers) based on said asymmetric carbon atom(s), and the present invention includes both any one of these stereoisomers and mixtures containing those multiple stereoisomers in any ratio. Isomers due to conformation or tautomerism may also be formed, and such isomers or mixtures thereof are also included in compound (I) of the present invention.

Compound (I) of the present invention may be labeled or substituted with isotopes (e.g., ²H, ³H, ¹³C, ¹⁴C, ¹⁵N, ¹⁸F, ³²P, ³⁵S, ¹²⁵I, etc.). The isotope-labeled or isotopically-substituted compounds can be used as tracers (PET tracers) for positron emission tomography (PET), for example, and are useful in the field of medical diagnosis and the like.

The compound (I) of the present invention or a pharmaceutically acceptable salt thereof may be crystal, and may have a single crystal form or a mixture of several crystal forms.

The compound (I) of the present invention or a pharmaceutically acceptable salt thereof may also include intramolecular salts or adducts thereof and their solvates. Their solvates are those in which a solvent molecule is coordinated to compound (I) or a salt thereof, and hydrates are also included. For example, hydrates of compound (I) or a salt thereof, ethanol solvates, dimethyl sulfoxide solvates, etc. are included.

The compound (I) of the present invention may be a prodrug.

Prodrugs of compound (I) of the present invention are compounds that are converted to compound (I) in vivo by reactions with enzymes or stomach acid. A prodrug of compound (I) may be a monoester or diester of a phosphate group, the ester functional group of which preferably has a structure that is easily hydrolyzed or metabolized after administration to the patient. Examples of ester functional groups of such prodrugs include C₁₋₆ alkyl esters optionally substituted with acyloxy groups, phenyl esters, benzyl esters, and the like (see Bioorganic Chemistry, 1984; 12: p. 118-129). In addition, as prodrugs other than the above monoesters or diesters of phosphate groups, see, for example, compounds having groups derived from phosphate groups, etc. as described in Current opinion in investigational drugs, 2006; 7: p. 109-117, J. Med. Chem. 1994; 37: p. 1857-1864, and J. Med. Chem. 2000; 43: p.4570-4574.

As another form of a prodrug of compound (I), for example, when compound (I) has an amino group, a compound in which the amino group is acylated, alkylated, or phosphorylated (e.g., the amino group of compound (I) is eicosanoylated, alanylated, pentylaminocarbonylated, (5-methyl-2-oxo-1,3-dioxolen-4-yl)methoxycarbonylated, tetrahydrofurylated, pyrrolidylmethylated, pivaloyloxymethylated, acetoxymethylated, tert-butylated, etc.); when compound (I) has a hydroxy group, a compound in which the hydroxy group is acylated, alkylated, phosphorylated, borylated (e.g. compound (I) is acetylated, palmitoylated, propanoylated, pivaloylated, succinylated, fumarylated, alanylated, dimethylaminomethylcarbonylated, etc.); when compound (I) has a carboxy group, a compound in which the carboxy group is esterified or amidated (e.g., the carboxy group of compound (I) is ethyl esterified, phenyl esterified, carboxymethyl esterified, dimethylaminomethyl esterified, pivaloyloxymethyl esterified, 1-{(ethoxycarbonyl)oxy}ethyl esterified, phthalidyl esterified, (5-methyl-2-oxo-1,3-dioxolen-4-yl)methyl esterified, 1-{[(cyclohexyloxy)carbonyl]oxy}ethyl esterified, methylamidated, etc.). These compounds can be produced by methods known per se. Furthermore, prodrugs of compound (I) may be hydrated or unhydrated. Prodrugs of compound (I) may also be those that transform into compound (I) under physiological conditions, as described in "Molecular Design", pp. 163-198 in "Development of Pharmaceuticals", Vol. 7, published by Hirokawa Shoten 1990.

### (Production methods of the compound (I) of the present invention)

The production methods of the compound (I) of the present invention or a pharmaceutically acceptable salt thereof are explained below. The compound (I) of the present invention also encompasses compound (I') and compound (I"). In the following, therefore, the production methods of compound (I') and compound (I") are also referred to as the production method of compound (I).

As an example of the production method of compound (I), representative production methods (A) to (E) are described below. However, the production method is not limited thereto. In addition, the following production methods and steps may be combined with each other.

Compound (I) (e.g., compound (I-1) to compound (I-283) described in the below-mentioned Examples) can be produced by the following production methods (A) to (E), the below-mentioned Reference Examples, Examples, or a method analogous thereto, and the like.

Each starting material compound may form a salt if the reaction is not inhibited, and such salts are the same as those of compound (I). If a specific manufacturing method is not described, the starting material compounds can be easily obtained and used commercially, or can be manufactured according to methods known per se or methods analogous thereto. The intermediates produced in the following manufacturing process may be isolated and purified by column chromatography, recrystallization, distillation, or the like, or may be used in the following process without isolation.

The contents of all patent documents, non-patent documents, or reference documents that are expressly cited herein may all be incorporated herein by reference.

### [Production method (A)]

In this production method, compound (1-1) and compound (2-1) are condensed to give compound (Ia).

Compound (1-1) synthesized by a method known per se (e.g., US Patent No. 10,836,736, etc.) can be preferably used.

### Production method (A)

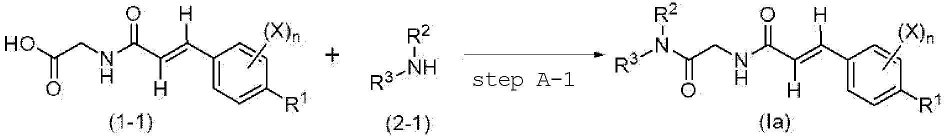

wherein each symbol is as defined above.

### (Step A-1)

In this step, compound (1-1) and compound (2-1) are condensed in the presence of a condensing agent to produce compound (Ia).

The amount of compound (2-1) to be used is 1 - 5 mol, preferably 1 - 3 mol, per 1 mol of compound (1-1).

The condensing agent includes o-(7-azabenzotriazol-1-yl)-N,N,N',N'-tetramethyluronium hexafluorophosphate (HATU), 1-ethyl-3-(3'-dimethylaminopropyl)carbodiimide hydrochloride (EDC-HCl) (WSC hydrochloride), dicyclohexylcarbodiimide (DCC), diisopropylcarbodiimide (DIC), benzotriazol-1-yl-oxy-tris-pyrrolidino-phosphonium hexafluorophosphate (PyBop), O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU), etc., preferably HATU or WSC hydrochloride.

The amount of the condensing agent to be used is generally 1 - 10 mol, preferably 1 - 5 mol, per 1 mol of compound (1-1).

The reaction may be performed in the copresence of 1-hydroxybenzotriazole (HOBt), ethyl 1-hydroxy-1H-1,2,3-triazole-5-carboxylate (HOCt), 1-hydroxy-7-azabenzotriazole (HOAt), or the like as a condensation additive.

The amount of the condensation additive to be used is generally 0 - 1.5 mol per 1 mol of compound (1-1).

Examples of the base include organic bases such as triethylamine, pyridine, N,N-diisopropylethylamine, and the like. Among these, triethylamine or N,N-diisopropylethylamine is preferred.

The amount of the base to be used is generally 1 - 5 mol, preferably 1.5 mol, per 1 mol of compound (1-1).

This reaction can be performed in a solvent that does not affect the reaction. Examples of the reaction solvent include, but are not particularly limited to, aromatic hydrocarbons such as toluene, xylene, and the like; amide solvents such as N,N-dimethylformamide, N,N-dimethylacetamide, and the like; ethers such as diethyl ether, tetrahydrofuran, dioxane, and the like; halogenated hydrocarbons such as chloroform, dichloromethane, and the like; nitriles such as acetonitrile and the like, or a mixture thereof can be mentioned.

The reaction temperature is generally -78°C to room temperature, preferably 0°C to room temperature, and the reaction time is generally 1 to 48 hr.

### [Production method (B)]

In this production method, compound (3-1) and compound (4-1) are condensed to give compound (Ia).

As compound (3-1), a commercially available product, or a compound synthesized by a method known per se can be preferably used.

### Production method (B)

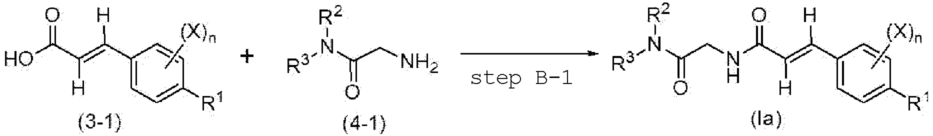

wherein each symbol is as defined above.

### (Step B-1)

In this step, compound (3-1) and compound (4-1) are condensed in the presence of a condensing agent to produce compound (Ia).

The amount of compound (4-1) to be used is 1 - 5 mol, preferably 1 - 3 mol, per 1 mol of compound (3-1).

In step B-1, the reaction can be performed under the same conditions as in the aforementioned step A-1 except that compound (3-1) is used instead of compound (1-1), and compound (4-1) is used instead of compound (2-1).

### [Production method (C)]

In this production method, compound (1-1) and compound (2-2) are condensed in the presence of a condensing agent to obtain compound (Ib), which is converted to compound (Ic) by eliminating the amino-protecting group (P¹), and subjected to a reductive amination reaction with R⁴-CHO in the presence of a reducing agent to give the compound (Id) of the present invention.

### Production method (C)

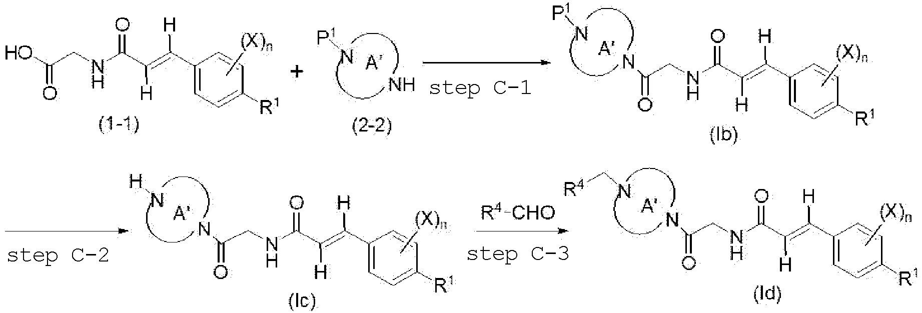

wherein P¹ is a protecting group, R⁴ is a hydrogen atom or a C₂₋₅ alkyl group optionally substituted by a halogen atom, a hydroxy group, or a C₁₋₆ alkoxy group, ring A' is a nitrogen-containing non-aromatic heterocyclic group optionally further having a hetero atom as a ring-constituting atom and optionally further substituted by substituent(s) selected from the aforementioned substituent group b, and other symbols are as defined above.

### (Step C-1)

In this step, compound (1-1) and compound (2-2) are condensed in the presence of a condensing agent to produce compound (Ib).

In step C-1, the reaction can be performed under the same conditions as in the aforementioned step A-1 except that compound (2-2) is used instead of compound (2-1).

The amount of compound (2-2) to be used is 1 - 5 mol, preferably 1 - 3 mol, per 1 mol of compound (1-1).

### (Step C-2)

In this step, the amino-protecting group (P¹) is eliminated to obtain compound (Ic).

For the deprotection step of step C-2, the reaction conditions can be selected according to the kind of protecting group (P1). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protective Groups in Organic Synthesis, 3rd Ed. 2004, "Protecting Groups 3rd Ed." by P. J. Kocienski, etc., or as described in the examples below.

### (step C-3)

In this step, compound (Ic) is reacted with R⁴-CHO in a solvent that does not affect the reaction, in the presence of a reducing agent and an acid (reductive amination reaction) to obtain the compound (Id) of the present invention.

The amount of R⁴-CHO to be used is 1 - 3 mol, preferably 1 - 2 mol, per 1 mol of compound (Ic).

Examples of the reducing agent include sodium triacetoxyborohydride, sodium borohydride, and the like.

The amount of the reducing agent to be used is 1 - 10 mol, preferably 1 - 3 mol, per 1 mol of compound (Ic).

Examples of the acid include organic acids such as acetic acid and the like; Lewis acids such as titanium (IV) chloride, titanium tetraisopropoxide and the like, and the like.

The amount of the acid to be used is 1 - 10 mol, preferably 1 - 3 mol, per 1 mol of compound (Ic).

The reaction solvent is not particularly limited. For example, aromatic hydrocarbons such as benzene, toluene, xylene, and the like; alcohols such as methanol, ethanol, and the like; ethers such as tetrahydrofuran and the like; halogenated hydrocarbons such as chloroform, dichloromethane, and the like or a mixture thereof can be mentioned.

The reaction temperature is generally -10°C to 100°C, preferably 10°C to 50°C, and the reaction time is generally 1 hr to 48 hr.

### [Production method (D)]

In this production method, compound (1-1) and compound (2-3) are condensed to obtain compound (Ie), and the protecting group (P²) is eliminated to convert the compound to the compound (If) of the present invention.

### Production method (D)

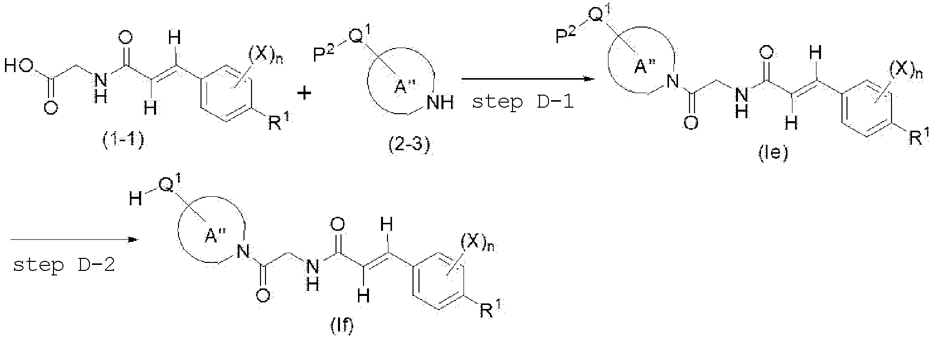

wherein P² is a protecting group, Q¹ is an oxygen atom or C(=O)O* (* is a binding site with P²), ring A" is a nitrogen-containing non-aromatic heterocyclic group optionally further having a hetero atom as a ring-constituting atom and optionally further substituted by substituent(s) selected from the aforementioned substituent group b, and other symbols are as defined above.

### (Step D-1)

In this step, compound (1-1) and compound (2-3) are condensed in the presence of a condensing agent to produce compound (Ie).

In step D-1, the reaction can be performed under the same conditions as in the aforementioned step A-1 except that compound (2-3) is used instead of compound (2-1).

The amount of compound (2-3) to be used is 1 - 5 mol, preferably 1 - 3 mol, per 1 mol of compound (1-1).

### (Step D-2)

In this step, the protecting group (P²) of compound (Ie) is eliminated to obtain compound (If).

For the deprotection process in step D-2, the reaction conditions can be selected according to the kind of protecting group (P2). The conditions for the deprotection reaction can be selected by methods known per se, such as "Protective Groups in Organic Synthesis, 4th Ed." published by Wiley-Interscience in 2007 by Theodora W. Greene and Peter G. M. Wuts; "Protecting Groups 3rd Ed." published by Thieme in 2004 by P. J. Kocienski, etc., or as described in the Examples below.

### [Production method (E)]

In this production method, compound (Ig) and compound (5-1) are subjected to a cross coupling reaction in the presence of a metal catalyst and a base to obtain compound (Ih) of the present invention.

### Production method (E)

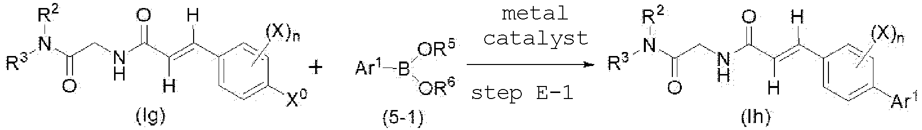

wherein X⁰ is a halogen atom, Ar¹ is an optionally substituted C₆₋₁₄ aryl group, R⁵ and R⁶ are each independently a hydrogen atom or an alkyl group, or R⁵ and R⁶ are optionally bonded to each other to form, together with a boron atom, a cyclic boronic acid ester such as boronic acid pinacol ester and the like, and other symbols are as defined above.

### [Step E-1]

This step can be performed by a cross coupling reaction (Suzuki coupling reaction) of compound (Ig) and an arylboronic acid or arylboronic acid ester (e.g., arylboronic acid pinacol ester, etc.) (compound (5-1)) in a solvent that does not affect the reaction, in the presence of a metal catalyst (e.g., palladium catalyst) and a base.

Compound (Ig) can be produced by the aforementioned production method (A) or (B), or a method analogous thereto.

Compound (5-1) may be a commercially available product or can be produced by methods known per se [e.g., methods described in "Advanced Organic Chemistry, 4th Ed." by Jerry March, "Comprehensive Organic Transformations, 2nd Ed." by Richard C. Larock] or by a method analogous thereto.

The amount of compound (5-1) to be used is generally 1 - 4 mol per 1 mol of compound (Ig).

Metal catalysts include palladium catalysts such as palladium(II) acetate, palladium(II) chloride, dichlorobis(tricyclohexylphosphine)palladium(II), tris(dibenzylideneacetone)dipalladium(0), bis(dibenzylideneacetone)palladium(0) (Pd₂(dba)₃), tetrakis(triphenylphosphine)palladium(0), palladium(II) chloride and diphenylphosphinoferrocene (PdCl₂(dppf)), PdCl₂(dppf) dichloromethane complex, palladium carbon(0), bis(triphenylphosphine)palladium dichloride, and the like; copper catalysts such as copper(I) iodide, copper(I) bromide, copper(I) chloride, copper(II) chloride, and the like; iron catalysts such as iron(III) acetylacetonate, and the like; nickel catalysts such as bis(1,5-cyclooctadiene)nickel(0), bis(acetylacetonate)nickel(II), and the like; ruthenium catalysts such as dichloro(p-cymene)ruthenium(II) dimer, ruthenium alumina, and the like. A palladium catalyst is preferred, and among which dichloro bis(tricyclohexylphosphine)palladium(II) is more preferred.

Where necessary, phosphine ligands such as 1,1'-bis(diphenylphosphino)ferrocene (dppf), 2-dicyclohexylphosphino-2',6'-dimethoxybiphenyl (SPhos), 2-dicyclohexylphosphino-2',4',6'-triisopropylbiphenyl (XPhos), 2-dicyclohexylphosphino-2',6'-diisopropoxybiphenyl (RuPhos), 3,6-dimethoxy-2-dicyclohexylphosphino-2',4' ,6'-triisopropylbiphenyl (BrettPhos), tri-t-butylphosphine, tricyclohexylphosphine, [4-(N,N-dimethylamino)phenyl]di-tert-butylphosphine (AmPhos), (S)-1-[(RP)-2-(dicyclohexylphosphino)ferrocenyl]ethyl di-tert-butylphosphine (JosiPhos), and the like; phenanthrolin and the like may also be added.

The amount of the metal catalyst to be used is generally 0.01 mol - 1 mol, preferably, 0.05 mol - 0.1 mol, per 1 mol of compound (Ig).

The amount of the ligand to be used is generally 0.05 mol - 1 mol, preferably 0.1 mol - 0.4 mol, per 1 mol of compound (Ig).

Examples of the base include alkali metal amides such as lithium diisopropylamide, sodium amide, lithium bis trimethylsilylamide, and the like; alkali metal carbonate salts such as sodium carbonate, potassium carbonate, sodium hydrogen carbonate, potassium hydrogen carbonate, and the like; alkali metal phosphate salts such as sodium phosphate, potassium phosphate, and the like; and amines such as triethylamine, N,N-diisopropylethylamine, pyridine, N-methylmorpholine, and the like.

The amount of the base to be used is generally 1 mol - 5 mol, preferably 2 mol - 4.5 mol, per 1 mol of compound (Ig).

This step can be performed in the presence or absence of an additive. Examples of the additive include alkali metal halides such as potassium fluoride and the like.

The amount of the additive to be used is generally 1 mol - 5 mol, preferably 1.5 - 2.5 mol, per 1 mol of compound (Ig).

The reaction solvent is not particularly limited and includes, for example, amides such as N,N-dimethylformamide, N-methylpyrrolidone, and the like; ethers such as tetrahydrofuran, 1,4-dioxane, and the like; halogenated hydrocarbons such as chloroform, dichloromethane, and the like; aromatic hydrocarbons such as toluene, xylene, and the like; nitriles such as acetonitrile and the like; water; a mixture of these, and the like.

The reaction temperature is generally -78°C to 200°C, preferably -78°C to 120°C.

The reaction time is generally 0.5 to 12 hr.

Compound (I) or a pharmaceutically acceptable salt thereof obtained by the above-mentioned production methods can be isolated and purified by a general separation means, for example, recrystallization, distillation, chromatography, and the like.

When the compound (I) of the present invention or a pharmaceutically acceptable salt thereof exists as an optical isomer based on an asymmetric carbon, it can be separated into individual optical isomers by conventional optical resolution means (e.g., fractional crystallization method, resolution using a chiral column). Alternatively, the optical isomers can be synthesized using optically pure starting materials. Furthermore, optical isomers can also be synthesized by stereoselectively carrying out each reaction using chiral auxiliary groups or asymmetric catalysts.

### (Medicament (pharmaceutical compositions) of the present invention)

Medicament of the present invention is a drug for preventing and/or treating diseases whose symptoms can be alleviated by B0AT1 inhibition, containing compound (I), or a pharmaceutically acceptable salt thereof as the active ingredient, or a B0AT1 inhibitor comprising compound (I) or a pharmaceutically acceptable salt thereof.

Compound (I') and compound (I''), which are novel compounds, are also encompassed in compound (I), and therefore, medicaments containing compound (I') or compound (I'') as an active ingredient are also encompassed in the medicament of the present invention containing compound (I), or a pharmaceutically acceptable salt thereof as the active ingredient.

The medicament of the present invention may be a medicament comprising only compound (I) or a pharmaceutically acceptable salt thereof (or only a B0AT1 inhibitor comprising compound (I) or a pharmaceutically acceptable salt thereof), or a pharmaceutical composition comprising said compound (I) or a pharmaceutically acceptable salt thereof and a pharmaceutically acceptable carrier, etc. The medicament of the present invention can be administered to a subject (e.g., mouse, rat, hamster, rabbit, cat, dog, bovine, sheep, monkey, human, etc.) in a prophylactically effective amount or a therapeutically effective amount.

Examples of the pharmaceutically acceptable carrier include excipient (e.g., starch, lactose, sugar, calcium carbonate, calcium phosphate, etc.), binder (e.g., starch, gum arabic, carboxymethylcellulose, hydroxypropylcellulose, crystalline cellulose, etc.), lubricant (e.g., magnesium stearate, talc, etc.), disintegrant (e.g., carboxymethylcellulose, talc, etc.), solvent (e.g., water for injection, physiological brine, Ringer's solution, alcohol, propylene glycol, polyethylene glycol, sesame oil, corn oil, olive oil, cottonseed oil, etc.), solubilizing agent (e.g., polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.), suspending agent (e.g., surfactants such as stearyltriethanolamine, sodium lauryl sulfate, laurylaminopropionate, lecithin, benzalkonium chloride, benzethonium chloride, glyceryl monostearate, and the like; hydrophilic polymers such as poly(vinyl alcohol), polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil, etc.), isotonic agent (e.g., sodium chloride, glycerol, D-mannitol, D-sorbitol, glucose, etc.), buffering agent (e.g., buffers such as phosphate, acetate, carbonate, citrate, and the like, etc.), soothing agent (e.g., benzyl alcohol, etc.), antiseptic (e.g., p-hydroxybenzoic acid esters, chlorobutanol, benzyl alcohol, phenethyl alcohol, dehydroacetic acid, sorbic acid, etc.), antioxidant (e.g., sulfite, ascorbate, etc.), colorant (e.g., water-soluble food tar color (e.g., food colors such as Food Color Red Nos. 2 and 3, Food Color yellow No.4 and No.5, Food Color Blue Nos. 1 and 2, and the like), water insoluble lake pigment (e.g., the aforementioned aluminum salts of the above water-soluble food tar color), natural dye (e.g., β-carotene, chlorophyll, red iron oxide), etc.), sweetening agent (e.g., saccharin sodium, dipotassium glycyrrhizinate, stevia, etc.), and the like.

The medicament (pharmaceutical composition) of the present invention is prepared by mixing the above-mentioned ingredients, and then preparing the mixture according to known means, for example, oral administration dosage forms such as tablets, fine granules, granules, capsules, dry syrups, and the like, or parenteral dosage forms such as injections (e.g., subcutaneous injection, intravenous injection, intramuscular injection, intraperitoneal injection, intravenous infusion, etc.), topical formulations (e.g., transdermal, ointment, lotion, patch), suppository (e.g., rectal suppository, vaginal suppository), pellet, nasal agent, transpulmonary agent (inhalation agent), ophthalmic agent, implantable agent, microcapsule, liposome, and the like. Among the medicament of the present invention, tablets and the like for oral administration are preferred.

The content of the compound (I) of the present invention, or a pharmaceutically acceptable salt thereof, in the medicament (pharmaceutical composition) of the present invention varies depending on the preparation form. It is generally within the range of about 0.01 to 100 wt%, preferably about 0.1 to 50 wt%, further preferably about 0.5 to 20 wt%, based on the whole preparation.

The dose of the compound (I) of the present invention, or a pharmaceutically acceptable salt thereof, can be selected according to the subject of administration (age, weight, general health condition, sex, degree of disease, etc. of the subject), route of administration, type of disease, kind of concomitant drug, and the like. For example, in the case of a human, when administered orally to an adult patient (weighing approximately 60 kg), the dose of compound (I), or a pharmaceutically acceptable salt thereof, is usually 0.001 mg to 1000 mg, preferably 0.01 mg to 100 mg in terms of the active ingredient, once or several times per day. It can be administered before, after, or between meals. The duration of administration is not limited.

The compound (I) of the present invention, or a pharmaceutically acceptable salt thereof is particularly effective for the prophylactic or therapeutic use for diseases whose symptoms can be alleviated by a B0AT1 inhibitory action, especially, amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric academia, and the like.

The compound (I) of the present invention, or a pharmaceutically acceptable salt thereof can be administered in combination with other drug (concomitant drug) (combined use), as long as the efficacy thereof is not impaired. The concomitant drug is not particularly limited and, for example, one or more known drugs conventionally used for the treatment of "diseases whose symptoms can be alleviated by B0AT1 inhibitory action" exemplified above can be preferably used.

Specifically, when the compound (I) of the present invention or a pharmaceutically acceptable salt thereof is used for the treatment and/or prevention of amino acid metabolism disorders such as phenylketonuria, hypertyrosinemia (type 1-3), hypermethioninemia, maple syrup urine disease, homocystinuria, nonketotic hyperglycinemia, propionic acidemia, methylmalonic acidemia, isovaleric academia, and the like, concomitant drugs to be used in combination include, for example, vitamins (e.g., folic acid (vitamin B9), nicotinamide (vitamin B3), thiamine (vitamin B1), pyridoxine (vitamin B6), etc.) and drugs to alleviate various symptoms of amino acid metabolism disorders (e.g., L-dopa, LNAA such as 5-hydroxytryptophan, sapropterin hydrochloride, pegvaliase, nicotinic acid, nitisinone, S-adenosylmethionine, betaine, aspirin, dipyridamole, sodium benzoate, dextromethorphan, ketamine, antidepressants, anxiolytics, ADHD medications, etc.).

When a concomitant drug is used, the administration period is not limited and can be administered simultaneously or with a time interval to the subject of administration. In the administration with a time interval, the medicament of the present invention may be administered first and the concomitant drug may be administered later, or the concomitant drug may be administered first and the medicament of the present invention may be administered later. Each administration method may be the same or different. In addition, a single preparation containing the compound (I) of the present invention or a pharmaceutically acceptable salt thereof, and a concomitant drug in combination can also be administered.

The dose of the concomitant drug can be appropriately selected with the dose used clinically as the standard. The mixing ratio of the compound of the present invention or a pharmaceutically acceptable salt thereof, and a concomitant drug can be appropriately determined according to the subject of administration (age, body weight, general health condition, gender, severity of the disease, etc.), administration route, type of disease, type of concomitant drug, and the like.

The mass ratio of the compound (I) or a pharmaceutically acceptable salt thereof and the concomitant drug is not particularly limited.

The concomitant drug that complements and/or enhances the therapeutic effect of the compound (I) or a pharmaceutically acceptable salt thereof includes not only those that have been found to date but also those that will be found in the future based on the mechanism described above.

In addition, to complement and/or enhance the therapeutic effect of compound (I) or its pharmaceutically acceptable salt, it may also be useful in combination with dietary therapy or enzyme replacement therapy to avoid the intake of certain amino acids.

The medicament or pharmaceutical composition of the present invention may be provided in the form of a kit together with instructions on how to administer the medicament or pharmaceutical composition. The drug in the kit is supplied by a container manufactured from a material that will effectively sustain the activity of the components of the medicament or pharmaceutical composition for a long period of time, will not adsorb on the inside of the container, and will not alter the components. For example, a sealed glass ampoule may contain buffer and the like sealed in the presence of a neutral, inert gas such as nitrogen gas.

The kit may also be accompanied by instructions for use. The instructions for use of the kit may be printed on paper or other media, or may be stored on an electromagnetically readable medium such as a CD-ROM or DVD-ROM and supplied to the user.

### [Example]

The present invention is explained in more detail in the following by referring to Reference Examples, Examples, and Experimental Examples, which do not limit the present invention. The present invention may be modified without departing from the scope of the invention. The reagents, apparatuses, materials and the like used in the present invention are commercially available unless particularly indicated.

% indicates mol/mol % for yields, and weight % for others unless otherwise specified. Room temperature indicates a temperature of 15°C to 30°C unless otherwise specified. * in the following structural formulas indicates racemic carbon.

Other abbreviations used in the text show the following meanings.
THF: tetrahydrofuran
DMF: N,N-dimethylformamide
HATU: o-(7-azabenzotriazol-1-yl)-N,N,N',N'-tetramethyluronium hexafluorophosphate
WSC: 1-ethyl-3-(3'-dimethylaminopropyl)carbodiimide
DABSO: bis(sulfur dioxide)-1,4-diazabicyclo[2.2.2]octane adduct
HOBt: 1-hydroxybenzotriazole

The starting compounds used in the following Reference Examples and Examples are known compounds unless particularly indicated, and those synthesized and identified according to methods known per se (e.g., US Patent No. 10,836,736; Bioorganic & Medicinal Chemistry Letters, 2017, 27(21), 4805-4811; J. Med. Chem., 2014, 57, 3687-3706; WO 2013/067274), or methods analogous thereto were used.

### Reference Example 1:

### Production of tert-butyl (3R)-3-(5-methyl-1,3,4-oxadiazol-2-yl)piperazine-1-carboxylate

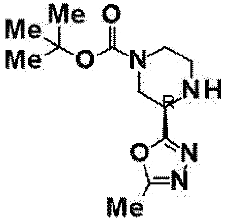

(1) To a solution of (2R)-1-benzyloxycarbonyl-4-tert-butoxycarbonylpiperazine-2-carboxylic acid (28.9 g) in chloroform (160 ml) were sequentially added acetylhydrazine (7.05 g), WSC hydrochloride (22.8 g), 1-hydroxybenzotriazole monohydrate (18.2 g), and triethylamine (16 g), and the reaction mixture was stirred at room temperature for 3 hr 40 min. To the reaction mixture was added water (180 ml) and the organic layer was extracted with chloroform. The combined organic layer was washed with water and saturated brine, sequentially, dried over anhydrous magnesium sulfate, concentrated under reduced pressure, and dried under reduced pressure to give O1-benzyl-O4-tert-butyl(2R)-2-(acetamidocarbamoyl)piperazine-1,4-dicarboxylate (35.3 g; yield 100%) as a pale-yellow amorphous.
   MS(ESI)m/z:421.1[M+H]⁺
(2) To a solution of O1-benzyl-O4-tert-butyl(2R)-2-(acetamidocarbamoyl)piperazine-1,4-dicarboxylate (35.3 g) obtained in the aforementioned (1) in THF (150 ml) was added methyl N-(triethylammonium sulfonyl)carbamate (19.1 g), and the mixture was stirred at 70°C for 1 hr. Then, methyl N-(triethylammonium sulfonyl)carbamate (1.70 g) was added, and the mixture was stirred at 70°C for 40 min. Methyl N-(triethylammonium sulfonyl)carbamate (1.89 g) was further added, and the mixture was stirred at 70°C for 1 hr. The mixture was allowed to cool, and the solvent was evaporated under reduced pressure. To the residue were added ethyl acetate and saturated brine, and the organic layer was extracted with ethyl acetate. The combined organic layer was washed with saturated brine, dried over anhydrous magnesium sulfate, and concentrated under reduced pressure. The obtained residue was purified by silica gel column chromatography (solvent:chloroform/methanol=100/0 - 99/1) to give O1-benzyl-O4-tert-butyl(2R)-2-(5-methyl-1,3,4-oxadiazol-2-yl)piperazine-1,4-dicarboxylate (29.6 g; yield 93%) as a pale-yellow oil. MS(ESI)m/z:403.3[M+H]⁺
(3) To a solution of O1-benzyl-O4-tert-butyl(2R)-2-(5-methyl-1,3,4-oxadiazol-2-yl)piperazine-1,4-dicarboxylate (29.6 g) obtained in the aforementioned (2) in ethanol (250 ml) was added a 54% water suspension (6.4 g) of 5% palladium/carbon (2.96 g) adjusted by adding water, and the reaction mixture was stirred for 6.5 hr under a hydrogen atmosphere at room temperature. The flask was purged with a nitrogen gas, and the reaction mixture was filtered through celite and washed three times with ethanol (50 ml). The obtained filtrate was concentrated under reduced pressure, and the residue was purified by silica gel column chromatography (solvent:chloroform/methanol=50/1 - 40/1, followed by aqueous ammonia 1%-containing chloroform/methanol=40/1 - 10/1). Fraction containing impurities was concentrated, and the residue was purified again by silica gel column chromatography (solvent:chloroform/methanol=99/1 - 97/3). A fraction containing the objective compound, and the fraction with high purity obtained by the first column chromatography were mixed and concentrated under reduced pressure, and the obtained residue was purified again by silica gel column chromatography (solvent:chloroform/methanol=99/1 - 97/3 - 95/5) to give the title compound (6.6 g; yield 34%) as colorless crystals. MS(ESI)m/z:269.2[M+H]⁺

### Reference Example 2:

### Production of (E)-3-[4-(5-methyl-1,2,4-oxadiazol-3-yl)phenyl]prop-2-ene-carboxylic acid

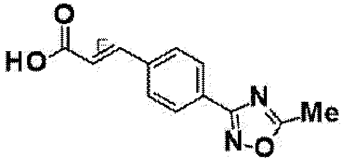

To a solution of 4-(5-methyl-1,2,4-oxadiazol-3-yl)benzaldehyde (1 g) in ethanol (12 ml) were added malonic acid (610 mg) and pyridine (0.12 ml), and the mixture was stirred with heating under reflux for 5 hr. The precipitated solid was collected by filtration and dried under reduced pressure to give the title compound (520 mg; yield 43%) as a white solid.
MS(ESI)m/z:231.1[M+H]⁺

### Reference Example 3:

### Production of methyl 4-methoxy-3-(2-methoxyethylamino)butanoate hydrochloride

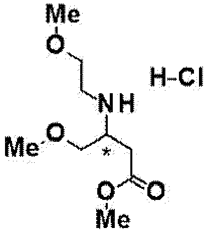

A solution of methyl 4-methoxy-3-oxobutanoate (885 µl), 2-methoxyethanamine (590 µl), and acetic acid (390 µ1) in chloroform (20 ml) was stirred at room temperature for 10 min, sodium triacetoxyborohydride (1.73 g) was added, and the reaction mixture was stirred at room temperature for 50 min. The reaction mixture was cooled to 0°C, and hydrochloric acid (4 mol/l ethyl acetate solution) (15 ml) was slowly added. After stirring at room temperature for a while, the reaction mixture was concentrated, diisopropyl ether was added, and sonicated. The solution was concentrated, ethyl acetate was added, and sonicated. Furthermore, the solution was concentrated, diethyl ether was added, and sonicated. The precipitated solid was collected by filtration, washed with diethyl ether, and dried under vacuum at 60°C to give the title compound (1.17 g, yield 71%) as a yellow solid. MS(ESI)m/z:205.9[M+H]⁺

### Reference Example 4:

### Production of methyl 3-[tert-butoxycarbonyl(2-methoxyethyl)amino]-4-methoxybutanoate

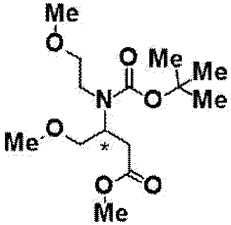

To a solution of methyl 4-methoxy-3-(2-methoxyethylamino)butanoate hydrochloride (240 mg) obtained in Reference Example 3 in dichloromethane (5 ml) were added tert-butoxycarbonyl tert-butylcarbonate (467 mg) and triethylamine (650 µl), and the reaction mixture was stirred at room temperature for 70 min. Furthermore, tert-butoxycarbonyl tert-butylcarbonate (265 mg) was added, and the reaction mixture was stirred at room temperature for 1.5 hr. Water was added, and the aqueous layer was extracted with chloroform. The combined organic layer was filtered through a phase separator and concentrated, and the residue was purified by silica gel column chromatography to give methyl 3-[tert-butoxycarbonyl(2-methoxyethyl)amino]-4-methoxybutanoate (127 mg; yield 42%) as a colorless oil.
MS(ESI)m/z:206.2[M+H-Boc]⁺

### Reference Example 5:

Production of N-methyl-4,5,6,7-tetrahydropyrazolo[1,5-a]pyrazine-2-carboxamide 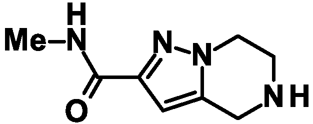
(1) To a solution of 5-phenylmethoxycarbonyl-6,7-dihydro-4H-pyrazolo[1,5-a]pyrazine-2-carboxylic acid (200 mg) in DMF (4 ml) were added aqueous methylamine solution (9.5 mol/l, 1 ml), HATU (253 mg), and N,N-diisopropylethylamine (0.35 ml), and the reaction mixture was stirred at room temperature overnight. To the reaction mixture was added saturated aqueous sodium hydrogen carbonate, and the aqueous layer was extracted with ethyl acetate. The organic layer was washed with saturated brine, dried over anhydrous sodium sulfate, concentrated under reduced pressure, and dried under reduced pressure to give benzyl 2-(methylcarbamoyl)-6,7-dihydro-4H-pyrazolo[1,5-a]pyrazine-5-carboxylate as a crude product (208.6 mg).
(2) To a solution of the crude product (208.6 mg) of benzyl 2-(methylcarbamoyl)-6,7-dihydro-4H-pyrazolo[1,5-a]pyrazine-5-carboxylate obtained in the aforementioned (1) in ethanol (5 ml) was added under a nitrogen atmosphere 10% palladium/carbon (200 mg), the mixture was purged with a hydrogen gas and the reaction mixture was stirred at room temperature for 3 hr. A flask was purged with a nitrogen gas, the reaction mixture was filtered through celite, and the obtained filtrate was concentrated under reduced pressure and dried under reduced pressure to give the title compound as a crude product (119.6 mg) .

### Reference Example 6:

### Production of (8R)-8-methyl-5,6,7,8-tetrahydro-[1,2,4]triazolo[1,5-a]pyrazine hydrochloride

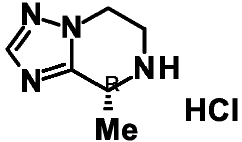

(1) To a solution of (2R)-2-[(2-methylpropan-2-yl)oxycarbonylamino]propanoic acid (503 mg) and formamidine hydrochloride (235 mg) in DMF (15 ml) were added HATU (1.24 g) and N,N-diisopropylethylamine (1.4 ml), and the reaction mixture was stirred at room temperature for 20 min. Then, acetic acid (1.55 ml) and 2-hydrazinoethanol (0.36 ml) were added, and the reaction mixture was stirred at room temperature for 19 hr. The reaction mixture was diluted with ethyl acetate, and saturated aqueous sodium hydrogen carbonate was slowly added thereto at 0°C. The organic layer was separated, and the aqueous layer was extracted with ethyl acetate. The organic layer was combined, dried over anhydrous sodium sulfate, and concentrated under reduced pressure. The residue was purified by silica gel column chromatography to give tert-butyl N-[(1R)-1-[2-(2-hydroxyethyl)-1,2,4-triazol-3-yl]ethyl]carbamate (515 mg; yield 29%) as a yellow oil.
   MS(ESI)m/z:257.1[M+H]⁺
(2) To a solution of tert-butyl N-[(1R)-1-[2-(2-hydroxyethyl)-1,2,4-triazol-3-yl]ethyl]carbamate (408 mg) obtained in the aforementioned (1) in 1,4-dioxane (12 ml) was added cyanomethylenetributylphosphorane (0.82 ml), under a nitrogen atmosphere, and the reaction mixture was heated to 150°C and stirred for 60 min under a nitrogen atmosphere in a microwave reactor. The reaction mixture was concentrated under reduced pressure, and the residue was purified by silica gel column chromatography to give tert-butyl (8R)-8-methyl-6,8-dihydro-5H-[1,2,4]triazolo[1,5-a]pyrazine-7-carboxylate (291 mg; yield 158%) as a yellow viscous oil.
   MS(ESI)m/z:239.0[M+H]⁺
(3) To a solution of tert-butyl (8R)-8-methyl-6,8-dihydro-5H-[1,2,4]triazolo[1,5-a]pyrazine-7-carboxylate (290 mg) obtained in the aforementioned (2) in ethyl acetate was added hydrogen chloride-ethyl acetate solution (4 mol/ml), and the reaction mixture was stirred at room temperature for 18 hr. The reaction mixture was concentrated under reduced pressure to give the title compound as a crude product (135 mg).

### Reference Example 7:

### Production of N-methyl-5,6,7,8-tetrahydro-[1,2,4]triazolo[1,5-a]pyrazine-2-carboxamide

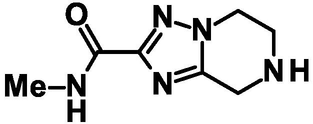

(1) To a solution of ethyl 5,6,7,8-tetrahydro-[1,2,4]triazolo[1,5-a]pyrazine-2-carboxylate (500 mg) in THF (12 ml) were added N,N-diisopropylethylamine (1.4 ml) and benzyl chloroformate (0.54 ml), and the reaction mixture was stirred at room temperature overnight. To the reaction mixture was added aqueous ammonium chloride solution, and the aqueous layer was extracted with ethyl acetate. The organic layer was washed with saturated brine, dried over anhydrous sodium sulfate, and concentrated under reduced pressure. The residue was purified by silica gel column chromatography to give 7-O-benzyl 2-O-ethyl 6,8-dihydro-5H-[1,2,4]triazolo[1,5-a]pyrazine-2,7-dicarboxylate (910 mg; yield 108.1%) as a colorless viscous oil.
   MS(ESI)m/z:331.1[M+H]⁺
(2) To a solution of 7-O-benzyl 2-O-ethyl 6,8-dihydro-5H-[1,2,4]triazolo[1,5-a]pyrazine-2,7-dicarboxylate (857 mg) obtained in the aforementioned (1) in ethanol (6 ml) was added aqueous sodium hydroxide solution (1 mol/l, 4 ml), and the reaction mixture was stirred at room temperature for 3 hr. The reaction mixture was neutralized with hydrochloric acid, and the aqueous layer was extracted with ethyl acetate. The organic layer was washed with saturated brine, dried over anhydrous sodium sulfate, concentrated under reduced pressure, and dried under reduced pressure to give 7-phenylmethoxycarbonyl-6,8-dihydro-5H-[1,2,4]triazolo[1,5-a]pyrazine-2-carboxylic acid (620 mg; yield 79.1%) as a colorless viscous oil.
   MS(ESI)m/z:303.1[M+H]⁺
(3) To a solution of 7-phenylmethoxycarbonyl-6,8-dihydro-5H-[1,2,4]triazolo[1,5-a]pyrazine-2-carboxylic acid (200 mg) obtained in the aforementioned (2) and HATU (377 mg) in DMF (4 ml) were added aqueous methylamine solution (9.5 mol/l, 1.67 ml) and N,N-diisopropylethylamine (0.4 ml), and the reaction mixture was stirred at room temperature overnight. To the reaction mixture was added saturated aqueous sodium hydrogen carbonate, and the aqueous layer was extracted with ethyl acetate. The organic layer was washed with saturated brine, dried over anhydrous sodium sulfate, concentrated under reduced pressure, and dried under reduced pressure to give benzyl 2-(methylcarbamoyl)-6,8-dihydro-5H-[1,2,4]triazolo[1,5-a]pyrazine-7-carboxylate as a crude product (209 mg).
(4) To a solution of the crude product (209 mg) of benzyl 2-(methylcarbamoyl)-6,8-dihydro-5H-[1,2,4]triazolo[1,5-a]pyrazine-7-carboxylate obtained in the aforementioned (3) in ethanol (5 ml) was added under a nitrogen atmosphere 10% palladium/carbon (100 mg), and the reaction mixture was purged with a hydrogen gas and stirred at room temperature for 3 hr. A flask was purged with a nitrogen gas, the reaction mixture was filtered through celite, and the obtained filtrate was concentrated under reduced pressure and dried under reduced pressure to give the title compound as a crude product (119.9 mg) .

### Reference Example 8:

### Production of N,N-dimethyl-5,6,7,8-tetrahydro-[1,2,4]triazolo[1,5-a]pyrazine-2-carboxamide

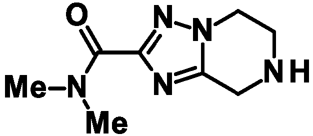

(1) To a solution of 7-phenylmethoxycarbonyl-6,8-dihydro-5H-[1,2,4]triazolo[1,5-a]pyrazine-2-carboxylic acid (200 mg) obtained in Reference Example 7 (2) and HATU (377 mg) in DMF (4 ml) were added aqueous dimethylamine solution (9.5 mol/l, 0.35 ml) and N,N-diisopropylethylamine (0.35 ml), and the reaction mixture was stirred at room temperature overnight. To the reaction mixture was added saturated aqueous sodium hydrogen carbonate, and the aqueous layer was extracted with ethyl acetate. The organic layer was washed with saturated brine, dried over anhydrous sodium sulfate, concentrated under reduced pressure, and dried under reduced pressure to give benzyl 2-(dimethylcarbamoyl)-6,8-dihydro-5H-[1,2,4]triazolo[1,5-a]pyrazine-7-carboxylate as a crude product (217.9 mg).
(2) To a solution of the crude product (217.9 mg) of benzyl 2-(dimethylcarbamoyl)-6,8-dihydro-5H-[1,2,4]triazolo[1,5-a]pyrazine-7-carboxylate obtained in the aforementioned (1) in ethanol (4 ml) was added 10% palladium/carbon (150 mg) under a nitrogen atmosphere, and the reaction mixture was purged with hydrogen gas and stirred at room temperature for 3 hr. A flask was purged with a nitrogen gas, the reaction mixture was filtered through celite, and the obtained filtrate was concentrated under reduced pressure and dried under reduced pressure to give the title compound as a crude product (129.1 mg) .

### Reference Example 9:

### Production of spiro[6,7-dihydro-5H-[1,2,4]triazolo[1,5-a]pyrazine-8,1'-cyclopropane] hydrochloride

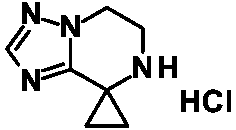

(1) To a solution of 1-[(2-methylpropan-2-yl)oxycarbonylamino]cyclopropane-1-carboxylic acid (965 mg) and formamidine hydrochloride (435 mg) in DMF (30 ml) were added HATU (2.20 g) and N,N-diisopropylethylamine (2.5 ml), and the reaction mixture was stirred at room temperature for 70 min. Then, acetic acid (2.75 ml) and 2-hydrazinoethanol (0.65 ml) were added, and the reaction mixture was stirred at room temperature for 19 hr. The reaction mixture was diluted with ethyl acetate, and saturated aqueous sodium hydrogen carbonate was slowly added at 0°C. The organic layer was separated, and the aqueous layer was extracted with ethyl acetate. The organic layers were combined, dried over anhydrous sodium sulfate, and concentrated under reduced pressure. The residue was purified by silica gel column chromatography to give tert-butyl N-[1-[2-(2-hydroxyethyl)-1,2,4-triazol-3-yl]cyclopropyl]carbamate (1.12 g; yield 39.2%) as a yellow oil. MS(ESI)m/z:269.2[M+H]⁺
(2) To a solution of tert-butyl N-[1-[2-(2-hydroxyethyl)-1,2,4-triazol-3-yl]cyclopropyl]carbamate (1.12 g) obtained in the aforementioned (1) in 1,4-dioxane (30 ml) was added cyanomethylenetributylphosphorane (2.6 ml), and the reaction mixture was heated to 150°C and stirred for 60 min under a nitrogen atmosphere in a microwave reactor. The reaction mixture was concentrated under reduced pressure, and the residue was purified by silica gel column chromatography to give tert-butyl spiro[5,6-dihydro-[1,2,4]triazolo[1,5-a]pyrazine-8,1'-cyclopropane]-7-carboxylate (280 mg; yield 59.6%) as a brown oil.
   MS(ESI)m/z:251.1[M+H]⁺
(3) To tert-butyl spiro[5,6-dihydro-[1,2,4]triazolo[1,5-a]pyrazine-8,1'-cyclopropane]-7-carboxylate (275 mg) obtained in the aforementioned (2) was added hydrogen chloride-ethyl acetate solution (4 mol/1.37 ml), and the reaction mixture was stirred at room temperature for 5 days. The precipitated solid was collected by filtration, washed with ethyl acetate, and dried under reduced pressure at 50°C to give the title compound as a crude product (189 mg).

### Reference Example 10:

### Production of 1-(4,5,6,7-tetrahydropyrazolo[1,5-a]pyrazin-2-yl)ethanol trifluoroacetate

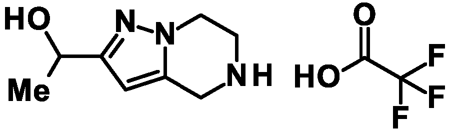

(1) A solution of tert-butyl 2-formyl-6,7-dihydro-4H-pyrazolo[1,5-a]pyrazine-5-carboxylate (346 mg) in THF (6 ml) was cooled to -78°C, methylmagnesium bromide diethyl ether solution (3 mol/l, 0.5 ml) was added, and the reaction mixture was stirred overnight while allowing to warm to room temperature. To the reaction mixture was added water, and the mixture was extracted with ethyl acetate. The organic layer was washed with saturated brine, dried over anhydrous sodium sulfate, and concentrated under reduced pressure. The residue was purified by silica gel column chromatography to give tert-butyl 2-(1-hydroxyethyl)-6,7-dihydro-4H-pyrazolo[1,5-a]pyrazine-5-carboxylate (240 mg; yield 71.1%) as a colorless amorphous.
   MS(ESI)m/z:268.4[M+H]⁺
(2) To a suspension of tert-butyl 2-(1-hydroxyethyl)-6,7-dihydro-4H-pyrazolo[1,5-a]pyrazine-5-carboxylate (250 mg) obtained in the aforementioned (1) in dichloromethane (4 ml) was added trifluoroacetic acid (4 ml), and the reaction mixture was stirred at room temperature for 2 hr. The reaction mixture was concentrated under reduced pressure and dried under reduced pressure to give the title compound as a crude product (263 mg).

### Reference Example 11:

### Production of N-[(1-fluorocyclopropyl)methyl]-1-(5-methyl-1,3,4-oxadiazol-2-yl)methanamine

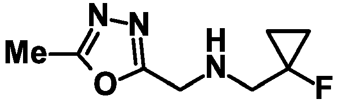

(1) To a solution of (1-fluorocyclopropyl)methanamine hydrochloride (250 mg) in dichloromethane (10 ml) were added triethylamine (0.83 ml) and 2-nitrobenzenesulfonyl chloride (662 mg) under ice-cooling, and the reaction mixture was stirred at room temperature overnight. To the reaction mixture was added aqueous ammonium chloride solution, and the mixture was extracted with chloroform. The organic layer was washed with saturated brine, dried over anhydrous sodium sulfate, and concentrated under reduced pressure. The residue was purified by silica gel column chromatography to give N-[(1-fluorocyclopropyl)methyl]-2-nitrobenzenesulfonamide (291 mg; yield 53.3%) as a pale-yellow solid.
   MS(ESI)m/z:273.0[M-H]⁻
(2) To a solution of N-[(1-fluorocyclopropyl)methyl]-2-nitrobenzenesulfonamide (290 mg) obtained in the aforementioned (1) in DMF (6 ml) were added sodium hydride (about 60%w/w, 64 mg) and 2-(bromomethyl)-5-methyl-1,3,4-oxadiazole (281 mg) under ice-cooling, and the reaction mixture was stirred at 80°C for 4 hr. To the reaction mixture was added water, and the mixture was extracted with ethyl acetate. The organic layer was washed with saturated brine, dried over anhydrous sodium sulfate, and concentrated under reduced pressure. The residue was purified by silica gel column chromatography to give N-[(1-fluorocyclopropyl)methyl]-N-[(5-methyl-1,3,4-oxadiazol-2-yl)methyl]-2-nitrobenzenesulfonamide as a crude product (240 mg) .
(3) To a solution of the crude product (240 mg) of N-[(1-fluorocyclopropyl)methyl]-N-[(5-methyl-1,3,4-oxadiazol-2-yl)methyl]-2-nitrobenzenesulfonamide obtained in the aforementioned (2) in acetonitrile (6 ml) were added 4-ethylbenzenethiol (0.22 ml) and cesium carbonate (448 mg), and the reaction mixture was stirred at room temperature for 3 hr. The reaction mixture was filtered through celite, concentrated under reduced pressure, and dried under reduced pressure to give the title compound as a crude product (196 mg).

### Reference Example 12:

### Production of 1-methoxy-N-[(5-methyl-1,3,4-oxadiazol-2-yl)methyl]propan-2-amine

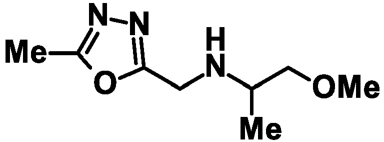

To a solution of (5-methyl-1,3,4-oxadiazol-2-yl)methanamine hydrochloride (300 mg) in dichloromethane (10 ml) were added triethylamine (0.31 ml), 1-methoxypropan-2-one (180 mg), acetic acid (0.11 ml), and sodium triacetoxyborohydride (640 mg), and the reaction mixture was stirred at room temperature overnight. To the reaction mixture was added saturated aqueous sodium hydrogen carbonate, and the mixture was extracted with chloroform. The organic layer was washed with saturated brine, dried over anhydrous sodium sulfate, concentrated under reduced pressure, and dried under reduced pressure to give the title compound as a crude product (371 mg).

### Reference Examples 13 - 14:

Crude products of the compounds described in the following Table 1-1 were obtained by treating the corresponding starting compounds in the same manner as in Reference Example 12.

**[Table 1-1]**

| Reference Example No. | structural formula |
|---|---|
| 13 | 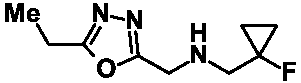 |
| 14 | 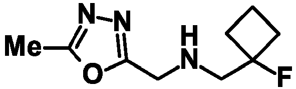 |

### Reference Example 15:

### Production of 1-[[(3S)-pyrrolidin-3-yl]methyl]pyrazole trifluoroacetate

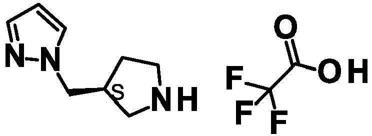

(1) tert-Butyl (3S)-3-(hydroxymethyl)pyrrolidine-1-carboxylate (202 mg), cyanomethylenetributylphosphorane (0.47 ml), and 1H-pyrazole (68 mg) were sequentially added to a 1,4-dioxane (10 ml), and the reaction mixture was stirred at external temperature 125°C for 2 hr. The reaction solution was concentrated to dryness, toluene was added to the obtained residue, and the solvent was evaporated. The residue was purified by reversed-phase HPLC (10 mmol/l aqueous ammonium carbonate solution/acetonitrile=70/30 - 40/60) to give tert-butyl (3S)-3-(pyrazol-1-ylmethyl)pyrrolidine-1-carboxylate (145 mg; yield 57%) as an orange amorphous.
   MS(ESI)m/z:364.4[M+H-tBu]⁺
(2) To a solution of tert-butyl (3S)-3-(pyrazol-1-ylmethyl)pyrrolidine-1-carboxylate (146 mg) obtained in the aforementioned (1) in dichloromethane (2 ml) was added trifluoroacetic acid (0.2 ml), and the mixture was stirred at room temperature for 2 hr. The reaction solution was concentrated to dryness, toluene was added to the obtained residue and the solvent was evaporated to give the title compound as a crude product (154.1 mg).

### Reference Example 16:

A crude product of the compound described in the following Table 1-2 was obtained by treating the corresponding starting compound in the same manner as in Reference Example 15.

**[Table 1-2]**

| Reference Example No. | structural formula |
|---|---|
| 16 | 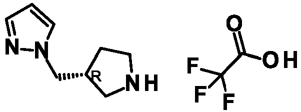 |

### Reference Example 17:

### Production of (2R,4S)-N-methyl-4-pyrazol-1-ylpyrrolidine-2-carboxamide trifluoroacetate

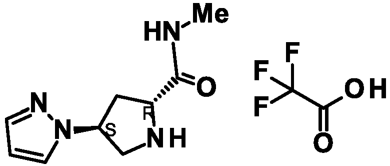

(1) 1-O-tert-butyl 2-O-methyl (2R,4R)-4-hydroxypyrrolidine-1,2-dicarboxylate (302 mg), cyanomethylenetributylphosphorane (0.54 ml), and 1H-pyrazole (85 mg) were sequentially added to a dioxane (6 ml), and the reaction mixture was stirred at external temperature 110°C for 2 hr. The reaction solution was concentrated to dryness, and the residue was purified by reversed-phase HPLC (10 mmol/l aqueous ammonium carbonate solution/acetonitrile=70/30 - 40/60) to give 1-O-tert-butyl 2-O-methyl (2R,4S)-4-pyrazol-1-ylpyrrolidine-1,2-dicarboxylate (251 mg; yield 69%) as a yellow amorphous.
   MS(ESI)m/z:196.1[M+H]⁺
(2) To a solution of 1-O-tert-butyl 2-O-methyl (2R,4S)-4-pyrazol-1-ylpyrrolidine-1,2-dicarboxylate (247 mg) obtained in the aforementioned (1) in methanol (5.5 ml) was added 1 mol/l aqueous sodium hydroxide solution (1.6 ml), and the reaction mixture was stirred at room temperature for 2 hr. To the reaction mixture was added 1 mol/l hydrochloric acid (1.6 ml), and the mixture was concentrated to dryness and the residue was purified by reversed-phase HPLC (10 mmol/l aqueous ammonium carbonate solution/acetonitrile=100/0 - 70/30) to give (2R,4S)-1-[(2-methylpropan-2-yl)oxycarbonyl]-4-pyrazol-1-ylpyrrolidine-2-carboxylic acid (162 mg; yield 69%) as colorless crystals. MS(ESI)m/z:282.1[M+H]⁺
(3) To a solution of (2R,4S)-1-[(2-methylpropan-2-yl)oxycarbonyl]-4-pyrazol-1-ylpyrrolidine-2-carboxylic acid (75 mg) obtained in the aforementioned (2), methanamine hydrochloride (18 mg), and HATU (111 mg) in DMF (1.5 ml) was added N,N-diisopropylethylamine (0.14 ml), and the reaction mixture was stirred at room temperature for 2 hr. The solvent of the reaction mixture was evaporated from the reaction mixture, and the residue was purified by reversed-phase HPLC (10 mmol/l aqueous ammonium carbonate solution/acetonitrile=80/20 - 50/50) to give tert-butyl (2R,4S)-2-(methylcarbamoyl)-4-pyrazol-1-ylpyrrolidine-1-carboxylate (37 mg; yield 47%) as a colorless amorphous. MS(ESI)m/z:295.1[M+H]⁺
(4) To a solution of tert-butyl (2R,4S)-2-(methylcarbamoyl)-4-pyrazol-1-ylpyrrolidine-1-carboxylate (34 mg) obtained in the aforementioned (3) in dichloromethane (1 ml) was added trifluoroacetic acid (0.1 ml), and the mixture was stirred at room temperature for 2 hr. The reaction solution was concentrated to dryness, toluene was added to the obtained residue and the solvent was evaporated to give the title compound as a crude product (36 mg).

### Reference Example 19:

### Production of 6-bromo-4-methoxy-1,2,3,4-tetrahydroisoquinoline

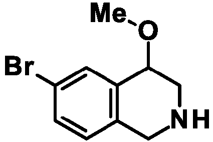

(1) To a solution of 6-bromo-2-(2-nitrophenyl)sulfonyl-3,4-dihydro-1H-isoquinolin-4-ol (300 mg) in toluene (2 ml) were added tetrabutylammonium hydrogen sulfate (25 mg), iodomethane (206 mg), and potassium hydroxide (50%w/v, 2 ml), and the reaction mixture was stirred at 50°C for 1 hr. To the reaction mixture was added water, and the aqueous layer was extracted with ethyl acetate. The organic layer was washed with saturated brine, dried over anhydrous sodium sulfate, and concentrated under reduced pressure. The residue was purified by silica gel column chromatography to give 6-bromo-4-methoxy-2-(2-nitrophenyl)sulfonyl-3,4-dihydro-1H-isoquinoline (295 mg; yield 95%) as a colorless viscous oil.
   MS(ESI)m/z:397.1/399.1[M+H]⁺
(2) To a solution of 6-bromo-4-methoxy-2-(2-nitrophenyl)sulfonyl-3,4-dihydro-1H-isoquinoline (295 mg) obtained in the aforementioned (1) in DMF (2 ml) were added potassium carbonate (191 mg) and phenylmethanethiol (172 mg), and the reaction mixture was stirred at room temperature for 3 hr. To the reaction mixture was added water, and the aqueous layer was extracted with ethyl acetate. The organic layer was washed with saturated brine, dried over anhydrous sodium sulfate, concentrated under reduced pressure, and dried under reduced pressure to give a crude product (167 mg; yield 99%) of the title compound as a brown viscous oil.

### Reference Example 20:

### Production of 6-ethyl-5,6,7,8-tetrahydro-[1,2,4]triazolo[1,5-a]pyrazine

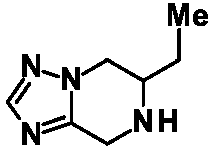

(1) To a solution of 6-bromo-[1,2,4]triazolo[1,5-a]pyrazine (500 mg), cesium carbonate (1.6 g), and 4,4,5,5-tetramethyl-2-vinyl-1,3,2-dioxaborolane (580 mg) in 1,4-dioxane (4 ml) was added water (1 ml), and the reaction mixture was heated to 140°C and stirred for 60 min in a microwave reactor. The reaction mixture was concentrated under reduced pressure, and dried under reduced pressure. The residue was purified by silica gel column chromatography to give 6-ethenyl-[1,2,4]triazolo[1,5-a]pyrazine (238 mg; yield 65%) as a yellow solid.
   ¹H-NMR (400 MHz, CDCl₃):δ 9.27 (d, 1H), 8.50-8.41 (m, 2H), 6.79(dd, 1H), 6. 40(dd, 1H), 5.59(dd, 1H) .
(2) To a solution of 6-ethenyl-[1,2,4]triazolo[1,5-a]pyrazine (238 mg) obtained in the aforementioned (1) in ethanol (2 ml) were added acetic acid (1 ml) and palladium hydroxide (25 mg), the reaction mixture was heated to 50°C under a hydrogen atmosphere and stirred for 4 hr. The reaction mixture was filtered through celite and neutralized with saturated aqueous sodium hydrogen carbonate solution, and the aqueous layer was extracted with ethyl acetate. The organic layer was washed with saturated brine, dried over anhydrous sodium sulfate, and concentrated under reduced pressure. The residue was purified by silica gel column chromatography to give 6-ethyl-5,6,7,8-tetrahydro-[1,2,4]triazolo[1,5-a]pyrazine (172 mg; yield 49%) as a yellow viscous oil.
   MS(ESI)m/z:152.9[M+H]⁺

### Reference Example 21:

### Production of 6-(methoxymethyl)-5,6,7,8-tetrahydro-[1,2,4]triazolo[1,5-a]pyrazine

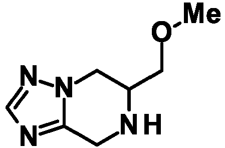

(1) To a solution of 6-bromo-[1,2,4]triazolo[1,5-a]pyrazine (250 mg) in THF (1 ml) were added tributyl(methoxymethyl)tin (605 mg) and (2-dicyclohexylphosphino-2',4',6'-triisopropyl-1,1'-biphenyl)[2-(2'-amino-1,1'-biphenyl)]palladium(II) methanesulfonate (106 mg), and the reaction mixture was heated to 80°C and stirred for 3 hr. The reaction mixture was concentrated under reduced pressure, dried under reduced pressure, and concentrated under reduced pressure. The residue was purified by silica gel column chromatography to give 6-(methoxymethyl)-[1,2,4]triazolo[1,5-a]pyrazine (52 mg; yield 19%) as a white solid.
   MS(ESI)m/z:165.1[M+H]⁺
(2) To a solution of 6-(methoxymethyl)-[1,2,4]triazolo[1,5-a]pyrazine (52 mg) obtained in the aforementioned (1) in ethanol (2.4 ml) were added acetic acid (4.7 ml) and palladium hydroxide (10 mg), and the reaction mixture was heated to 70°C and stirred for 3 hr under a hydrogen atmosphere. The reaction mixture was filtered through celite and neutralized with saturated aqueous sodium hydrogen carbonate solution, and the aqueous layer was extracted with a mixed solution of chloroform/methanol=4:1. The organic layer was washed with saturated brine, dried over anhydrous sodium sulfate, and concentrated under reduced pressure to give 6-(methoxymethyl)-5,6,7,8-tetrahydro-[1,2,4]triazolo[1,5-a]pyrazine (38 mg; yield 94%) as a brown viscous oil.
   MS(ESI)m/z:168.9[M+H]⁺

### Reference Example 22:

### Production of 1,2,3,4-tetrahydroisoquinoline-6-sulfonamide

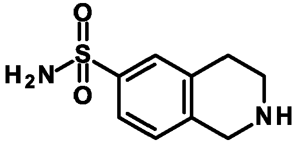

(1) To a solution of benzyl 6-bromo-3,4-dihydro-1H-isoquinoline-2-carboxylate (300 mg) in 2-propanol (3 ml) were added DABSO (210 mg), N-cyclohexyl-N-methylcyclohexanamine (2.8 mmol, 600 µl), and bis(di-tert-butyl(4-dimethylaminophenyl)phosphine)dichloropalladium(II) (31 mg), and the reaction mixture was heated to 110°C and stirred for 60 min in a microwave reactor. The reaction mixture was ice-cooled, sodium hypochlorite (10%w/v, 1.4 ml) and ammonia (9.8 mol/l methanol solution, 0.18 ml) were added, and the mixture was stirred for 30 min. Sodium hypochlorite (10%w/v, 1.4 ml) was further added, and the mixture was allowed to warm to room temperature and stirred for 30 min. To the reaction mixture was added saturated aqueous sodium thiosulfate solution, and the aqueous layer was extracted with ethyl acetate. The organic layer was washed with saturated brine, dried over anhydrous magnesium sulfate, and concentrated under reduced pressure. The residue was purified by silica gel column chromatography to give benzyl 6-sulfamoyl-3,4-dihydro-1H-isoquinoline-2-carboxylate (58 mg; yield 19%) as a yellow viscous oil.
   MS(ESI)m/z:345.2[M-H]⁻
(2) To a solution of benzyl 6-sulfamoyl-3,4-dihydro-1H-isoquinoline-2-carboxylate (58 mg) obtained in the aforementioned (1) in methanol (1 ml) was added palladium hydroxide (20 mg), and the reaction mixture was stirred at 60°C for 2 hr under a hydrogen atmosphere. The reaction mixture was filtered through celite, and the filtrate was concentrated under reduced pressure to give a crude product (36 mg) of the title compound as a colorless viscous oil.

### Reference Example 23:

### Production of N-methyl-1,2,3,4-tetrahydroisoquinoline-6-sulfonamide

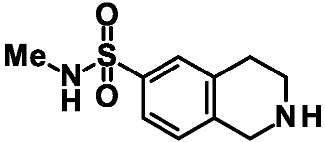

(1) To a solution of benzyl 6-bromo-3,4-dihydro-1H-isoquinoline-2-carboxylate (200 mg) in 2-propanol (2 ml) were added DABSO (140 mg), N-cyclohexyl-N-methylcyclohexanamine (2.8 mmol, 370 µl), and bis(di-tert-butyl(4-dimethylaminophenyl)phosphine)dichloropalladium(II) (21 mg), and the reaction mixture was heated to 110°C and stirred for 60 min in a microwave reactor. The reaction mixture was ice-cooled, sodium hypochlorite (10%w/v, 1 ml) and methylamine (9.8 mol/l methanol solution, 0.11 ml) were added, and the mixture was stirred for 30 min. To the reaction mixture was added saturated aqueous sodium thiosulfate solution, and the aqueous layer was extracted with ethyl acetate. The organic layer was washed with saturated brine, dried over anhydrous magnesium sulfate, and concentrated under reduced pressure. The residue was purified by silica gel column chromatography to give benzyl 6-(methylsulfamoyl)-3,4-dihydro-1H-isoquinoline-2-carboxylate (64 mg; yield 31%) as a yellow oil.
   MS(ESI)m/z:359.3[M-H]⁻
(2) To a solution of benzyl 6-methylsulfamoyl-3,4-dihydro-1H-isoquinoline-2-carboxylate (64 mg) obtained in the aforementioned (1) in methanol (1 ml) was added palladium hydroxide (10 mg), and the reaction mixture was stirred at 60°C for 2 hr under a hydrogen atmosphere. The reaction mixture was filtered through celite, and the filtrate was concentrated under reduced pressure to give a crude product (40 mg) of the title compound as a brown viscous oil.

### Reference Example 2 4:

### Production of pyrrolidin-1-yl (4,5,6,7-tetrahydropyrazolo[1,5-a]pyrazin-3-yl)methanone hydrochloride

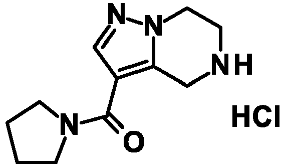

(1) To a solution of 5-[(2-methylpropan-2-yl)oxycarbonyl]-6,7-dihydro-4H-pyrazolo[1,5-a]pyrazine-3-carboxylic acid (90 mg) in acetonitrile (1.1 ml) were added HOBt (55 mg), N,N-diisopropylethylamine (70 µl), pyrrolidine (30 µl), and WSC hydrochloride (76 mg), and the reaction mixture was stirred at room temperature for 60 min. To the reaction mixture was added saturated aqueous ammonium chloride solution, and the aqueous layer was extracted with ethyl acetate. The organic layers were combined, dried over anhydrous sodium sulfate, and concentrated under reduced pressure to give a crude product (108 mg) of tert-butyl 3-(pyrrolidine-1-carbonyl)-6,7-dihydro-4H-pyrazolo[1,5-a]pyrazine-5-carboxylate as a colorless oil.
(2) To a solution of tert-butyl 3-(pyrrolidine-1-carbonyl)-6,7-dihydro-4H-pyrazolo[1,5-a]pyrazine-5-carboxylate (108 mg) obtained in the aforementioned (1) in ethyl acetate (1 ml) was added hydrogen chloride (4 mol/l ethyl acetate solution, 1 ml), and the mixture was stirred at room temperature for 60 min. The reaction mixture was concentrated under reduced pressure, diisopropyl ether was added to the residue, and the precipitated solid was collected by filtration to give the title compound (70 mg; yield 69%) as a white solid. MS(ESI)m/z:221.2[M+H]⁺

### Reference Examples 25 to 28:

The compounds described in the following Table 1-3 were obtained by treating the corresponding starting compounds in the same manner as in Reference Example 24.

**[Table 1-3]**

| Reference Example No. | structural formula | instrument analysis data |
|---|---|---|
| 25 | 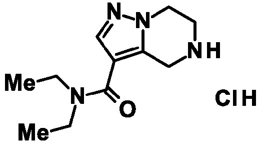 | MS(ESI) m/z:223.2 [M+H]+ |
| 26 | 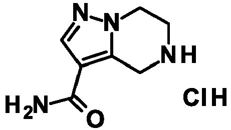 | MS(ESI) m/z:167.1 [M+H]+ |
| 27 | 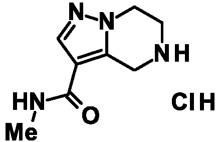 | MS(ESI) m/z:181.1 [M+H]+ |
| 28 | 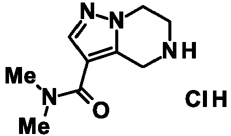 | MS(ESI) m/z:195.1 [M+H]+ |

### Reference Example 29:

### Production of tert-butyl 5-fluoro-1,2,3,4-tetrahydroisoquinoline-6-carboxylate

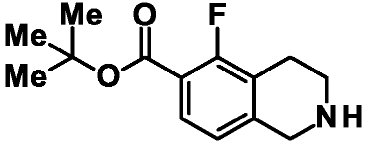

(1) 6-Bromo-5-fluoro-1,2,3,4-tetrahydroisoquinoline (1 g) was dissolved in 1,4-dioxane (15 ml) and water (7.5 ml). Sodium hydrogen carbonate (730 mg) and benzyl chloroformate (930 mg) were added thereto, and the mixture was stirred at room temperature for 5 hr. Water was added to the reaction solution, and the mixture was extracted with ethyl acetate. The organic layer was washed with saturated brine, dried, and concentrated. The residue was purified by silica gel column chromatography (solvent:hexane/ethyl acetate =100/0 - 65/35) to give benzyl 6-bromo-5-fluoro-3,4-dihydro-1H-isoquinoline-2-carboxylate (1.4 g; 84%) as a colorless oil.
   MS(ESI)m/z:363.8/365.8[M+H]⁺
(2) Benzyl 6-bromo-5-fluoro-3,4-dihydro-1H-isoquinoline-2-carboxylate (1.4 g) obtained in the aforementioned (1) was dissolved in acetonitrile (6 ml) and methanol (3 ml). Bis (tri-tert-butylphosphine)palladium (98 mg), molybdenum hexacarbonyl (1.1 g), and diazabicycloundecene (1.2 ml) were added thereto, and the reaction mixture was heated to 150°C and stirred for 30 min in a microwave reactor. The reaction solution was concentrated, and the residue was purified by silica gel column chromatography (solvent:hexane/ethyl acetate =100/0 - 75/25) to give 2-O-benzyl 6-O-methyl 5-fluoro-3,4-dihydro-1H-isoquinoline-2,6-dicarboxylate (992 mg; 66%) as a gray oil.
   MS(ESI)m/z:344.1[M+H]⁺
(3) 2-O-benzyl 6-O-methyl 5-fluoro-3,4-dihydro-1H-isoquinoline-2,6-dicarboxylate (992 mg) obtained in the aforementioned (2) was dissolved in methanol (8.5 ml), 4 M lithium hydroxide solution (2.5 ml) was added, and the mixture was stirred at room temperature for 3 hr. The reaction mixture was neutralized with 1 M hydrochloric acid and concentrated. The residue was azeotropically distilled with toluene, and dried under reduced pressure to give 5-fluoro-2-phenylmethoxycarbonyl-3,4-dihydro-1H-isoquinoline-6-carboxylic acid as a crude product (837 mg).
(4) The crude product (837 mg) of 5-fluoro-2-phenylmethoxycarbonyl-3,4-dihydro-1H-isoquinoline-6-carboxylic acid obtained in the aforementioned (3) was dissolved in toluene (8.5 ml), 1,1-di-tert-butoxy-N,N-dimethyl-methanamine (44 ml) was added, and the mixture was stirred at 120°C for 1 hr. The reaction mixture was concentrated, and the residue was purified by silica gel column chromatography to give 2-O-benzyl 6-O-tert-butyl 5-fluoro-3,4-dihydro-1H-isoquinoline-2,6-dicarboxylate (238 mg; 22%) as a colorless oil. MS(ESI)m/z:386.2[M+H]⁺
(5) 2-O-benzyl 6-O-tert-butyl 5-fluoro-3,4-dihydro-1H-isoquinoline-2,6-dicarboxylate (238 mg) obtained in the aforementioned (4) was dissolved in ethanol (2 ml), dihydroxy palladium (24 mg) was added, and the mixture was stirred under a hydrogen atmosphere at room temperature for 3 hr. The reaction solution was concentrated to give the title compound as a crude product (155 mg).

### Reference Examples 30 to 32:

Crude products of the compound described in the following Table 1-4 were obtained by treating the corresponding starting compound in the same manner as in Reference Example 29.

**[Table 1-4]**

| Reference Example No. | structural formula |
|---|---|
| 30 | 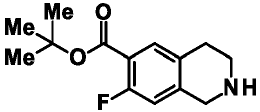 |
| 31 | 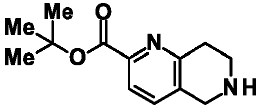 |
| 32 | 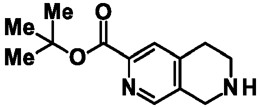 |

### Reference Example 33:

### Production of tert-butyl 3-[4-(trifluoromethyl)phenyl]-4,5,6,7-tetrahydropyrazolo[1,5-a]pyrazine-2-carboxylate

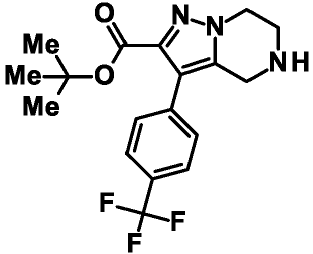

(1) To a mixture of ethyl 4,5,6,7-tetrahydropyrazolo[1,5-a]pyrazine-2-carboxylate (1.0 g) and dichloromethane (20 ml) were added triethylamine (0.8 ml) and benzyl chloroformate (0.76 ml), and the mixture was stirred at room temperature for 2 hr. To the reaction mixture was added saturated aqueous ammonium chloride solution, and the mixture was extracted with chloroform. The organic layer was dried over anhydrous magnesium sulfate and concentrated under reduced pressure to give 5-O-benzyl 2-O-ethyl 6,7-dihydro-4H-pyrazolo[1,5-a]pyrazine-2,5-dicarboxylate as a crude product.
(2) To a mixture of the crude product of 5-O-benzyl 2-O-ethyl 6,7-dihydro-4H-pyrazolo[1,5-a]pyrazine-2,5-dicarboxylate obtained in the aforementioned (1) and ethanol (20 ml) was added 4N aqueous sodium hydroxide solution (1.5 ml), and the mixture was stirred at room temperature for 2 hr. The reaction mixture was neutralized with 1N hydrochloric acid, and the mixture was extracted with ethyl acetate. The organic layer was washed with saturated brine, dried over anhydrous magnesium sulfate, and concentrated under reduced pressure to give 5-phenylmethoxycarbonyl-6,7-dihydro-4H-pyrazolo[1,5-a]pyrazine-2-carboxylic acid as a crude product.
(3) To a mixed solution of the crude product of 5-phenylmethoxycarbonyl-6,7-dihydro-4H-pyrazolo[1,5-a]pyrazine-2-carboxylic acid obtained in the aforementioned (2), and toluene (15 ml) was added N,N-dimethylformamide di-tert-butyl acetal (6 ml), and the mixture was stirred under a nitrogen atmosphere at 120°C for 30 min. After cooling to room temperature, the reaction mixture was concentrated under reduced pressure. The residue was purified by silica gel column chromatography (solvent:n-hexane/ethyl acetate =65/35 - 45/55) to give 5-O-benzyl 2-O-tert-butyl 6,7-dihydro-4H-pyrazolo[1,5-a]pyrazine-2,5-dicarboxylate (1.46 g; yield 80%) as a yellow oil. MS(ESI)m/z:302.2 [M-tBu+2H]+
(4) To a mixture of 5-O-benzyl 2-O-tert-butyl 6,7-dihydro-4H-pyrazolo[1,5-a]pyrazine-2,5-dicarboxylate (480 mg) obtained in the aforementioned (3) and acetonitrile (7 ml) was added N-bromosuccinimide (270 mg), and the mixture was stirred at room temperature for 2 hr. To the reaction mixture were added saturated sodium thiosulfate aqueous solution and 1N potassium carbonate aqueous solution, and the mixture was extracted with chloroform. The organic layer was dried over anhydrous magnesium sulfate, and concentrated under reduced pressure. The residue was purified by silica gel column chromatography (solvent:n-hexane/ethyl acetate =67/33 - 52/48) to give 5-O-benzyl 2-O-tert-butyl 3-bromo-6,7-dihydro-4H-pyrazolo[1,5-a]pyrazine-2,5-dicarboxylate (490 mg; yield 84%) as a colorless oil.
   MS(ESI)m/z: 380.1/382.1 [M-tBu+2H]+
(5) To a mixture of 5-O-benzyl 2-O-tert-butyl 3-bromo-6,7-dihydro-4H-pyrazolo[1,5-a]pyrazine-2,5-dicarboxylate (110 mg) obtained in the aforementioned (4) and 1,4-dioxane (1.8 ml) were added pinacol 4-(trifluoromethyl)phenylboronate (112 mg), [4-(di-tert-butylphosphino)-N,N-dimethylaniline-2-(2'-aminobiphenyl)]palladium(II) methanesulfonic acid (17 mg), and 2N-aqueous sodium carbonate solution (0.9 ml), and the reaction mixture was heated to 140°C and stirred for 1 hr in a microwave reactor. After cooling to room temperature, water was added to the reaction mixture, and the mixture was extracted with ethyl acetate. The organic layer was washed with saturated brine, dried over anhydrous magnesium sulfate, and concentrated under reduced pressure. The residue was purified by silica gel column chromatography (solvent:n-hexane/ethyl acetate =60/40 - 40/60) to give 5-O-benzyl 2-O-tert-butyl 3-[4-(trifluoromethyl)phenyl]-6,7-dihydro-4H-pyrazolo[1,5-a]pyrazine-2,5-dicarboxylate (123 mg; yield 97%) as a yellow oil.
   MS(ESI)m/z: 446.4 [M-tBu+2H]+
(6) To a mixture of 5-O-benzyl 2-O-tert-butyl 3-[4-(trifluoromethyl)phenyl]-6,7-dihydro-4H-pyrazolo[1,5-a]pyrazine-2,5-dicarboxylate (123 mg) obtained in the aforementioned (5), methanol (1.5 ml), and THF (0.5 ml) was added 20% palladium hydroxide (20 mg), and the mixture was stirred under a hydrogen atmosphere at 50°C for 2 hr. The reaction mixture was filtered through celite, and concentrated under reduced pressure to give a crude product (90 mg) of the title compound as a yellow oil.
   MS(ESI)m/z:368.06[M+H]⁺

### Reference Examples 34 to 36:

Crude products of the compounds described in the following Table 1-5 were obtained by treating the corresponding starting compounds in the same manner as in Reference Example 33.

**[Table 1-5]**

| Reference Example No. | structural formula |
|---|---|
| 34 | 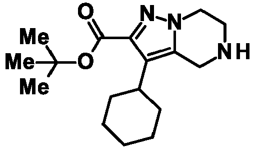 |
| 35 | 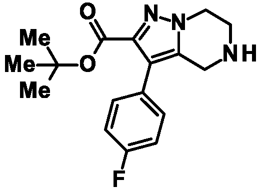 |
| 36 | 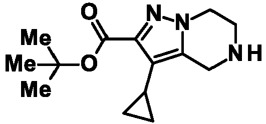 |

### Reference Example 37:

### Production of tert-butyl 3-bromo-4,5,6,7-tetrahydropyrazolo[1,5-a]pyrazine-2-carboxylate

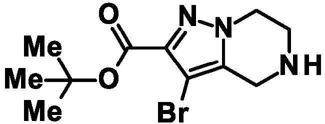

To a mixture of palladium acetate (12 mg) and dichloromethane (6 ml) were added triethylamine (0.03 ml), triethylsilane (0.2 ml) and 5-O-benzyl 2-O-tert-butyl 3-bromo-6,7-dihydro-4H-pyrazolo[1,5-a]pyrazine-2,5-dicarboxylate (490 mg) obtained in Reference Example 33 (4) under a nitrogen atmosphere, and the mixture was stirred at room temperature for 1 hr. The reaction mixture was filtered through celite, and concentrated under reduced pressure. The residue was purified by silica gel column chromatography (solvent:n-hexane/ethyl acetate =70/30 - 0/100) to give the title compound (90 mg; yield 27%) as a pale-yellow solid.
MS(ESI)m/z:246.1/248.1 [M-tBu+2H]+

### Reference Example 38:

### Production of 7-bromo-1,2,3,4-tetrahydropyrido[1,2-a]pyrazin-6-one hydrochloride

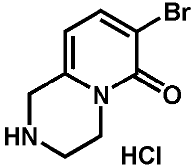

(1) To a solution of tert-butyl 6-oxo-3,4-dihydro-1H-pyrido[1,2-a]pyrazine-2-carboxylate (182 mg) in dichloromethane (1.8 ml) was added N-bromosuccinimide (65 mg), and the mixture was stirred at room temperature for 3 hr. To the reaction mixture was added aqueous sodium thiosulfate solution, and the mixture was extracted with ethyl acetate. The organic layer was washed with saturated brine, dried over anhydrous magnesium sulfate, and concentrated under reduced pressure. The residue was purified by silica gel column chromatography (solvent:n-hexane/ethyl acetate =100/0 - 40/60) to give tert-butyl 7-bromo-6-oxo-3,4-dihydro-1H-pyrido[1,2-a]pyrazine-2-carboxylate (42.2 mg; yield 18%) as a colorless amorphous.
   MS(ESI)m/z:329.2/331.2[M+H]⁺
(2) To tert-butyl 7-bromo-6-oxo-3,4-dihydro-1H-pyrido[1,2-a]pyrazine-2-carboxylate (42.2 mg) obtained in the aforementioned (1) was added 4N hydrochloric acid-ethyl acetate solution (0.8 ml), and the mixture was stirred at room temperature for 18 hr. The reaction mixture was concentrated under reduced pressure to give the title compound (33.2 mg; yield 98%) as a white solid.
   MS(ESI)m/z:229.0/231.0[M+H]⁺

### Reference Example 39:

### Production of 6-bromo-1-(methoxymethyl)-1,2,3,4-tetrahydroisoquinoline

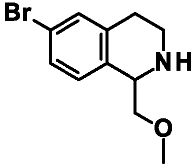

(1) To a solution of 2-(3-bromophenyl)ethanamine (1 g), 2-methoxyacetic acid (540 mg), and HATU (2.4 g) in dichloromethane (17 ml) was added triethylamine (1.4 ml), and the mixture was stirred at room temperature for 3 hr. To the reaction mixture was added water, and the mixture was extracted with ethyl acetate. The organic layer was washed successively with 1N hydrochloric acid and 1N aqueous sodium hydroxide solution, dried over anhydrous magnesium sulfate, and concentrated under reduced pressure to give a crude product (1.36 g) of N-[2-(3-bromophenyl)ethyl]-2-methoxyacetamide as a yellow oil.
(2) To a solution of the crude product (1.36 g) of N-[2-(3-bromophenyl)ethyl]-2-methoxyacetamide obtained in the aforementioned (1) in toluene (17 ml) was added phosphorus oxide (V), and the mixture was stirred at 140°C for 2 hr. After cooling to 0°C, water was added to the reaction mixture, and the mixture was extracted with ethyl acetate. The organic layer was washed with saturated brine, dried over anhydrous magnesium sulfate, and concentrated under reduced pressure to give a crude product (1.27 g) of 6-bromo-1-(methoxymethyl)-3,4-dihydroisoquinoline as a brown oil.
(3) To a solution of the crude product (1.27 g) of 6-bromo-1-(methoxymethyl)-3,4-dihydroisoquinoline obtained in the aforementioned (2) in methanol (17 ml) was added sodium borohydride (756 mg), and the mixture was stirred at room temperature for 18 hr. To the reaction mixture was added water, and the mixture was extracted with ethyl acetate. The organic layer was washed with saturated brine, dried over anhydrous magnesium sulfate, and concentrated under reduced pressure. The residue was purified by silica gel column chromatography (solvent:n-hexane/ethyl acetate=100/0 - 0/100) to give the title compound (64.1 mg; yield 5.0%) as an orange oil.
   MS(ESI)m/z:256.1/258.1[M+H]⁺

### Example 1:

### Production of (E)-N-[2-oxo-2-(3-oxopiperazin-1-yl)ethyl]-3-[4-(trifluoromethyl)phenyl]prop-2-enamide (compound (I-1))

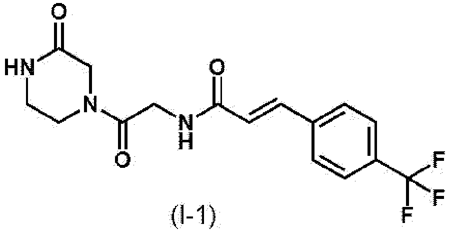

To a solution of 2-[[(E)-3-[4-(trifluoromethyl)phenyl]prop-2-enoyl]amino]acetic acid (2.00 g) in DMF (25 ml) were added piperazin-2-one (770 mg), N,N-diisopropylethylamine (1.5 ml), and HATU (3.36 g) at room temperature, and the reaction mixture was stirred at room temperature for 2 hr. To the reaction solution was added saturated aqueous ammonium chloride solution, the mixture was stirred for a while at room temperature, and the aqueous layer was extracted with ethyl acetate. The organic layer was washed with saturated brine, dried over anhydrous magnesium sulfate, and filtered through a cotton plug. The solvent was evaporated under reduced pressure, and the obtained residue was purified by silica gel column chromatography (solvent:chloroform/methanol=100/0 - 96/4). Fractions containing the objective compound were combined and concentrated, and the residue was washed by suspending in diisopropyl ether and ethyl acetate, and dried under reduced pressure at 60°C to give the title compound (1.90 g; yield 73%) as a white solid.
MS(ESI)m/z:356.3[M+H]⁺

### Examples 2 to 102:

The compounds described in the following Table 2-1 to Table 2-21 were obtained by treating the corresponding starting compounds in the same manner as in Example 1.

**[Table 2-1]**

| Example No. (compound No.) | structural formula | instrument analysis data |
|---|---|---|
| 2 (I-2) | 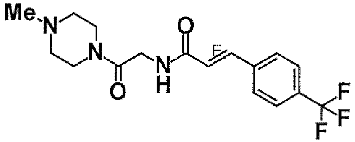 | MS (ESI) m/z:356.2 [M+H]+ |
| 3 (I-3) | 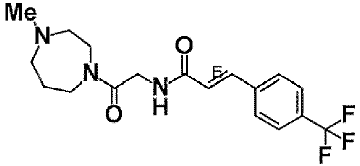 | MS(ESI) m/z:370.3 [M+H]+ |
| 4 (I-4) | 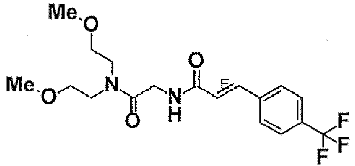 | MS(ESI) m/z:389.1 [M+H]+ |
| 5 (I-5) | 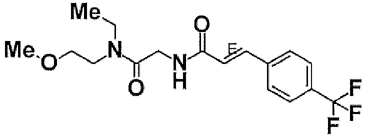 | MS(ESI) m/z:359.1 [M+H]+ |
| 6 (I-6) | 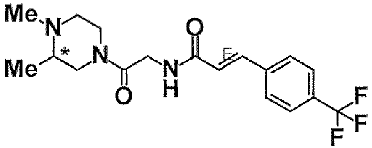 | MS(ESI) m/z:370. 5 [M+H]+ |

**[Table 2-2]**

| Example No. (compound No.) | structural formula | instrument analysis data |
|---|---|---|
| 7 (I-7) | 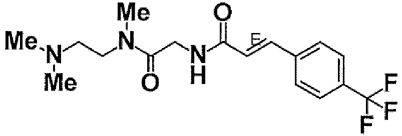 | MS(ESI) m/z:358.3 [M+H] + |
| 8 (I-8) | 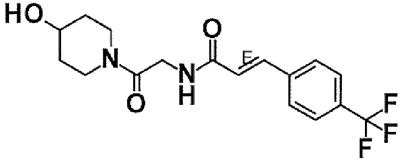 | MS(ESI) m/z:357.2 [M+H]+ |
| 9 (I-9) | 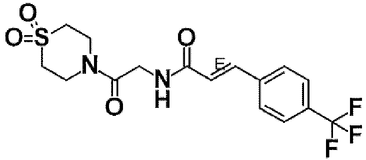 | MS(ESI) m/z:391.3 [M+H]+ |
| 10 (I-10) | 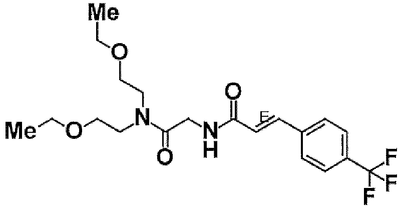 | MS(ESI) m/z:417.4 [M+H]+ |
| 11 (I-11) | 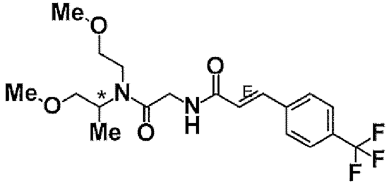 | MS (ESI) m/z:403.0 [M+H]+ |

**[Table 2-3]**

| Example No. (compound No.) | structural formula | instrument analysis data |
|---|---|---|
| 12 (I-12) | 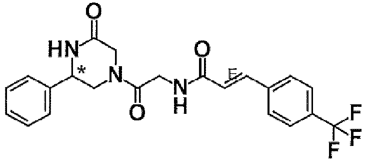 | MS(ESI) m/z:432.1 [M+H]+ |
| 13 (I-13) | 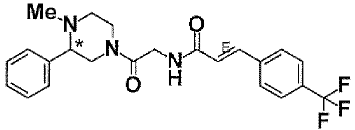 | MS(ESI) m/z:432.1 [M+H]+ |
| 14 (I-14) | 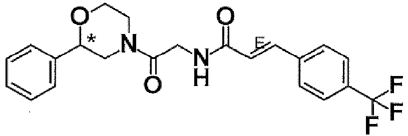 | MS(ESI) m/z:419.1 [M+H]+ |
| 15 (I-15) | 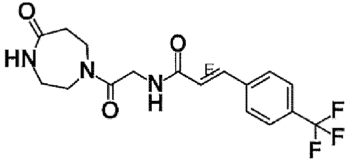 | MS(ESI) m/z:370.1 [M+H]+ |
| 16 (I-16) | 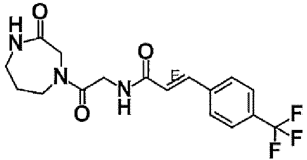 | MS(ESI) m/z:370.1 [M+H]+ |

**[Table 2-4]**

| Example No. (compound No.) | structural formula | instrument analysis data |
|---|---|---|
| 17 (I-17) | 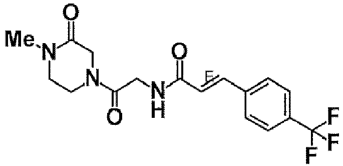 | MS(ESI) m/z:370.1 [M+H]+ |
| 18 (I-18) | 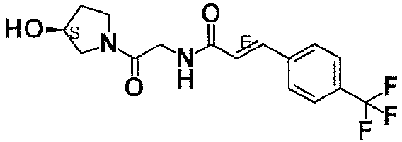 | MS(ESI) m/z:343.1 [M+H]+ |
| 19 (I-19) | 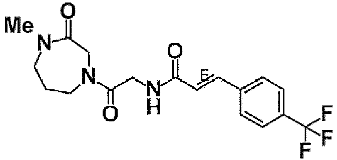 | MS(ESI) m/z:384.0 [M+H]+ |
| 20 (I-20) | 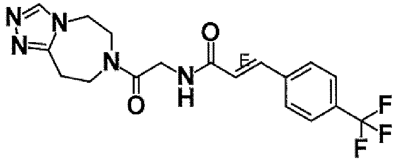 | MS(ESI) m/z:394.1 [M+H]+ |
| 21 (I-21) | 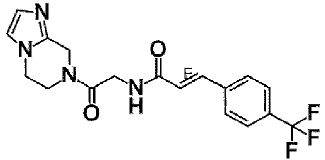 | MS(ESI) m/z:379.3 [M+H]+ |

**[Table 2-5]**

| Example No. (compound No.) | structural formula | instrument analysis data |
|---|---|---|
| 22 (I-22) | 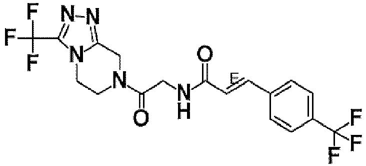 | MS (ESI) m/z:448. 2 [M+H]+ |
| 23 (I-23) | 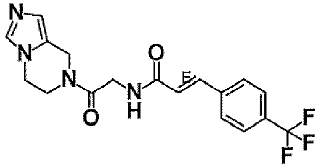 | MS(ESI) m/z:379.2 [M+H]+ |
| 24 (I-24) | 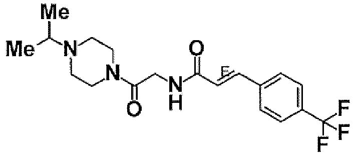 | MS(ESI) m/z:384.1 [M+H]+ |
| 25 (I-25) | 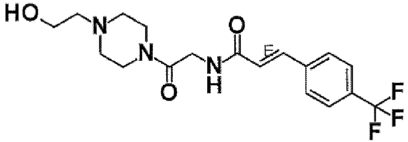 | MS(ESI) m/z:386.1 [M+H]+ |
| 26 (I-26) | 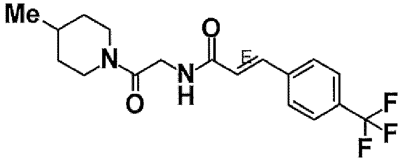 | MS(ESI) m/z:355.1 [M+H]+ |

**[Table 2-6]**

| Example No. (compound No.) | structural formula | instrument analysis data |
|---|---|---|
| 27 (I-27) | 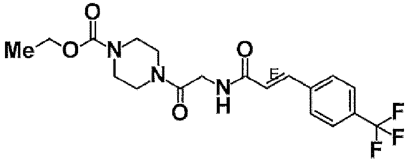 | MS(ESI) m/z:414.1 [M+H]+ |
| 28 (I-28) | 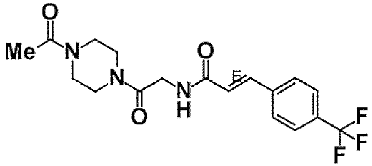 | MS(ESI) m/z:384.1 [M+H]+ |
| 29 (I-29) | 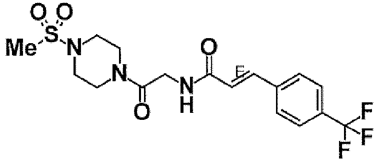 | MS(ESI) m/z:420.2 [M+H]+ |
| 30 (I-30) | 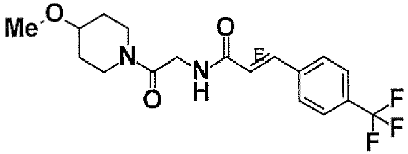 | MS(ESI) m/z:371.2 [M+H]+ |
| 31 (I-31) | 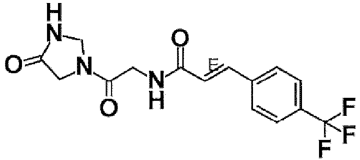 | MS(ESI) m/z:342.1 [M+H]+ |

**[Table 2-7]**

| Example No. (compound No.) | structural formula | instrument analysis data |
|---|---|---|
| 32 (I-32) | 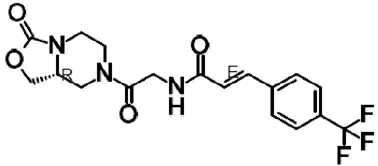 | MS(ESI) m/z:398.1 [M+H]+ |
| 33 (I-33) | 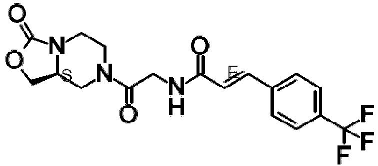 | MS (ESI) m/z:398.1 [M+H]+ |
| 34 (I-34) | 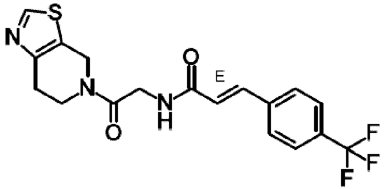 | MS(ESI) m/z:396.0 [M+H]+ |
| 35 (I-35) | 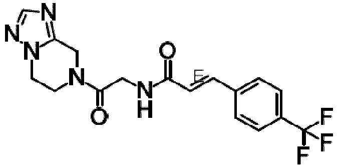 | MS(ESI) m/z:378.1 [M-H]- |
| 36 (I-36) | 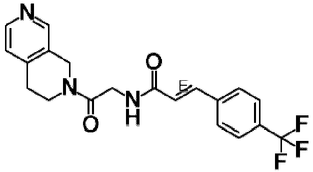 | MS (ESI) m/z:390.2 [M+H]+ |

**[Table 2-8]**

| Example No. (compound No.) | structural formula | instrument analysis data |
|---|---|---|
| 37 (I-37) | 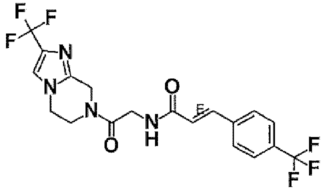 | MS(ESI) m/z:447. 2 [M+H]+ |
| 38 (I-38) | 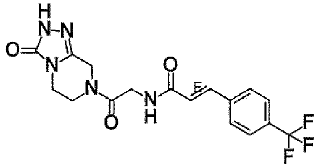 | MS(ESI) m/z:394.2 [M-H]- |
| 39 (I-39) | 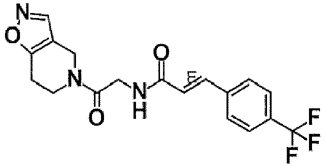 | MS(ESI) m/z:378.2 [M-H]- |
| 40 (I-40) | 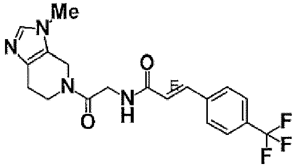 | MS(ESI) m/z:393.2 [M+H]+ |
| 41 (I-41) | 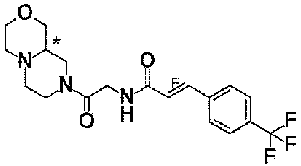 | MS(ESI) m/z:398.2 [M+H]+ |

**[Table 2-9]**

| Example No. (compound No.) | structural formula | instrument analysis data |
|---|---|---|
| 42 (I-42) | 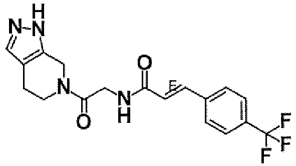 | MS(ESI) m/z:379.2 [M+H] + |
| 43 (I-43) | 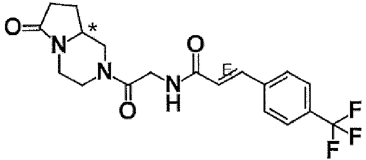 | MS(ESI) m/z:396.1 [M+H]+ |
| 44 (I-44) | 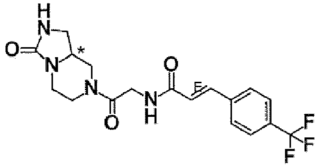 | MS(ESI) m/z:397.1 [M+H] + |
| 45 (I-45) | 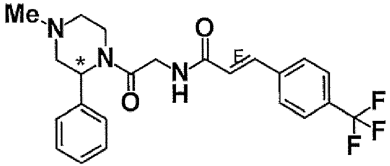 | MS(ESI) m/z:432.1 [M+H]+ |
| 46 (I-46) | 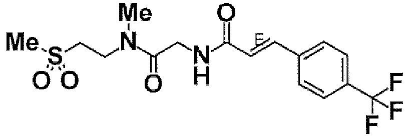 | MS(ESI) m/z:393.4 [M+H]+ |

**[Table 2-10]**

| Example No. (compound No.) | structural formula | instrument analysis data |
|---|---|---|
| 47 (I-47) | 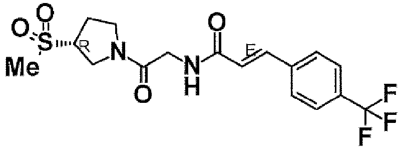 | MS(ESI) m/z:405.1 [M+H]+ |
| 48 (I-48) | 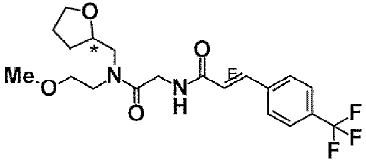 | MS (ESI) m/z:415.6 [M+H]+ |
| 49 (I-49) | 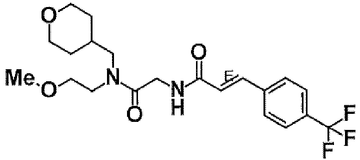 | MS(ESI) m/z:429. 3 [M+H]+ |
| 50 (I-50) | 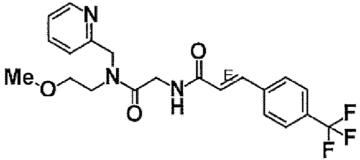 | MS(ESI) m/z:422.6 [M+H]+ |
| 51 (I-51) | 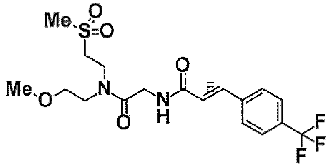 | MS(ESI) m/z:437.3 [M+H] + |

**[Table 2-11]**

| Example No. (compound No.) | structural formula | instrument analysis data |
|---|---|---|
| 52 (I-52) | 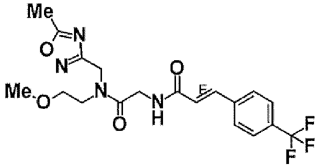 | MS(ESI) m/z:427.3 [M+H]+ |
| 53 (I-53) | 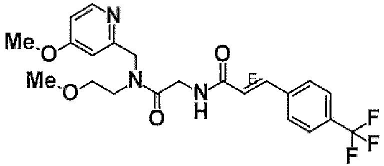 | MS(ESI) m/z:452.5 [M+H]+ |
| 54 (I-54) | 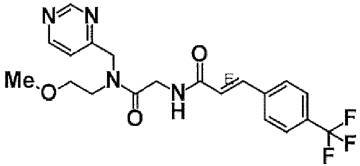 | MS(ESI) m/z:423.2 [M+H] + |
| 55 (I-55) | 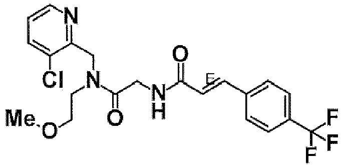 | MS(ESI) m/z:454.2/ 456.2 [M-H]- |
| 56 (I-56) | 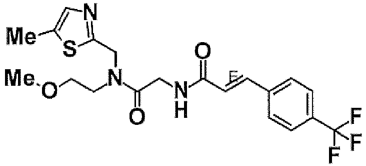 | MS(ESI) m/z:442.2 [M+H]+ |

**[Table 2-12]**

| Example No. (compound No.) | structural formula | instrument analysis data |
|---|---|---|
| 57 (I-57) | 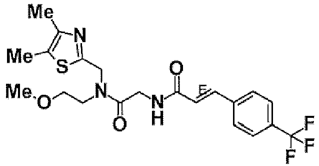 | MS(ESI) m/z:456.3 [M+H]+ |
| 58 (I-58) | 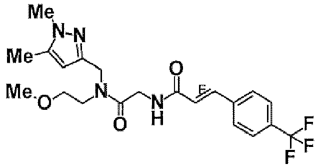 | MS(ESI) m/z:439.2 [M+H]+ |
| 59 (I-59) | 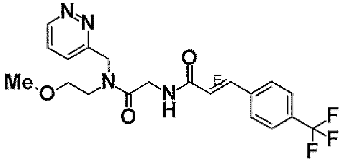 | MS(ESI) m/z:423.2 [M+H]+ |
| 60 (I-60) | 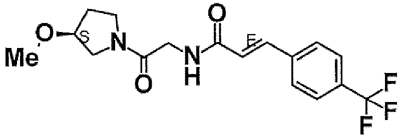 | MS(ESI) m/z:357.1 [M+H]+ |
| 61 (I-61) | 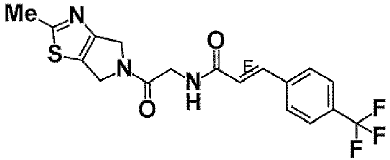 | MS(ESI) m/z:396.0 [M+H]+ |

**[Table 2-13]**

| Example No. (compound No.) | structural formula | instrument analysis data |
|---|---|---|
| 62 (I-62) | 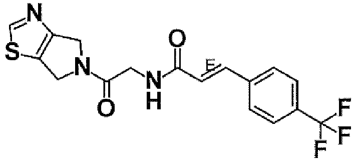 | MS (ESI) m/z:382.0 [M+H]+ |
| 63 (I-63) | 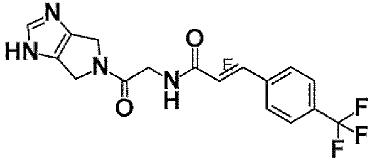 | MS(ESI) m/z:365.1 [M+H]+ |
| 64 (I-64) | 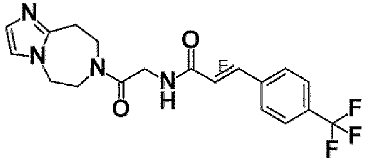 | MS(ESI) m/z:391.3 [M-H]- |
| 65 (I-65) | 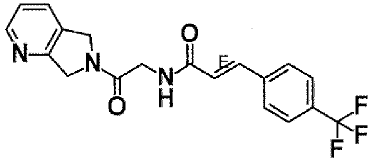 | MS (ESI) m/z:376. 2 [M+H]+ |
| 66 (I-66) | 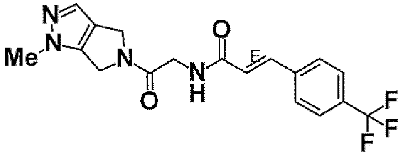 | MS(ESI) m/z:379.3 [M+H]+ |

**[Table 2-14]**

| Example No. (compound No.) | structural formula | instrument analysis data |
|---|---|---|
| 67 (I-67) | 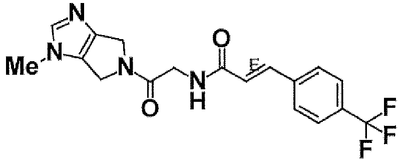 | MS(ESI) m/z:379.2 [M+H]+ |
| 68 (I-68) | 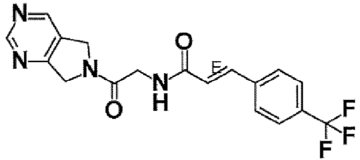 | MS(ESI) m/z:377.1 [M+H]+ |
| 69 (I-69) | 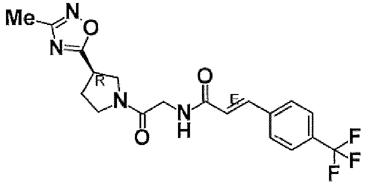 | MS (ESI) m/z:409.3 [M+H]+ |
| 70 (I-70) | 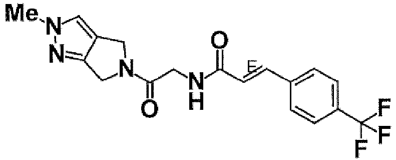 | MS(ESI) m/z:379.2 [M+H]+ |
| 71 (I-71) | 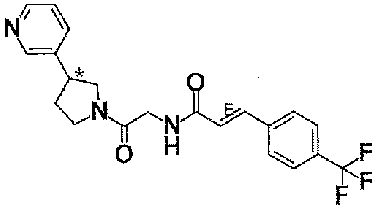 | MS(ESI) m/z:402.2 [M-H]- |

**[Table 2-15]**

| Example No. (compound No.) | structural formula | instrument analysis data |
|---|---|---|
| 72 (I-72) | 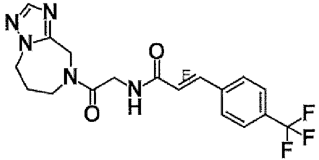 | MS(ESI) m/z:394.2 [M+H]+ |
| 73 (I-73) | 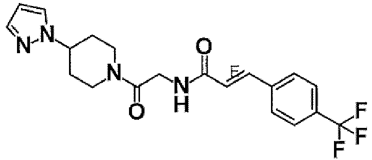 | MS(ESI) m/z:407.2 [M+H]+ |
| 74 (I-74) | 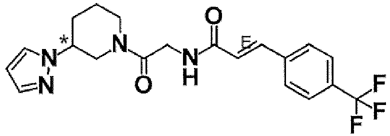 | MS(ESI) m/z:407.2 [M+H]+ |
| 75 (I-75) | 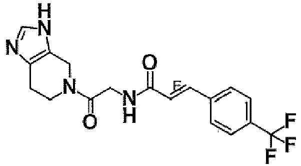 | MS(ESI) m/z:379.1 [M+H]+ |
| 76 (I-76) | 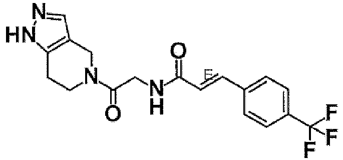 | MS(ESI) m/z:379.3 [M+H]+ |

**[Table 2-16]**

| Example No. (compound No.) | structural formula | instrument analysis data |
|---|---|---|
| 77 (I-77) | 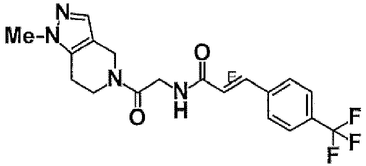 | MS(ESI) m/z:393.2 [M+H]+ |
| 78 (I-78) | 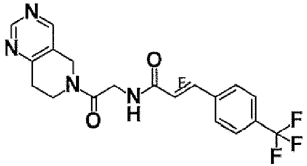 | MS(ESI) m/z:391.1 [M+H]+ |
| 79 (I-79) | 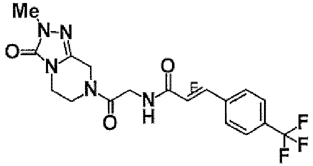 | MS(ESI) m/z:410.2 [M+H]+ |
| 80 (I-80) | 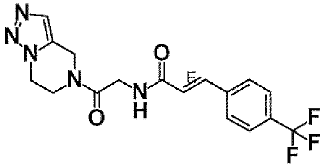 | MS (ESI) m/z:380.1 [M+H]+ |
| 81 (I-81) | 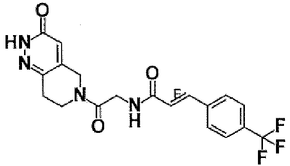 | MS(ESI) m/z:407.2 [M+H]+ |

**[Table 2-17]**

| Example No. (compound No.) | structural formula | instrument analysis data |
|---|---|---|
| 82 (I-82) | 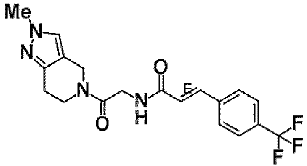 | MS(ESI) m/z:393.2 [M+H]+ |
| 83 (I-83) | 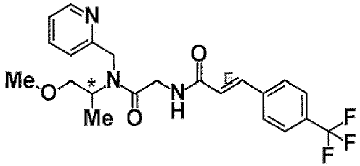 | MS (ESI) m/z:436. 3 [M+H]+ |
| 84 (I-84) | 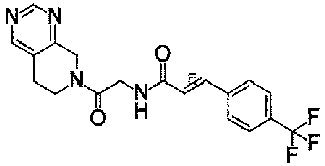 | MS (ESI) m/z:391.2 [M+H]+ |
| 85 (I-85) | 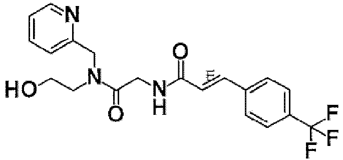 | MS(ESI) m/z:408.2 [M+H]+ |
| 86 (I-86) | 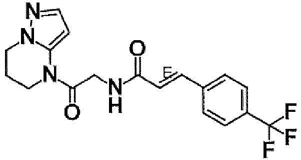 | MS(ESI) m/z:379.2 [M+H]+ |

**[Table 2-18]**

| Example No. (compound No.) | structural formula | instrument analysis data |
|---|---|---|
| 87 (I-87) | 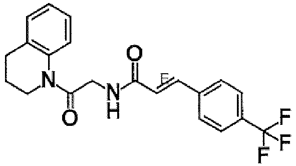 | MS(ESI) m/z:389.2 [M+H]+ |
| 88 (I-88) | 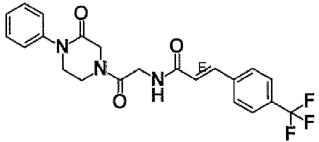 | MS(ESI) m/z:432.2 [M+H]+ |
| 89 (I-89) | 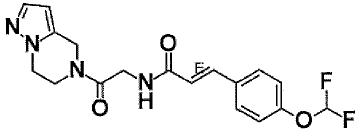 | MS(ESI) m/z:377.5 [M+H]+ |
| 90 (I-90) | 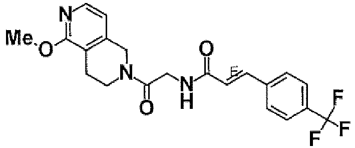 | MS(ESI) m/z:420.2 [M+H]+ |
| 91 (I-91) | 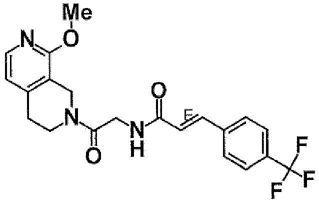 | MS(ESI) m/z:420.2 [M+H]+ |

**[Table 2-19]**

| Example No. (compound No.) | structural formula | instrument analysis data |
|---|---|---|
| 92 (I-92) | 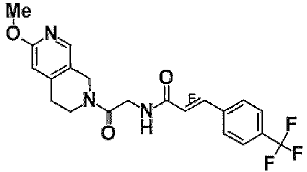 | MS (ESI) m/z:420.3 [M+H]+ |
| 93 (I-93) | 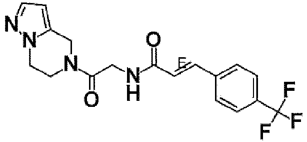 | MS(ESI) m/z:379.1 [M+H]+ |
| 94 (I-94) | 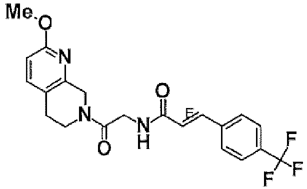 | MS(ESI) m/z:420.2 [M+H]+ |
| 95 (I-95) | 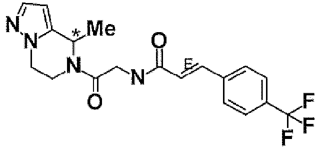 | MS(ESI) m/z:437.3 [M+H]+ |
| 96 (I-96) | 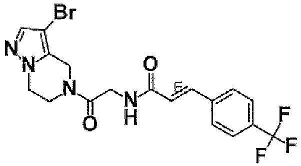 | MS(ESI) m/z:455. 1, 457.1 [M-H]- |

**[Table 2-20]**

| Example No. (compound No.) | structural formula | instrument analysis data |
|---|---|---|
| 97 (I-97) | 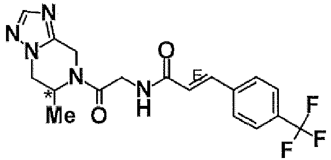 | MS(ESI) m/z:394.1 [M+H]+ |
| 98 (I-98) | 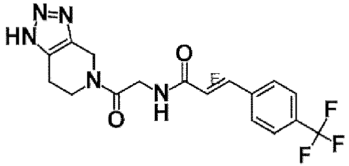 | MS(ESI) m/z:380.1 [M+H]+ |
| 99 (I-99) | 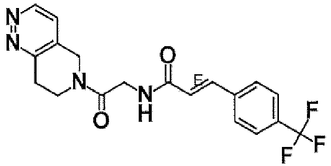 | MS(ESI) m/z:391.2 [M+H]+ |
| 100 (I-100) | 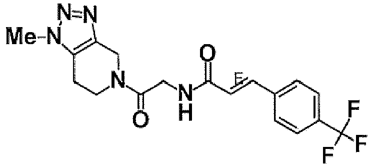 | MS(ESI) m/z:394.2 [M+H]+ |
| 101 (I-101) | 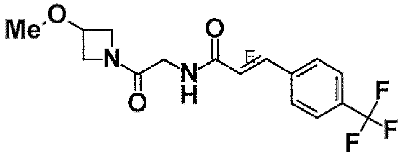 | MS (ESI) m/z:343. 0 [M+H]+ |

**[Table 2-21]**

| Example No. (compound No.) | structural formula | instrument analysis data |
|---|---|---|
| 102 (I-102) | 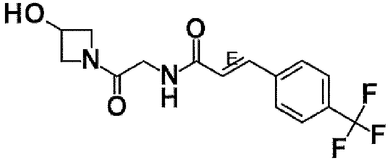 | MS(ESI) m/z:329.0 [M+H]+ |

### Example 103:

### Production of (E)-N-[2-(4-methylpiperazin-1-yl)-2-oxoethyl]-3-(4-phenylphenyl)prop-2-enamide (compound (1-103))

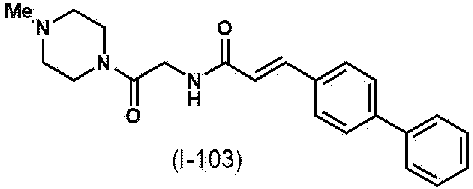

To a solution of (E)-3-(4-phenylphenyl)prop-2-ene carboxylic acid (50 mg), 2-amino-1-(4-methylpiperazin-1-yl)ethenone hydrochloride (56 mg), and HATU (110 mg) in DMF (3 ml) was added N,N-diisopropylethylamine (0.23 ml), and the reaction mixture was stirred at room temperature overnight. To the reaction mixture were added saturated aqueous sodium hydrogen carbonate solution and chloroform and the mixture was stirred and filtered through a phase separator. The filtrate was concentrated under reduced pressure, and the residue was purified by reversed-phase HPLC (10 mmol/l aqueous ammonium carbonate solution/acetonitrile=70/30 - 40/60) to give the title compound (62 mg; yield 77%) as a pale-yellow solid. MS(ESI)m/z:364.4[M+H]⁺

### Examples 104 to 118:

The compounds described in the following Table 3-1 to Table 3-3 were obtained by treating the corresponding starting compounds in the same manner as in Example 103.

**[Table 3-1]**

| Example No. (compound No.) | structural formula | instrument analysis data |
|---|---|---|
| 104 (I-104) | 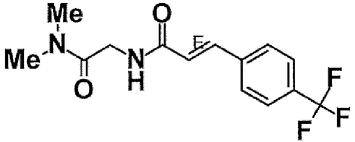 | MS (ESI) m/z:301.0 [M+H]+ |
| 105 (I-105) | 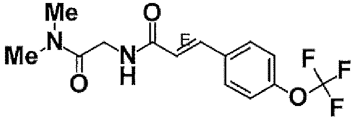 | MS(ESI) m/z:317.0 [M+H]+ |
| 106 (I-106) | 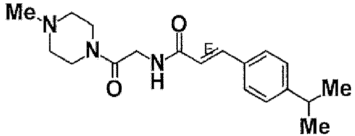 | MS(ESI) m/z:330.4 [M+H]+ |
| 107 (I-107) | 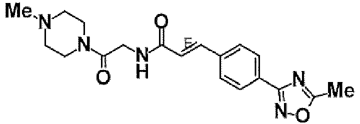 | MS(ESI) m/z:370.3 [M+H] + |

**[Table 3-2]**

| Example No. (compound No.) | structural formula | instrument analysis data |
|---|---|---|
| 108 (I-108) | 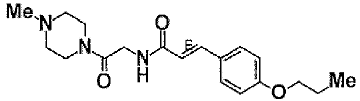 | MS(ESI) m/z:346.2 [M+H]+ |
| 109 (I-109) | 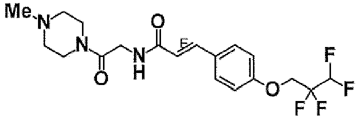 | MS (ESI) m/z:418.5 [M+H] + |
| 110 (I-110) | 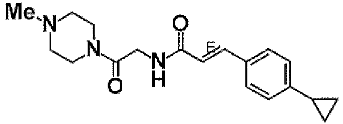 | MS(ESI) m/z:328.0 [M+H[+ |
| 111 (I-111) | 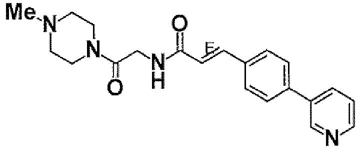 | MS(ESI) m/z:365.2 [M+H] + |
| 112 (I-112) | 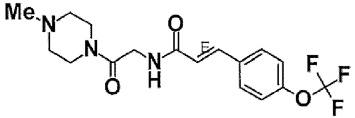 | MS(ESI) m/z:372.3 [M+H]+ |
| 113 (I-113) | 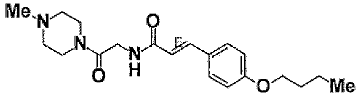 | MS(ESI) m/z:360.4 [M+H]+ |

**[Table 3-3]**

| Example No. (compound No.) | structural formula | instrument analysis data |
|---|---|---|
| 114 (I-114) | 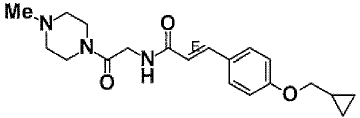 | MS(ESI) m/z:358.1 [M+H]+ |
| 115 (I-115) | 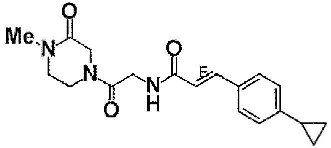 | MS(ESI) m/z:342.2 [M+H]+ |
| 116 (I-116) | 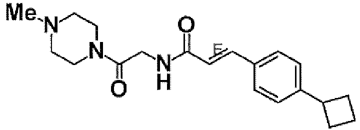 | MS(ESI) m/z:342.1 [M+H]+ |
| 117 (I-117) | 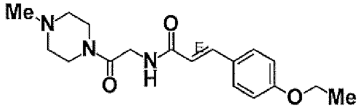 | MS(ESI) m/z:332.2 [M+H]+ |
| 118 (I-118) | 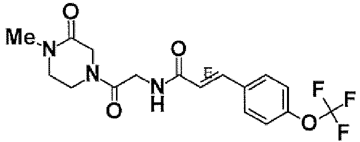 | MS(ESI) m/z:386.1 [M+H]+ |

### Example 119:

### Production of (E)-N-[2-(2,4-dimethylpiperazin-1-yl)-2-oxoethyl]-3-[4-(trifluoromethyl)phenyl]prop-2-enamide (compound (I-119))

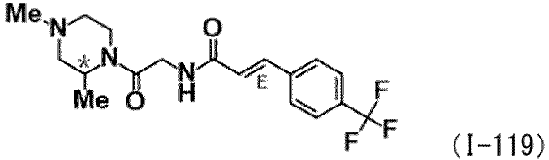

(1) To a solution of tert-butyl 3-methyl-4-[2-[[(E)-3-[4-(trifluoromethyl)phenyl]prop-2-enoyl]amino]acetyl]piperazine carboxylate (453 mg) obtained in the below-mentioned Example 152 in acetonitrile (2 ml) was added hydrochloric acid (4 mol/l ethyl acetate solution) (3 ml), and the reaction mixture was stirred at room temperature for 2 hr. The solvent was evaporated under reduced pressure to give a crude product (400 mg) of (E)-N-[2-(2-methylpiperazin-1-yl)-2-oxoethyl]-3-[4-(trifluoromethyl)phenyl]prop-2-enamide hydrochloride as an orange amorphous.
(2) To a solution of the crude product (400 mg) of (E)-N-[2-(2-methylpiperazin-1-yl)-2-oxoethyl]-3-[4-(trifluoromethyl)phenyl]prop-2-enamide hydrochloride obtained in the aforementioned (1) in dichloromethane (3.5 ml) were added formaldehyde (37 wt% methanol solution) (150 µl), acetic acid (500 µl), and sodium triacetoxyborohydride (330 mg), and the reaction mixture was stirred at room temperature for 1 hr. To the reaction mixture was added 0.5 mol/l aqueous NaOH solution, and the aqueous layer was extracted with chloroform. The organic layer was dried over anhydrous magnesium sulfate, and filtered through a cotton plug. The solvent was evaporated under reduced pressure, and the obtained residue was purified by silica gel column chromatography (solvent:chloroform/methanol=98/2 - 91/9) to give the title compound (240 mg; yield 64%) as a white amorphous. MS(ESI)m/z:370.2[M+H]⁺

### Examples 120 to 122:

The compounds described in the following Table 4 were obtained by treating the corresponding compounds of Examples 153 to 155 (compound (1-153) to compound (1-155)) in the same manner as in Example 119.

**[Table 4]**

| Example No. (compound No.) | structural formula | instrument analysis data |
|---|---|---|
| 120 (I-120) | 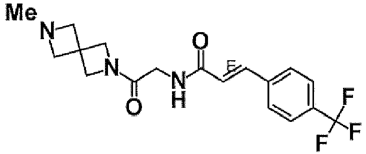 | MS(ESI) m/z:368.0 [M+H]+ |
| 121 (I-121) | 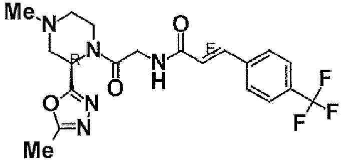 | MS(ESI) m/z:438.1 [M+H]+ |
| 122 (I-122) | 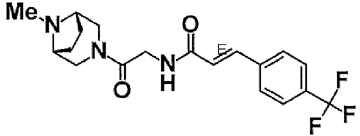 | MS (ESI) m/z:382.1 [M+H]+ |

### Example 123:

### Production of (2S)-4-methyl-1-[2-[[(E)-3-[4-(trifluoromethyl)phenyl]prop-2-enoyl]amino]acetyl]piperazine-2-carboxylic acid (compound (1-123))

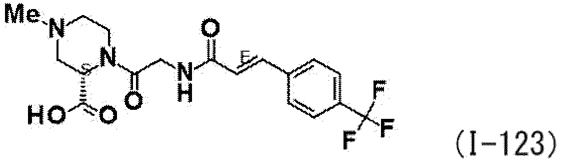

To a solution of methyl (2S)-4-methyl-1-[2-[[(E)-3-[4-(trifluoromethyl)phenyl]prop-2-enoyl]amino]acetyl]piperazine-2-carboxylate (compound (1-156)) (318 mg) obtained in the below-mentioned Example 156 in methanol (1.6 ml) was added 1 mol/l aqueous sodium hydroxide solution (1.6 ml), and the reaction mixture was stirred at room temperature for 3 hr. To the reaction mixture were added 1 mol/l hydrochloric acid (1.6 ml) and 1 mol/l aqueous sodium hydroxide solution (1 ml), and the solution was purified by reversed-phase HPLC (solvent:10 mmol/l aqueous ammonium carbonate solution/acetonitrile=80/20 - 65/35) to give the title compound (169 mg; yield 55%) as a white powder.
MS(ESI)m/z:400.1[M+H]⁺

### Examples 124 and 125:

The compounds described in the following Table 5 were obtained by treating the below-mentioned compounds of Examples 157 and 158 (compound (1-157) and compound (1-158)) in the same manner as in Example 123.

**[Table 5]**

| Example No. (compound No.) | structural formula | instrument analysis data |
|---|---|---|
| 124 (I-124) | 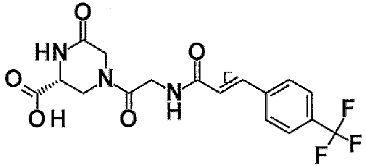 | MS(ESI) m/z:398.3 [M-H]- |
| 125 (I-125) | 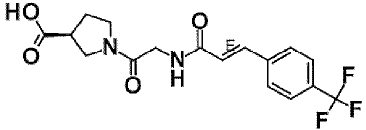 | MS (ESI) m/z:371.1 [M+H]+ |

### Example 126:

### Production of (E)-3-[4-(4-fluorophenyl)phenyl]-N-[2-(4-methylpiperazin-1-yl)-2-oxoethyl]prop-2-enamide (compound (I-126))

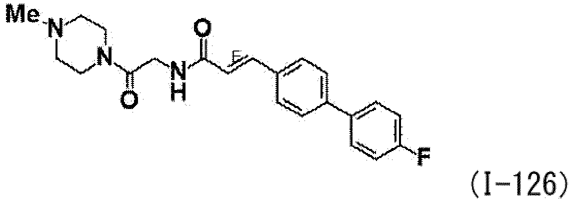

(E)-3-(4-bromophenyl)-N-[2-(4-methylpiperazin-1-yl)-2-oxoethyl]prop-2-enamide (100 mg), (4-fluorophenyl)boronic acid (57 mg), dichlorobis(tricyclohexylphosphine)palladium(II) (20 mg), and tripotassium phosphate (174 mg) were suspended in toluene (3 ml) and water (0.3 ml), and the reaction mixture was stirred at 110°C for 5 hr. The reaction mixture was allowed to cool and filtered through celite while washing with ethyl acetate, and the filtrate was concentrated under reduced pressure. The residue was purified by reversed-phase HPLC (solvent:10 mmol/l aqueous ammonium carbonate solution/acetonitrile=70/30 - 40/60) to give the title compound (91 mg; yield 87%) as a white solid.
MS(ESI)m/z:382.2[M+H]⁺

### Examples 127 and 128:

The compounds described in the following Table 6 were obtained by treating the corresponding starting compounds in the same manner as in Example 126.

**[Table 6]**

| Example No. (compound No.) | structural formula | instrument analysis data |
|---|---|---|
| 127 (I-127) | 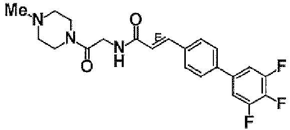 | MS(ESI) m/z:418.2 [M+H]+ |
| 128 (I-128) | 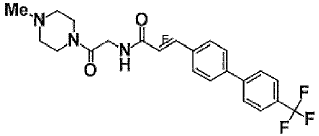 | MS(ESI) m/z : 432. 2 [M+H]+ |

### Example 129:

### Production of (E)-N-[2-[2-(1-hydroxy-1-methylethyl)-6,7-dihydro-4H-pyrazolo[1,5-a]pyrazin-5-yl]-2-oxoethyl]-3-[4-(trifluoromethyl)phenyl]prop-2-enamide (compound (1-129))

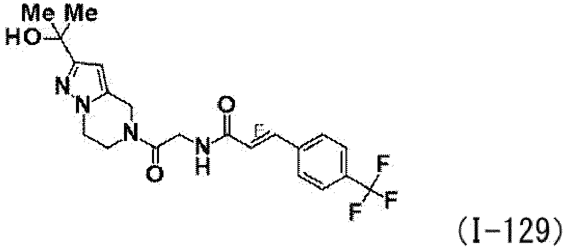

(1) To a solution of O5-benzyl O2-ethyl 6,7-dihydro-4H-pyrazolo[1,5-a]pyrazine-2,5-dicarboxylate (150 mg) in THF (5 ml) was added at -78°C methylmagnesium bromide (3 mol/l THF solution) (0.5 ml), and the mixture was stirred at -78°C for 1 hr and then stirred overnight while allowing to gradually warm to room temperature. Water was added to the reaction solution and the mixture was concentrated under reduced pressure to give 2-(1-hydroxy-1-methyl-ethyl)-6,7-dihydro-4H-pyrazolo[1,5-a]pyrazine as a crude product.
(2) DMF (5 ml), [[(E)-3-[4-(trifluoromethyl)phenyl]prop-2-enoyl]amino]acetic acid (130 mg), HATU (226 mg), and N,N-diisopropylethylamine (0.4 ml) were sequentially added to the crude product of 2-(1-hydroxy-1-methyl-ethyl)-6,7-dihydro-4H-pyrazolo[1,5-a]pyrazine obtained in the aforementioned (1), and the reaction mixture was stirred at room temperature overnight. To the reaction solution was added saturated aqueous sodium hydrogen carbonate solution, and the aqueous layer was partitioned with ethyl acetate. The combined organic layer was washed with saturated brine, dried over sodium sulfate, and concentrated under reduced pressure. The obtained residue was purified by silica gel column chromatography (solvent:ethyl acetate/methanol=100/0 - 95/5) to give the title compound (25 mg; yield 130) as a colorless solid.
   MS(ESI)m/z:437.3[M+H]⁺

### Example 130:

### Production of 4-methoxy-3-[2-methoxyethyl-[2-[[(E)-3-[4-(trifluoromethyl)phenyl]prop-2-enoyl]amino]acetyl]amino]-N-methylbutanamide (compound (1-130))

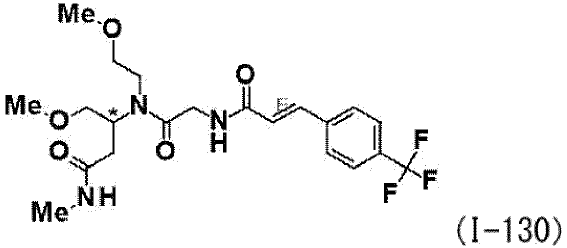

(1) To a solution of methyl 3-[tert-butoxycarbonyl(2-methoxyethyl)amino]-4-methoxybutanoate (127 mg) obtained in Reference Example 4 in THF (4 ml) was added 1 mol/l aqueous sodium hydroxide solution (500 µl), and the reaction mixture was stirred at room temperature for 16 hr. To the reaction mixture was added 1 mol/l hydrochloric acid (500 µl), and the mixture was concentrated under reduced pressure and azeotropically distilled twice with toluene to give 3-[tert-butoxycarbonyl(2-methoxyethyl)amino]-4-methoxybutanoic acid as a crude product.
(2) The crude product of 3-[tert-butoxycarbonyl(2-methoxyethyl)amino]-4-methoxybutanoic acid obtained in the aforementioned (1) was dissolved in DMF (4 ml), and 2 ml was taken therefrom. Monomethylamine (310 µl) and HATU (94.9 mg) were added thereto, and the reaction solution was stirred at room temperature for 40 min. Water was added to the reaction solution, and the aqueous layer was extracted with ethyl acetate. The combined organic layer was dried over sodium sulfate and filtered, and the filtrate was concentrated under reduced pressure to give tert-butyl N-(2-methoxyethyl)-N-[1-(methoxymethyl)-3-(methylamino)-3-oxopropyl]carbamate as a crude product (130 mg).
(3) To the crude product (130 mg) of tert-butyl N-(2-methoxyethyl)-N-[1-(methoxymethyl)-3-(methylamino)-3-oxopropyl]carbamate obtained in the aforementioned (2) was added hydrochloric acid (4 mol/l 1,4-dioxane solution) (2 ml), and the reaction mixture was stirred at room temperature for 80 min. The reaction mixture was concentrated to give 4-methoxy-3-(2-methoxyethylamino)-N-methylbutanamide hydrochloride as a crude product.
(4) The crude product of 4-methoxy-3-(2-methoxyethylamino)-N-methylbutanamide hydrochloride obtained in the aforementioned (3) and 2-[[(E)-3-[4-(trifluoromethyl)phenyl]prop-2-enoyl]amino]acetic acid (57.9 mg) were treated in the same manner as in Example 1 to give the title compound (29.8 mg; total yield 310) as a colorless amorphous.
   MS(ESI)m/z:460.1[M+H]⁺

### Example 131

### Production of (E)-N-[2-[(3R)-4-methyl-3-(5-methyl-1,3,4-oxadiazol-2-yl)piperazin-1-yl]-2-oxoethyl]-3-[4-(trifluoromethyl)phenyl]prop-2-enamide (compound (1-131))

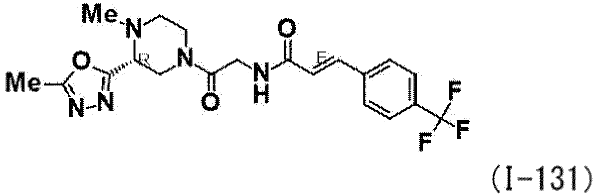

(E)-N-[2-[(3R)-3-(5-methyl-1,3,4-oxadiazol-2-yl)piperazin-1-yl]-2-oxoethyl]-3-[4-(trifluoromethyl)phenyl]prop-2-enamide (compound (1-167)) (103 mg) obtained in the below-mentioned Example 167 was treated in the same manner as in Example 119 (2) to give the title compound (88 mg; yield 92%) as a colorless amorphous. MS(ESI)m/z:438.2[M+H]⁺

### Example 132:

### Production of (2R)-1-methyl-4-[2-[[(E)-3-[4-(trifluoromethyl)phenyl]prop-2-enoyl]amino]acetyl]piperazine-2-carboxylic acid (compound (1-132))

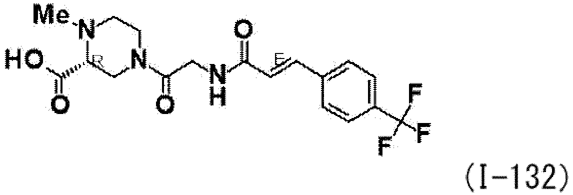

Methyl (2R)-1-methyl-4-[2-[[(E)-3-[4-(trifluoromethyl)phenyl]prop-2-enoyl]amino]acetyl]piperazine-2-carboxylate (compound (1-171)) obtained in the below-mentioned Example 171 was treated in the same manner as in Example 119 (2) to give the title compound (83 mg; yield 210) as a white powder.
MS(ESI)m/z:400.1[M+H]⁺

### Example 133:

### Production of (E)-N-(2-oxo-2-piperazin-1-yl ethyl)-3-[4-(trifluoromethyl)phenyl]prop-2-enamide hydrochloride (compound (I-133))

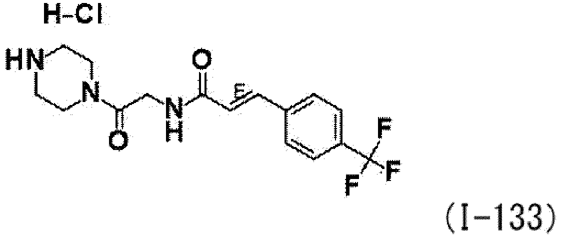

To a solution of tert-butyl 4-[2-[[(E)-3-[4-(trifluoromethyl)phenyl]prop-2-enoyl]amino]acetyl]piperazine-1-carboxylate (compound (1-160)) (600 mg) obtained in the below-mentioned Example 160 in methanol (5 ml) was added hydrochloric acid (4 mol/l 1,4-dioxane solution) (10 ml), and the mixture was stirred at room temperature for 1 hr. The solvent was concentrated under reduced pressure, the obtained residue was washed by suspending in acetonitrile and filtered under reduced pressure. The obtained solid was dried by heating to give the title compound (410 mg; yield 80%) as a white solid. MS(ESI)m/z:342.1[M+H]⁺

### Example 134:

### Production of N-ethyl-4-[2-[[(E)-3-[4-(trifluoromethyl)phenyl]prop-2-enoyl]amino]acetyl]piperazine-1-carboxamide (compound (1-134))

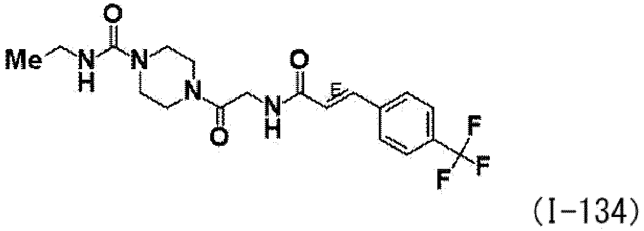

To a solution of (E)-N-(2-oxo-2-piperazin-1-ylethyl)-3-[4-(trifluoromethyl)phenyl]prop-2-enamide hydrochloride (compound (1-133)) (150 mg) obtained in Example 133 in dichloromethane (5 ml) were added triethylamine (0.15 ml) and ethyl isocyanate (0.04 ml), and the mixture was stirred at room temperature for 2 hr. Water was added to the reaction mixture, and the mixture was extracted with chloroform. The organic layer was washed with saturated brine, dried over magnesium sulfate, and concentrated under reduced pressure. The obtained residue was washed by suspending in diisopropyl ether, and filtered under reduced pressure. The obtained solid was dried by heating to give the title compound (137 mg; yield 84%) as a white solid.
MS(ESI)m/z:413.2[M+H]⁺

### Example 135:

### Production of 2-[2-methoxyethyl-[2-[[(E)-3-[4-(trifluoromethyl)phenyl]prop-2-enoyl]amino]acetyl]amino]acetic acid (compound (1-135))

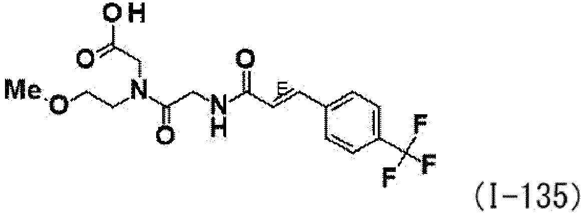

To a solution of ethyl 2-[2-methoxyethyl-[2-[[(E)-3-[4-(trifluoromethyl)phenyl]prop-2-enoyl]amino]acetyl]amino]acetate (compound (1-161)) (350 mg) obtained in the below-mentioned Example 161 in methanol (3 ml) was added 1 mol/l aqueous sodium hydroxide solution (2.5 ml), and the reaction mixture was stirred at room temperature for 1 hr. To the reaction mixture were added 1 mol/l hydrochloric acid (2.5 ml) and water (10 ml) and the mixture was stirred overnight. The precipitated solid was collected by filtration, washed by suspending in diisopropyl ether, and dried under reduced pressure to give the title compound (253 mg; yield 78%) as a white solid. MS(ESI)m/z:389.1[M+H]⁺

### Example 136, 137:

### Production of both enantiomers of (E)-N-[2-oxo-2-[3-oxo-5-phenylpiperazin-1-yl]ethyl]-3-[4-(trifluoromethyl)phenyl]prop-2-enamide (compound (1-136), compound (1-137))

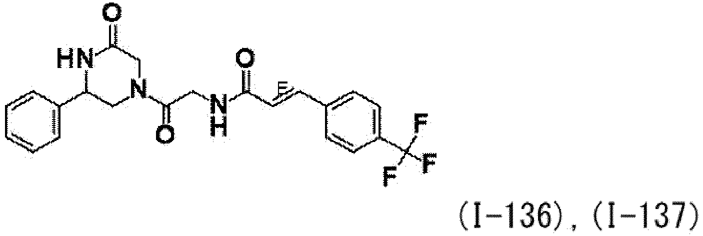

The racemic (E)-N-[2-oxo-2-(3-oxo-5-phenylpiperazin-1-yl)ethyl]-3-[4-(trifluoromethyl)phenyl]prop-2-enamide (compound (I-12)) (166 mg) produced in Example 12 was optically resolved by chiral HPLC to give two enantiomers of the title compound shown in the following Table 7.

**[Table 7]**

| Example No. (compound No.) | instrument analysis data | analysis condition |
|---|---|---|
| 136 (I-136) | MS(ESI) m/z: 432.2 [M+H]⁺ | column: CHIRALPAK IE-3 (4.6 x 150 mm) |
| | | mobile phase: ethanol |
| | | flow rate: 0.5 mL/min |
| | | temperature: 25°C |
| | | analysis channel: PDA 270.0 nm |
| | | retention time (min): 8.990 |
| 137 (enantiomer of compound (I-136)) | MS(ESI) m/z: 432.2 [M+H]⁺ | column: CHIRALPAK IE-3 (4.6 x 150 mm) |
| | | mobile phase: ethanol |
| | | flow rate: 0.5 mL/min |
| (I-137) | | temperature: 25°C |
| | | analysis channel: PDA 270.0 nm |
| | | retention time (min): 12.299 |

### Example 138:

### Production of (2R)-N,N,4-trimethyl-1-[2-[[(E)-3-[4-(trifluoromethyl)phenyl]prop-2-enoyl]amino]acetyl]piperazine-2-carboxamide (compound (1-138))

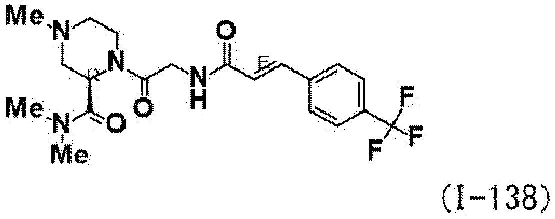

To a solution of (2R)-4-methyl-1-[2-[[(E)-3-[4-(trifluoromethyl)phenyl]prop-2-enoyl]amino]acetyl]piperazine-2-carboxylic acid (compound (1-169)) (30 mg) obtained in the below-mentioned Example 169 in DMF (0.5 ml) were added 9.5 mol/l aqueous dimethylamine solution (12 µl), N,N-diisopropylethylamine (20 µl), and HATU (43 mg), and the mixture was stirred at room temperature for 1 hr. To the reaction mixture were added water and DMSO, and the mixture was purified by reversed-phase HPLC (10 mmol/l aqueous ammonium carbonate solution/acetonitrile=70/30 - 40/60) to give the title compound (17 mg; yield 54%) as a white viscous oil. MS(ESI)m/z:427.3[M+H]⁺

### Example 139:

### Production of (2R)-N,4-dimethyl-1-[2-[[(E)-3-[4-(trifluoromethyl)phenyl]prop-2-enoyl]amino]acetyl]piperazine-2-carboxamide (compound (1-139))

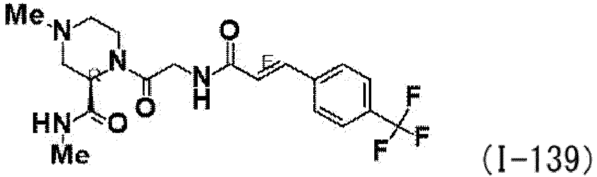

To a solution of (2R)-4-methyl-1-[2-[[(E)-3-[4-(trifluoromethyl)phenyl]prop-2-enoyl]amino]acetyl]piperazine-2-carboxylic acid (compound (1-169)) (30 mg) obtained in the below-mentioned Example 169 in DMF (0.5 ml) were added 9.5 mol/l aqueous dimethylamine solution (10 µl), N,N-diisopropylethylamine (18 µl), and HATU (40 mg), and the mixture was stirred at room temperature for 1 hr. To the reaction mixture were added water and DMSO, and the mixture was purified by reversed-phase HPLC (10 mmol/l aqueous ammonium carbonate solution/acetonitrile=80/20 - 50/50) to give the title compound (18 mg; yield 58%) as a white viscous oil. MS(ESI)m/z:413.2[M+H]⁺

### Example 140:

### Production of 7-[2-[[(E)-3-[4-(trifluoromethyl)phenyl]prop-2-enoyl]amino]acetyl]-6,8-dihydro-5H-imidazo[1,2-a]pyrazine-2-carboxylic acid (compound (1-140))

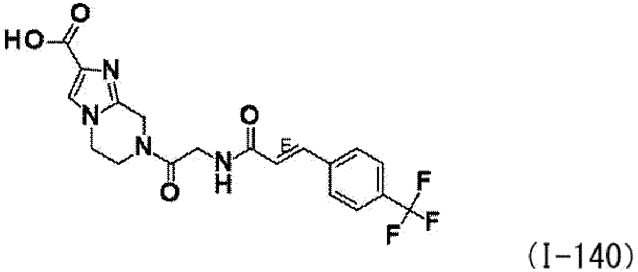

To a solution of ethyl 7-[2-[[(E)-3-[4-(trifluoromethyl)phenyl]prop-2-enoyl]amino]acetyl]-6,8-dihydro-5H-imidazo[1,2-a]pyrazine-2-carboxylate (compound (1-162)) (500 mg) obtained in the below-mentioned Example 162 in methanol (4 ml) was added 1 mol/l aqueous sodium hydroxide solution (1.2 ml), and the reaction mixture was stirred at room temperature for 1 hr. To the reaction mixture was added 1 mol/l hydrochloric acid (1.2 ml), and the precipitated solid was collected by filtration and washed by suspending in diisopropyl ether. The obtained solid was purified by reversed-phase HPLC (solvent:10 mmol/l aqueous ammonium carbonate solution/acetonitrile=90/10 - 60/40). The obtained solid was suspended in 1 mol/l hydrochloric acid, collected by filtration, washed by suspending in diisopropyl ether, and dried under reduced pressure to give the title compound (120 mg; yield 26%) as a white powder.
MS(ESI)m/z:423.2[M+H]⁺

### Example 141:

### Production of (E)-N-[2-[(2R,4R)-4-hydroxy-2-phenylpyrrolidin-1-yl]-2-oxoethyl]-3-[4-(trifluoromethyl)phenyl]prop-2-enamide (compound (1-141))

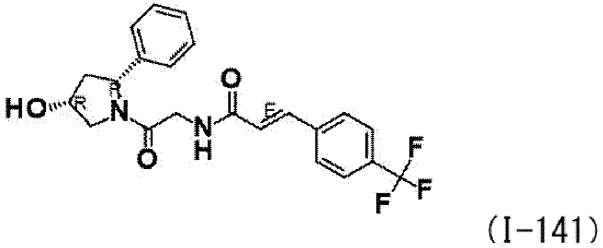

To a solution of the crude product of (E)-N-[2-[(2R,4R)-4-[tert-butyl(dimethyl)silyl]oxy-2-phenylpyrrolidin-1-yl]-2-oxoethyl]-3-[4-(trifluoromethyl)phenyl]prop-2-enamide (compound (1-163)) obtained in the below-mentioned Example 163 in THF (2 ml) was added 1 mol/l tetra-n-butylammonium fluoride-THF solution (0.6 ml), and the mixture was stirred at room temperature for 17 hr. To the reaction mixture was added water, and the mixture was extracted with ethyl acetate. The organic layer was washed with saturated brine and dried over anhydrous magnesium sulfate, and the insoluble material was filtered off. The filtrate was concentrated under reduced pressure, and the obtained residue was purified by silica gel column chromatography (hexane/ethyl acetate=50/50 - 0/100), and the fraction containing the objective compound was concentrated under reduced pressure. The obtained solid was washed with ethyl acetate and filtered to give the title compound (75 mg; yield 30%) as a white solid.
MS(ESI)m/z:419.2[M+H]⁺

### Example 142:

### Production of (E)-N-[2-[(3S)-3-[acetyl(methyl)amino]pyrrolidin-1-yl]-2-oxoethyl]-3-[4-(trifluoromethyl)phenyl]prop-2-enamide (compound (1-142))

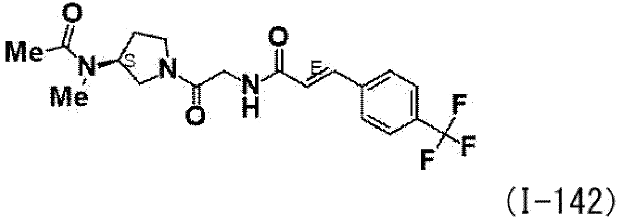

(1) tert-Butyl N-methyl-N-[(3S)-1-[2-[[(E)-3-[4-(trifluoromethyl)phenyl]prop-2-enoyl]amino]acetyl]pyrrolidin-3-yl]carbamate (compound (1-164)) (79 mg) obtained in the below-mentioned Example 164 was dissolved in dichloromethane (3 ml), 2,2,2-trifluoroacetic acid (0.05 ml) was added thereto, and the mixture was stirred at room temperature for 3 hr. The solvent was concentrated under reduced pressure, and the obtained residue was azeotropically distilled with toluene to give a crude product of (E)-N-[2-[(3S)-3-(methylamino)pyrrolidin-1-yl]-2-oxoethyl]-3-[4-(trifluoromethyl)phenyl]prop-2-enamide 2,2,2-trifluoroacetate as a pale-yellow solid.
(2) The crude product of (E)-N-[2-[(3S)-3-(methylamino)pyrrolidin-1-yl]-2-oxo-ethyl]-3-[4-(trifluoromethyl)phenyl]prop-2-enamide 2,2,2-trifluoroacetate obtained in the aforementioned (1) was dissolved in dichloromethane (1 ml), triethylamine (0.07 ml) and acetyl chloride (17 mg) were added thereto, and the mixture was stirred at room temperature for 1 hr. The reaction was discontinued by adding methanol to the reaction mixture, and the solvent was concentrated under reduced pressure. The obtained residue was purified by reversed-phase HPLC (solvent:10 mmol/l aqueous ammonium carbonate solution/acetonitrile=80/20 - 50/50) to give the title compound (62.5 mg; yield 94%) as a colorless viscous oil. MS(ESI)m/z:398.2[M+H]⁺

### Example 143:

### Production of (E)-N-[2-[(3S)-3-acetamidopyrrolidin-1-yl]-2-oxoethyl]-3-[4-(trifluoromethyl)phenyl]prop-2-enamide (compound (I-143))

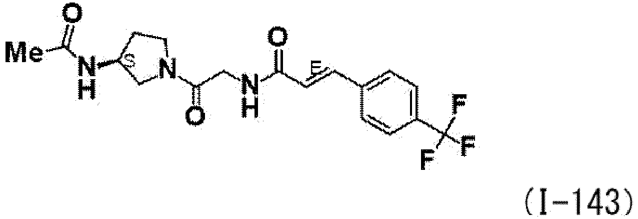

(1) tert-Butyl N-[(3S)-1-[2-[[(E)-3-[4-(trifluoromethyl)phenyl]prop-2-enoyl]amino]acetyl]pyrrolidin-3-yl]carbamate (compound (1-165)) (68 mg) obtained in the below-mentioned Example 165 was dissolved in dichloromethane (3 ml), 2,2,2-trifluoroacetic acid (0.1 ml) was added thereto, and the mixture was stirred at room temperature for 3 hr. The solvent was concentrated under reduced pressure, and the obtained residue was azeotropically distilled with toluene to give a crude product of (E)-N-[2-[(3S)-3-aminopyrrolidin-1-yl]-2-oxoethyl]-3-[4-(trifluoromethyl)phenyl]prop-2-enamide 2,2,2-trifluoroacetate as a pale-yellow solid.
(2) The crude product of (E)-N-[2-[(3S)-3-aminopyrrolidin-1-yl]-2-oxoethyl]-3-[4-(trifluoromethyl)phenyl]prop-2-enamide 2,2,2-trifluoroacetate obtained in the aforementioned (1) was dissolved in dichloromethane (1 ml), triethylamine (0.07 ml) and acetyl chloride (15 mg) were added thereto, and the mixture was stirred at room temperature for 1 hr. The reaction was discontinued by adding methanol to the reaction mixture, and the solvent was concentrated under reduced pressure. The obtained residue was purified by reversed-phase HPLC (solvent:10 mmol/l aqueous ammonium carbonate solution/acetonitrile=80/20 - 50/50) to give the title compound (42 mg; yield 71%) as a colorless solid.
   MS(ESI)m/z:384.2[M+H]⁺

### Example 144:

### Production of (3S)-N-methyl-1-[2-[[(E)-3-[4-(trifluoromethyl)phenyl]prop-2-enoyl]amino]acetyl]pyrrolidine-3-carboxamide (compound (1-144))

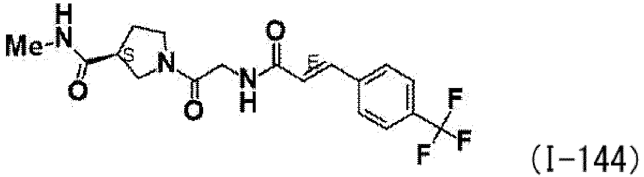

Methyl (3S)-1-[2-[[(E)-3-[4-(trifluoromethyl)phenyl]prop-2-enoyl]amino]acetyl]pyrrolidine-3-carboxylate (compound (I-158)) (42 mg) obtained in the below-mentioned Example 158 was dissolved in methylamine (2 mol/l THF solution) (3 ml), and the mixture was stirred at room temperature for 3 hr. The reaction solution was concentrated under reduced pressure, and the obtained residue was purified by reversed-phase HPLC (solvent:10 mmol/l aqueous ammonium carbonate solution/acetonitrile=70/30 - 40/60) to give the title compound (38 mg; yield 910) as a colorless powder.
MS (ESI) m/z: 384.2 [M+H]⁺

### Example 145:

### Production of (3S)-N,N-dimethyl-1-[2-[[(E)-3-[4-(trifluoromethyl)phenyl]prop-2-enoyl]amino]acetyl]pyrrolidine-3-carboxamide (compound (1-145))

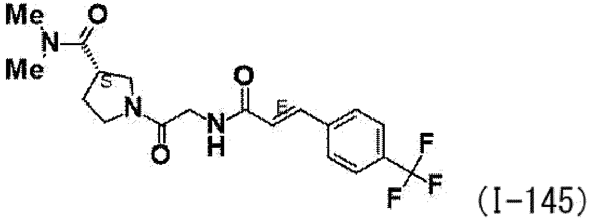

To a solution of (3S)-1-[2-[[(E)-3-[4-(trifluoromethyl)phenyl]prop-2-enoyl]amino]acetyl]pyrrolidine-3-carboxylic acid (compound (1-125)) (40 mg) obtained in Example 125 in DMF (1 ml) were added methylamine hydrochloride (9 mg) and HATU (45 mg). N,N-diisopropylethylamine (0.056 ml) was added dropwise thereto, and the mixture was stirred at room temperature for 1 hr. To the reaction solution was added saturated aqueous ammonium chloride solution, and the mixture was extracted with chloroform. The organic layer was dried over anhydrous sodium sulfate and concentrated under reduced pressure. The obtained residue was purified by reversed-phase HPLC (solvent:10 mmol/l aqueous ammonium carbonate solution/acetonitrile =70/30 - 40/60) to give the title compound (42 mg; yield 98%) as a colorless powder.
MS (ESI) m/z: 398.2[M+H]⁺

### Example 146:

### Production of (3S)-N,N-dimethyl-1-[2-[[(E)-3-[4-(trifluoromethyl)phenyl]prop-2-enoyl]amino]acetyl]pyrrolidine-3-carboxamide (compound (1-146))

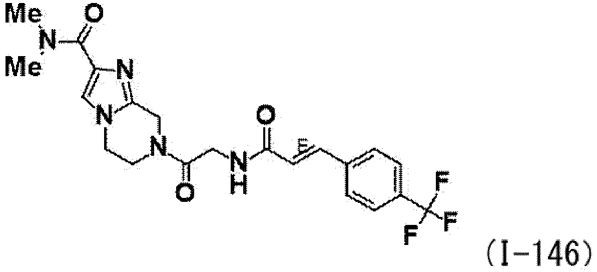

To a solution of 7-[2-[[(E)-3-[4-(trifluoromethyl)phenyl]prop-2-enoyl]amino]acetyl]-6,8-dihydro-5H-imidazo[1,2-a]pyrazine-2-carboxylic acid (compound (1-140)) (50 mg) produced in Example 140 in DMF (0.5 ml) were added 9.5 mol/l aqueous dimethylamine solution (15 µl), N,N-diisopropylethylamine (25 µl), and HATU (68 mg), and the mixture was stirred at room temperature for 1 hr. To the reaction mixture was added 10% aqueous potassium carbonate solution, and the mixture was extracted with a mixed solvent of chloroform:methanol (90:10). The organic layers were combined and dried over anhydrous magnesium sulfate, and the insoluble material was filtered off. The filtrate was concentrated under reduced pressure, and the obtained residue was purified by reversed-phase HPLC (10 mmol/l aqueous ammonium carbonate solution/acetonitrile=70/30 - 40/60) to give the title compound (36 mg; yield 68%) as a white powder.
MS(ESI)m/z:450.2[M+H]+

### Example 147:

### Production of (E)-N-[2-[2-methoxyethyl-[2-[2-methoxyethyl(methyl)amino]-2-oxoethyl]amino]-2-oxoethyl]-3-[4-(trifluoromethyl)phenyl]prop-2-enamide (compound (1-147))

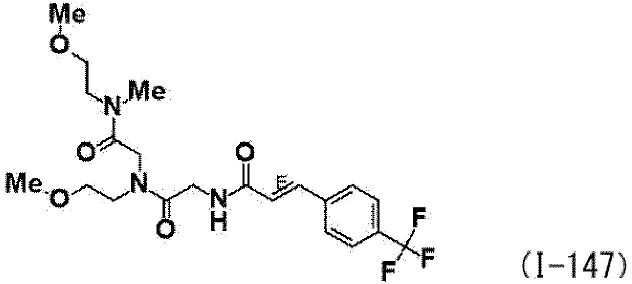

To a solution of 2-[2-methoxyethyl-[2-[[(E)-3-[4-(trifluoromethyl)phenyl]prop-2-enoyl]amino]acetyl]amino]acetic acid (compound (1-135)) (50 mg) produced in Example 135 in DMF (0.6 ml) were added 2-methoxy-N-methylethanamine (18 mg), N,N-diisopropylethylamine (33 µl), and HATU (73 mg), and the reaction mixture was stirred at room temperature for 1 hr. The reaction solution was diluted with water and purified by reversed-phase HPLC (10 mmol/l aqueous ammonium carbonate solution/acetonitrile=70/30 - 40/60) to give the title compound (49 mg; yield 83%) as a colorless viscous oil.
MS(ESI)m/z:460.1[M+H]+

### Example 148:

### Production of (E)-N-[2-[[2-[2-hydroxyethyl(methyl)amino]-2-oxoethyl]-(2-methoxyethyl)amino]-2-oxoethyl]-3-[4-(trifluoromethyl)phenyl]prop-2-enamide (compound (1-148))

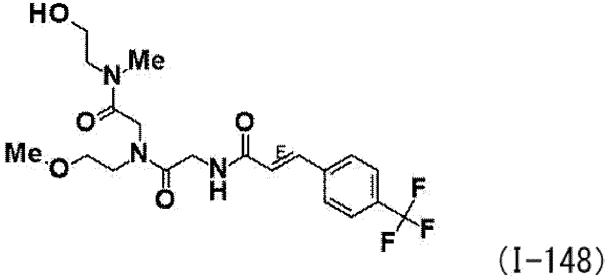

To a solution of 2-[2-methoxyethyl-[2-[[(E)-3-[4-(trifluoromethyl)phenyl]prop-2-enoyl]amino]acetyl]amino]acetic acid (compound (1-135)) (50 mg) produced in Example 135 in DMF (0.6 ml) were added 2-(methylamino)ethanol (15 mg), N,N-diisopropylethylamine (33 µl), and HATU (73 mg), and the reaction mixture was stirred at room temperature for 1 hr. The reaction solution was diluted with water and purified by reversed-phase HPLC (10 mmol/l aqueous ammonium carbonate solution/acetonitrile=80/20 - 50/50) to give the title compound (43 mg; yield 75%) as a colorless viscous oil.
MS(ESI)m/z:446.1[M+H]+

### Example 149:

### Production of (E)-N-[2-[[2-(dimethylamino)-2-oxoethyl]-(2-methoxyethyl)amino]-2-oxoethyl]-3-[4-(trifluoromethyl)phenyl]prop-2-enamide (compound (1-149))

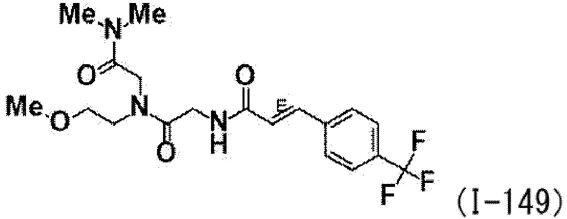

To a solution of 2-[2-methoxyethyl-[2-[[(E)-3-[4-(trifluoromethyl)phenyl]prop-2-enoyl]amino]acetyl]amino]acetic acid (compound (1-135)) (50 mg) produced in Example 135 in DMF (0.6 ml) were added 9.5 mol/l aqueous dimethylamine solution (22 µl), N,N-diisopropylethylamine (33 µl), and HATU (73 mg), and the reaction mixture was stirred at room temperature for 1 hr. The reaction solution was diluted with water and purified by reversed-phase HPLC (10 mmol/l aqueous ammonium carbonate solution/acetonitrile=70/30 - 40/60) to give the title compound (47 mg; yield 88%) as a colorless viscous oil.
MS (ESI)m/z:416.1[M+H] +

### Example 150:

### Production of (2R,4R)-4-hydroxy-N,N-dimethyl-1-[2-[[(E)-3-[4-(trifluoromethyl)phenyl]prop-2-enoyl]amino]acetyl]pyrrolidine-2-carboxamide (compound (1-150))

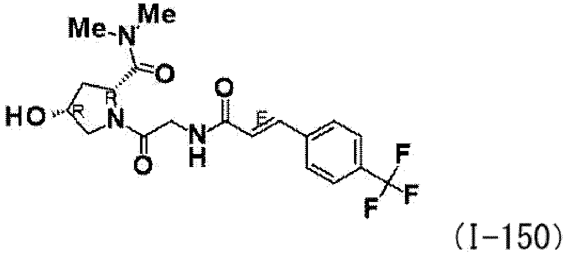

To a solution of (2R,4R)-4-hydroxy-1-[2-[[(E)-3-[4-(trifluoromethyl)phenyl]prop-2-enoyl]amino]acetyl]pyrrolidine-2-carboxylic acid (compound (1-170)) (70 mg) obtained in the below-mentioned Example 170 in DMF (1 ml) were added 9.5 mol/l aqueous dimethylamine solution (21 µl), N,N-diisopropylethylamine (40 µl), and HATU (84 mg), and the reaction mixture was stirred at room temperature for 1 hr. The reaction solution was diluted with water and purified by reversed-phase HPLC (10 mmol/l aqueous ammonium carbonate solution/acetonitrile=80/20 - 50/50) to give the title compound (50 mg; yield 67%) as a white solid.
MS(ESI)m/z:414.2[M+H]+

### Example 151:

### Production of (2R,4R)-4-hydroxy-N-(2-hydroxyethyl)-N-methyl-1-[2-[[(E)-3-[4-(trifluoromethyl)phenyl]prop-2-enoyl]amino]acetyl]pyrrolidine-2-carboxamide (compound (1-151))

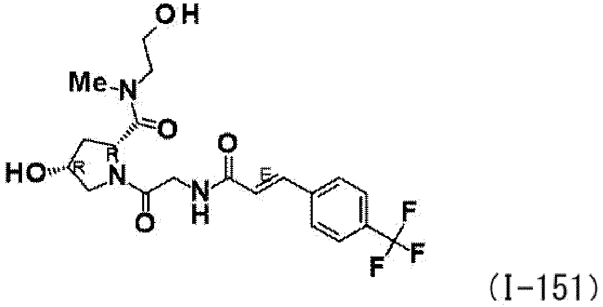

To a solution of (2R,4R)-4-hydroxy-1-[2-[[(E)-3-[4-(trifluoromethyl)phenyl]prop-2-enoyl]amino]acetyl]pyrrolidine-2-carboxylic acid (compound (1-170)) (70 mg) obtained in the below-mentioned Example 170 in DMF (1 ml) were added 2-(methylamino)ethanol (16 µl), N,N-diisopropylethylamine (40 µl), and HATU (84 mg), and the reaction mixture was stirred at room temperature for 1 hr. The reaction solution was diluted with water, and purified by reversed-phase HPLC (10 mmol/l aqueous ammonium carbonate solution/acetonitrile=80/20 - 50/50) to give the title compound (57 mg; yield 71%) as a colorless viscous oil.
MS(ESI)m/z:444.3[M+H]+

### Production of Examples 152 to 166:

The compounds described in the following Table 8-1 to Table 8-3 were obtained by treating the corresponding starting compounds in the same manner as in Example 1.

**[Table 8-1]**

| Example No. (compound No.) | structural formula | instrument analysis data |
|---|---|---|
| 152 (I-152) | 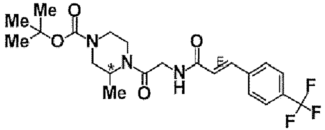 | MS(ESI):456. 3 [M+H]+ |
| 153 (I-153) | 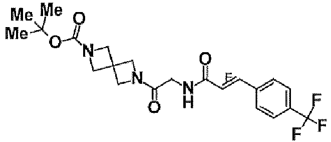 | MS (ESI) : 398. 3 [M+H-tert-Bu]+ |
| 154 (I-154) | 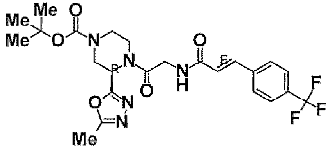 | MS(ESI):524.2 [M+H] + |
| 155 (I-155) | 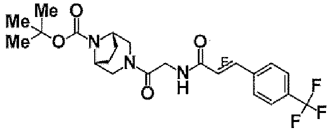 | MS (ESI) : 468. 2 [M+H] + |
| 156 (I-156) | 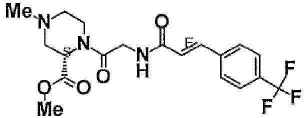 | MS (ESI): 414. 1 [M+H]+ |

**[Table 8-2]**

| Example No. (compound No.) | structural formula | instrument analysis data |
|---|---|---|
| 157 (I-157) | 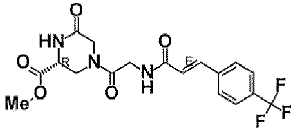 | MS (ESI) : 414.3 [M+H] + |
| 158 (I-158) | 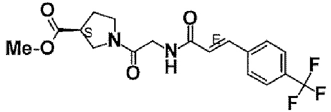 | MS (ESI) :386.0 [M+H] + |
| 159 (I-159) | 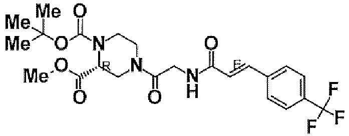 | MS(ESI):500.3 [M+H]+ |
| 160 (I-160) | 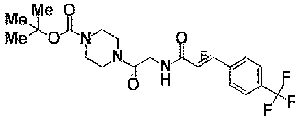 | MS (ESI) :442.3 [M+H]+ |
| 161 (I-161) | 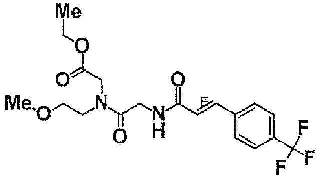 | MS (ESI) :417.2 [M+H]+ |

**[Table 8-3]**

| Example No. (compound No.) | structural formula | instrument analysis data |
|---|---|---|
| 162 (I-162) | 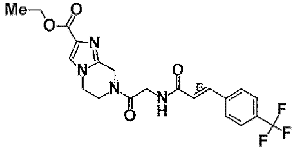 | MS (ESI) :451. 2 [M+H]+ |
| 163 (I-163) | 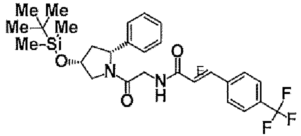 | MS (ESI):533.4[M+H]+ |
| 164 (I-164) | 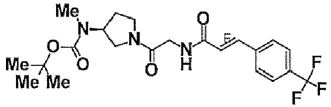 | MS (ESI):456.3[M+H]+ |
| 165 (I-165) | 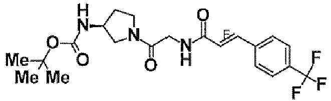 | MS(ESI):442. 2 [M+H]+ |
| 166 (I-166) | 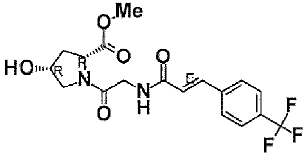 | MS(ESI):401.1[M+H]+ |

### Example 167:

### Production of (E)-N-[2-[(3R)-3-(5-methyl-1,3,4-oxadiazol-2-yl)piperazin-1-yl]-2-oxoethyl]-3-[4-(trifluoromethyl)phenyl]prop-2-enamide (compound (1-167))

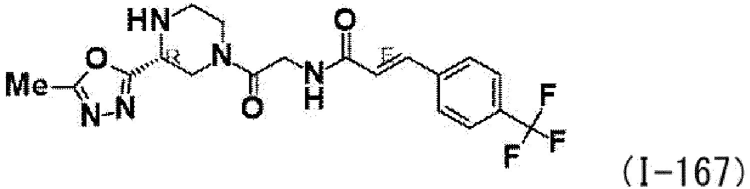

(1) To a solution of tert-butyl (3R)-3-(5-methyl-1,3,4-oxadiazol-2-yl)piperazine-1-carboxylate (150 mg) obtained in Reference Example 1 in dichloromethane (1 ml) was added trifluoroacetic acid (0.1 ml), and the reaction mixture was stirred at room temperature for 1 hr. Trifluoroacetic acid (0.1 ml) was further added thereto, and the reaction mixture was stirred at room temperature overnight, and was stirred at 50°C for 3 hr. The reaction mixture was allowed to cool, and concentrated with adding toluene to give a crude product of 2-methyl-5-[(2R)-piperazin-2-yl]-1,3,4-oxadiazole trifluoroacetate as pale-yellow crystals.
(2) The crude product of 2-methyl-5-[(2R)-piperazin-2-yl]-1,3,4-oxadiazole trifluoroacetate obtained in the aforementioned (1) and 2-[[(E)-3-[4-(trifluoromethyl)phenyl]prop-2-enoyl]amino]acetic acid (100 mg) were treated in the same manner as in Example 1 to give the title compound (103 mg; yield 43%) as a colorless solid. MS(ESI)m/z:424.2[M+H]⁺

### Example 168:

### Production of methyl (2R)-4-methyl-1-[2-[[(E)-3-[4-(trifluoromethyl)phenyl]prop-2-enoyl]amino]acetyl]piperazine-2-carboxylate (compound (1-168))

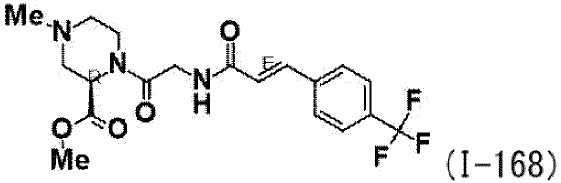

(1) To a solution of O1-tert-butyl O2-methyl (2R)-4-methylpiperazine-1,2-dicarboxylate (1.34 g) in dichloromethane (7 ml) was added 2,2,2-trifluoroacetic acid (3 ml), and the mixture was stirred at room temperature for 3 hr. The reaction solution was concentrated to dryness, and the obtained solid was collected by filtration and dried under reduced pressure at 60°C to give methyl (2R)-4-methylpiperazine-2-carboxylate 2,2,2-trifluoroacetate as a crude product (1.50 g).
(2) The crude product (1.07 g) of methyl (2R)-4-methylpiperazine-2-carboxylate 2,2,2-trifluoroacetate obtained in the aforementioned (1) and 2-[[(E)-3-[4-(trifluoromethyl)phenyl]prop-2-enoyl]amino]acetic acid (1.0 g) were treated in the same manner as in Example 1 to give the title compound (350 mg; yield 23%) as a colorless oil. MS(ESI)m/z:414.6[M+H]⁺

### Example 169:

### Production of (2R)-4-methyl-1-[2-[[(E)-3-[4-(trifluoromethyl)phenyl]prop-2-enoyl]amino]acetyl]piperazine-2-carboxylic acid (compound (1-169))

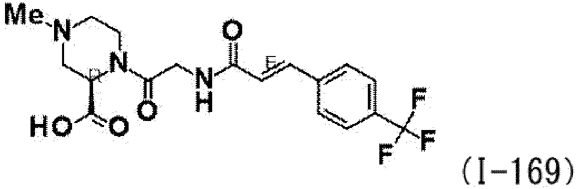

Methyl (2R)-4-methyl-1-[2-[[(E)-3-[4-(trifluoromethyl)phenyl]prop-2-enoyl]amino]acetyl]piperazine-2-carboxylate (compound (I-168)) (350 mg) obtained in Example 168 was treated in the same manner as in Example 123 to give the title compound (75 mg; yield 22%) as a white solid. MS(ESI)m/z:400.1[M+H]⁺

### Example 170:

### Production of (2R,4R)-4-hydroxy-1-[2-[[(E)-3-[4-(trifluoromethyl)phenyl]prop-2-enoyl]amino]acetyl]pyrrolidine-2-carboxylic acid (compound (I-170))

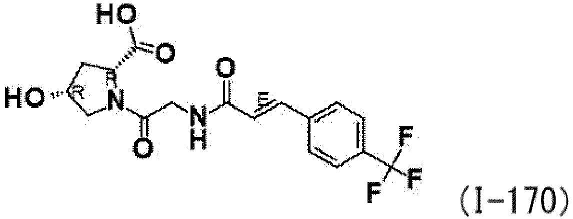

Methyl (2R,4R)-4-hydroxy-1-[2-[[(E)-3-[4-(trifluoromethyl)phenyl]prop-2-enoyl]amino]acetyl]pyrrolidine-2-carboxylate (compound (I-166)) (488 mg) obtained in Example 166 was treated in the same manner as in Example 123 to give the title compound (422 mg; yield 90%) as a white solid. MS(ESI)m/z:387.1[M+H]+

### Example 171:

### Production of methyl (2R)-1-methyl-4-[2-[[(E)-3-[4-(trifluoromethyl)phenyl]prop-2-enoyl]amino]acetyl]piperazine-2-carboxylate (compound (I-171))

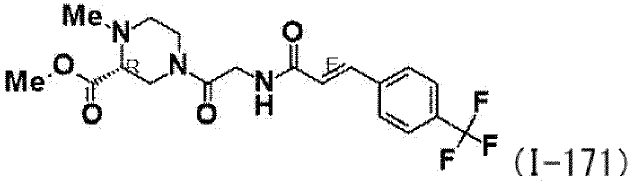

10-tert-Butyl 20-methyl (2R)-4-[2-[[(E)-3-[4-(trifluoromethyl)phenyl]prop-2-enoyl]amino]acetyl]piperazine-1,2-dicarboxylate (compound (I-159)) (416 mg) obtained in Example 159 was treated in the same manner as in Example 119 to give the title compound (416 mg; yield 98%) as a pale-yellow amorphous.
MS(ESI)m/z:414.1[M+H]⁺

### Examples 172 to 260:

The compounds described in the following Table 9-1 to Table 9-18 were obtained by treating the corresponding starting compounds in the same manner as in Example 1.

**[Table 9-1]**

| Example No. (compound No.) | structural formula | instrument analysis data |
|---|---|---|
| 172 (I-172) | 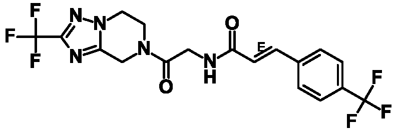 | MS (ESI) :446.2 [M-H]- |
| 173 (I-173) | 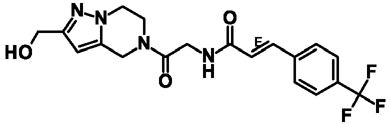 | MS (ESI) :409.2 [M+H]+ |
| 174 (I-174) | 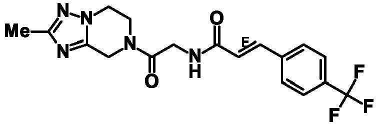 | MS(ESI):394.2 [M+H]+ |
| 175 (I-175) | 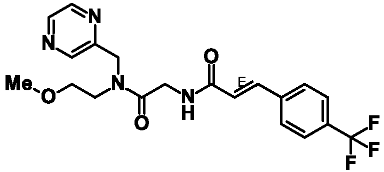 | MS (ESI) :423.5 [M+H]+ |
| 176 (I-176) | 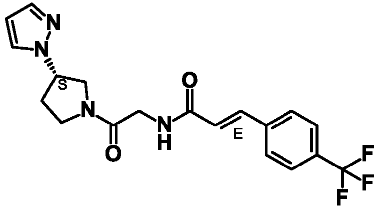 | MS(ESI):393.2 [M+H]+ |

**[Table 9-2]**

| Example No. (compound No.) | structural formula | instrument analysis data |
|---|---|---|
| 177 (I-177) | 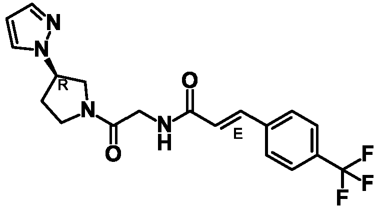 | MS (ESI) :393.2 [M+H]+ |
| 178 (I-178) | 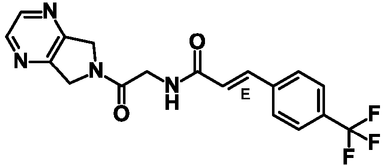 | MS (ESI) :377.1 [M+H]+ |
| 179 (I-179) | 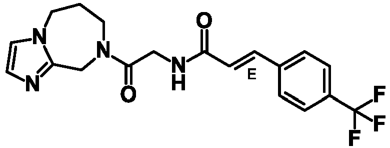 | MS (ESI) :393.2 [M+H]+ |
| 180 (I-180) | 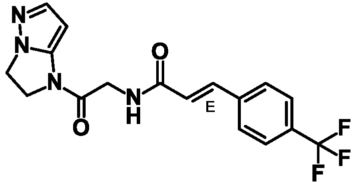 | MS (ESI) :363.0 [M-H]- |
| 181 (I-181) | 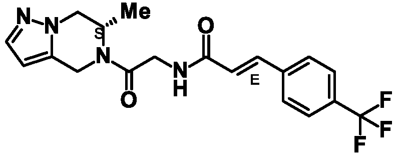 | MS (ESI) :393.2 [M+H]+ |

**[Table 9-3]**

| Example No. (compound No.) | structural formula | instrument analysis data |
|---|---|---|
| 182 (I-182) | 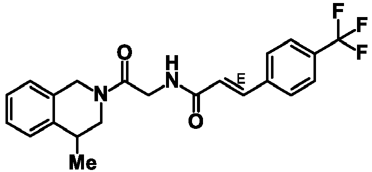 | MS (ESI) :403.3 [M+H]+ |
| 183 (I-183) | 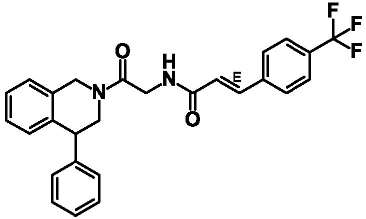 | MS (ESI) :465.3 [M+H]+ |
| 184 (I-184) | 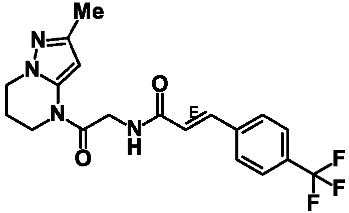 | MS (ESI) :393.3 [M+H]+ |
| 185 (I-185) | 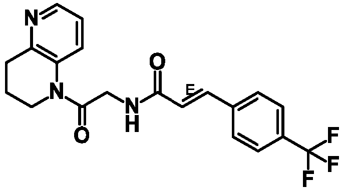 | MS(ESI):390.6 [M+H]+ |
| 186 (I-186) | 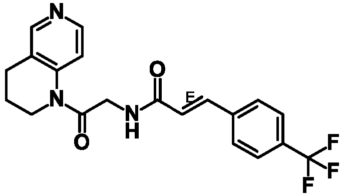 | MS (ESI) :390.2 [M+H]+ |

**[Table 9-4]**

| Example No. (compound No.) | structural formula | instrument analysis data |
|---|---|---|
| 187 (I-187) | 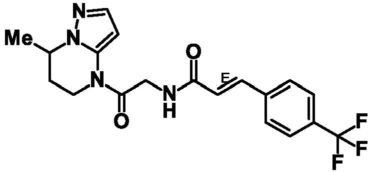 | MS (ESI) : 393.2 [M+H] + |
| 188 (I-188) | 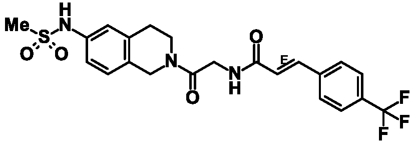 | MS(ESI) : 480.0 [M-H]- |
| 189 (I-189) | 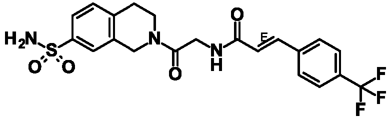 | MS(ESI) : 466.1 [M-H]- |
| 190 (I-190) | 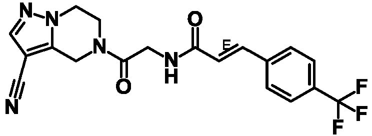 | MS (ESI) : 402.1 [M-H]- |
| 191 (I-191) | 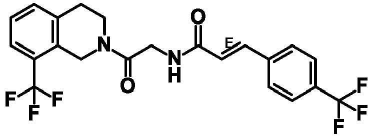 | MS (ESI) : 457.2 [M+H] + |

**[Table 9-5]**

| Example No. (compound No.) | structural formula | instrument analysis data |
|---|---|---|
| 192 (I-192) | 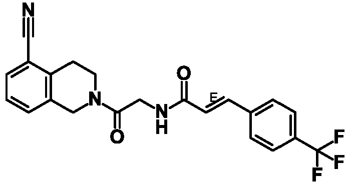 | MS (ESI) : 414.2 [M+H]+ |
| 193 (I-193) | 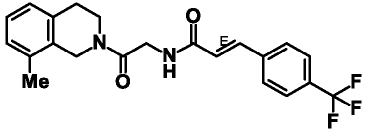 | MS(ESI) : 403.2 [M+H]+ |
| 194 (I-194) | 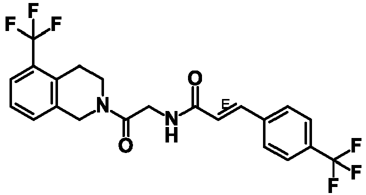 | MS (ESI) : 457.2 [M+H]+ |
| 195 (I-195) | 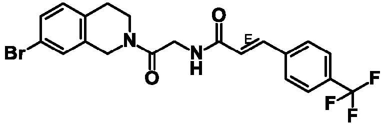 | MS(ESI) : 467.2/ 469.2 [M+H]+ |
| 196 (I-196) | 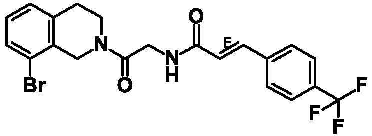 | MS(ESI) : 467.2/ 469.2 [M+H]+ |

**[Table 9-6]**

| Example No. (compound No.) | structural formula | instrument analysis data |
|---|---|---|
| 197 (I-197) | 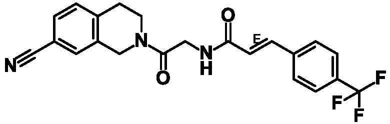 | MS (ESI) : 414.2 [M+H]+ |
| 198 (I-198) | 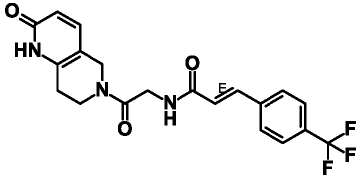 | MS (ESI) : 403.2 [M+H]+ |
| 199 (I-199) | 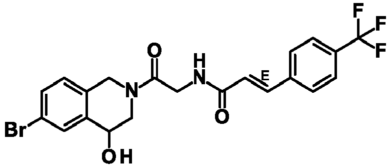 | MS (ESI) : 457.2 [M+H]+ |
| 200 (I-200) | 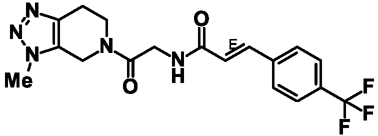 | MS(ESI) : 467.2/ 469.2[M+H]+ |
| 201 (I-201) | 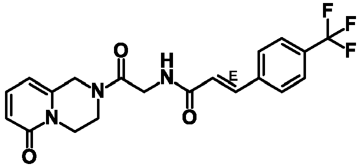 | MS(ESI) : 467.2/ 469.2 [M+H]+ |

**[Table 9-7]**

| Example No. (compound No.) | structural formula | instrument analysis data |
|---|---|---|
| 202 (I-202) | 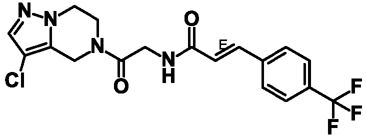 | MS (ESI) : 411.1/ 413.1[M-H]- |
| 203 (I-203) | 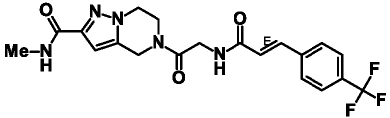 | MS (ESI) : 436.2 [M+H]+ |
| 204 (I-204) | 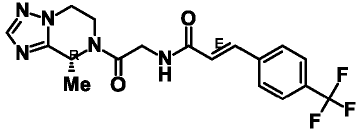 | MS (ESI) : 394.0 [M+H]+ |
| 205 (I-205) | 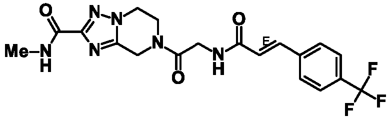 | MS (ESI) : 437.2 [M+H]+ |
| 206 (I-206) | 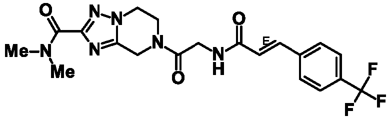 | MS (ESI) : 451.2 [M+H]+ |

**[Table 9-8]**

| Example No. (compound No.) | structural formula | instrument analysis data |
|---|---|---|
| 207 (I-207) | 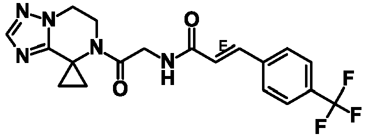 | MS (ESI) : 406.2 [M+H]+ |
| 208 (I-208) | 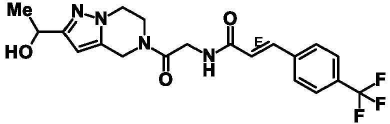 | MS (ESI) : 423.1 [M+H]+ |
| 209 (I-209) | 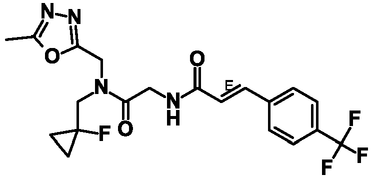 | MS (ESI) : 441.3 [M+H]+ |
| 210 (I-210) | 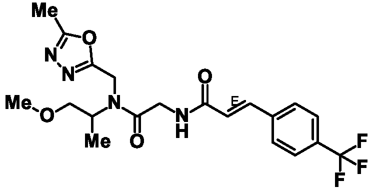 | MS (ESI) : 441.0 [M+H]+ |
| 211 (I-211) | 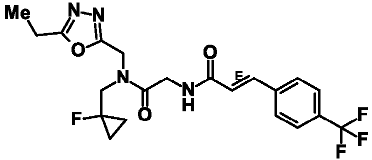 | MS (ESI) : 455.1 [M+H]+ |

**[Table 9-9]**

| Example No. (compound No.) | structural formula | instrument analysis data |
|---|---|---|
| 212 (I-212) | 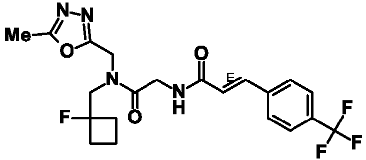 | MS (ESI) : 455.1 [M+H]+ |
| 213 (I-213) | 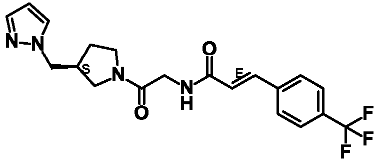 | MS (ESI) : 407.1 [M+H]+ |
| 214 (I-214) | 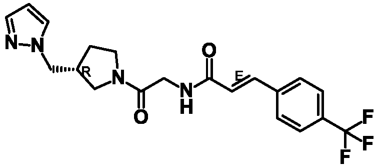 | MS (ESI) : 407.1 [M+H]+ |
| 215 (I-215) | 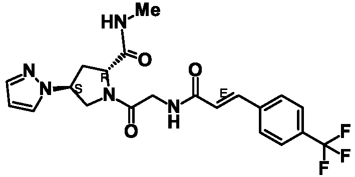 | MS (ESI) : 450.1 [M+H]+ |
| 216 (I-216) | 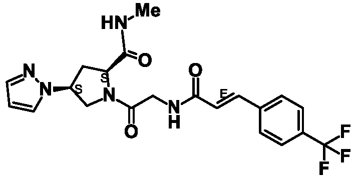 | MS (ESI) : 450.1 [M+H]+ |

**[Table 9-10]**

| Example No. (compound No.) | structural formula | instrument analysis data |
|---|---|---|
| 217 (I-217) | 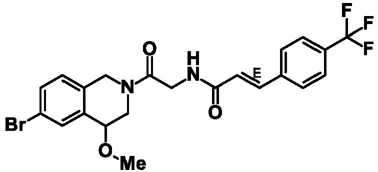 | MS(ESI) : 497.2/ 499.2 [M+H]+ |
| 218 (I-218) | 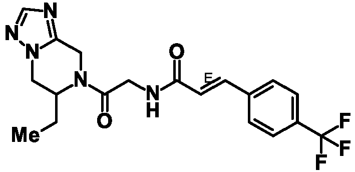 | MS (ESI) : 408.3 [M+H] + |
| 219 (I-219) | 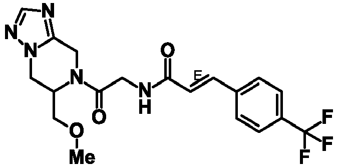 | MS (ESI) : 424.3 [M+H] + |
| 220 (I-220) | 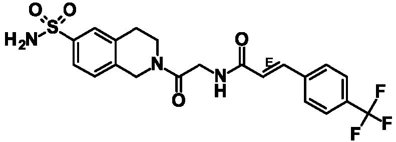 | MS(ESI) : 466.0 [M-H]- |
| 221 (I-221) | 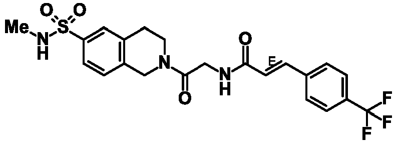 | MS (ESI) : 482.3 [M+H] + |

**[Table 9-11]**

| Example No. (compound No.) | structural formula | instrument analysis data |
|---|---|---|
| 222 (I-222) | 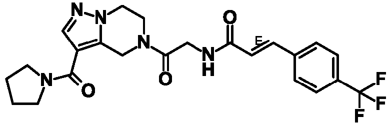 | MS (ESI) : 476.2 [M+H]+ |
| 223 (I-223) | 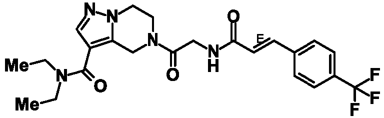 | MS (ESI) : 478.2 [M+H]+ |
| 224 (I-224) | 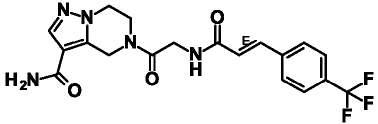 | MS (ESI) : 422.2 [M+H]+ |
| 225 (I-225) | 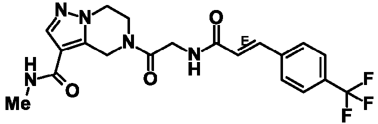 | MS (ESI) : 436.3 [M+H]+ |
| 226 (I-226) | 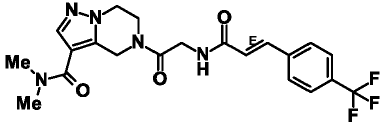 | MS (ESI) : 450.3 [M+H]+ |

**[Table 9-12]**

| Example No. (compound No.) | structural formula | instrument analysis data |
|---|---|---|
| 227 (I-227) | 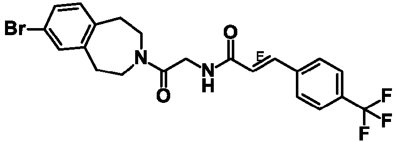 | MS (ESI) : 481.2 [M+H]+ |
| 228 (I-228) | 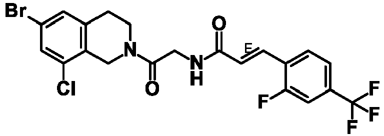 | MS(ESI) : 519.2 [M+H]+ |
| 229 (I-229) | 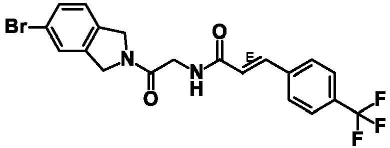 | MS(ESI) : 453.0/ 455.0[M+H]+ |
| 230 (I-230) | 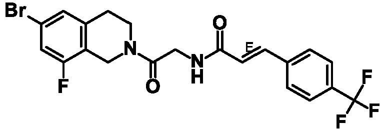 | MS(ESI) : 485.2/ 487.2 [M+H]+ |
| 231 (I-231) | 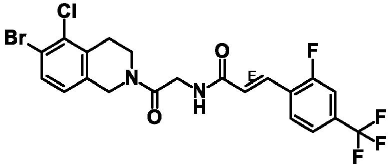 | MS (ESI) : 519.2/ 521.2 [M+H]+ |

**[Table 9-13]**

| Example No. (compound No.) | structural formula | instrument analysis data |
|---|---|---|
| 232 (I-232) | 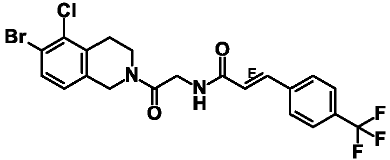 | MS(ESI) : 501.1/ 503.1 [M+H]+ |
| 233 (I-233) | 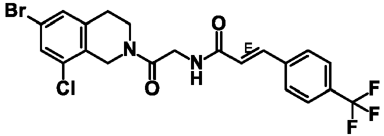 | MS(ESI) : 501.1/ 503.1[M+H]+ |
| 234 (I-234) | 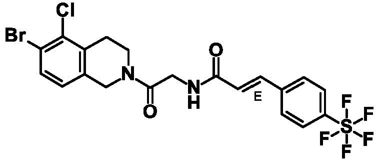 | MS(ESI) : 559.1/ 561.1 [M+H]+ |
| 235 (I-235) | 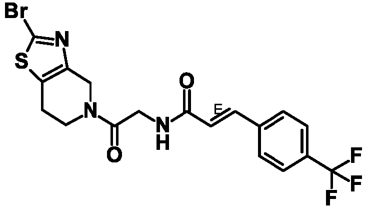 | MS(ESI) : 474.2/ 476.2[M+H]+ |
| 236 (I-236) | 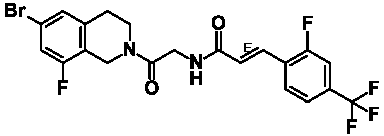 | MS(ESI) : 503.2/ 505.2 [M+H]+ |

**[Table 9-14]**

| Example No. (compound No.) | structural formula | instrument analysis data |
|---|---|---|
| 237 (I-237) | 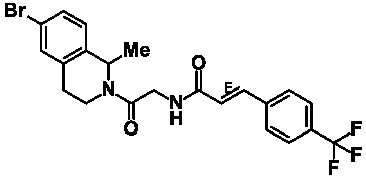 | MS (ESI ) : 481.1/ 483.2 [M+H]+ |
| 238 (I-238) | 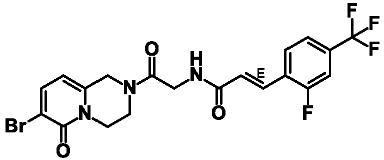 | MS (ESI) : 502.2 [M+H] + |
| 239 (I-239) | 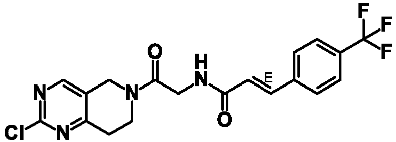 | MS (ESI) : 425.2/ 427.2[M+H]+ |
| 240 (I-240) | 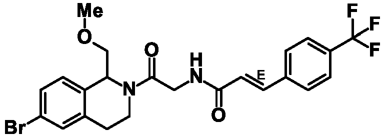 | MS (ESI) : 511.2/ 513.2[M+H]+ |
| 241 (I-241) | 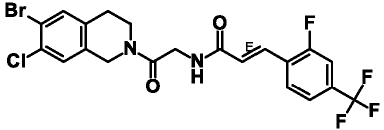 | MS (ESI) : 519.1/ 521.1/523.2 [M+H] + |

**[Table 9-15]**

| Example No. (compound No.) | structural formula | instrument analysis data |
|---|---|---|
| 242 (I-242) | 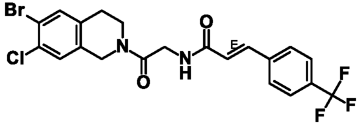 | MS (ESI) : 501.1/ 503.2/505.1 [M+H]+ |
| 243 (I-243) | 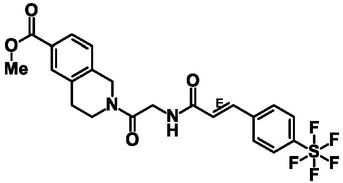 | MS (ESI) : 505.3 [M+H]+ |
| 244 (I-244) | 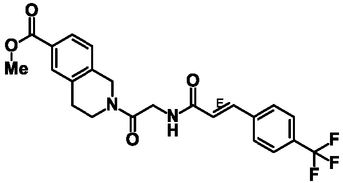 | MS (ESI) : 447.3 [M+H]+ |
| 245 (I-245) | 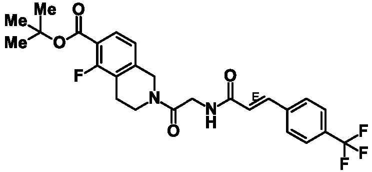 | MS (ESI) : 505.4 [M-H]- |
| 246 (I-246) | 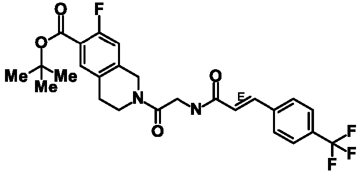 | MS (ESI) : 507.2 [M+H]+ |

**[Table 9-16]**

| Example No. (compound No.) | structural formula | instrument analysis data |
|---|---|---|
| 247 (I-247) | 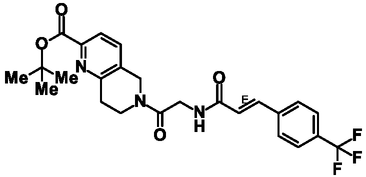 | MS (ESI) : 490.3 [M+H]+ |
| 248 (I-248) | 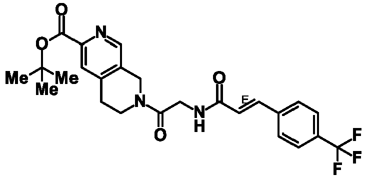 | MS (ESI) : 490.2 [M+H] + |
| 249 (I-249) | 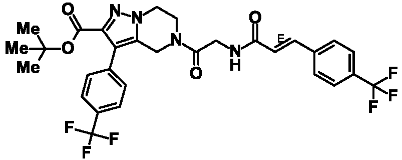 | MS (ESI) : 621.2 [M-H] - |
| 250 (I-250) | 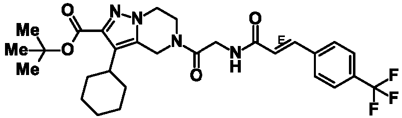 | MS (ESI) : 561.2 [M+H] + |
| 251 (I-251) | 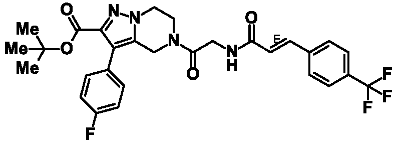 | MS (ESI) : 571.4 [M-H]- |

**[Table 9-17]**

| Example No. (compound No.) | structural formula | instrument analysis data |
|---|---|---|
| 252 (I-252) | 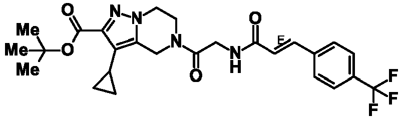 | MS (ESI) : 519.3 [M+H]+ |
| 253 (I-253) | 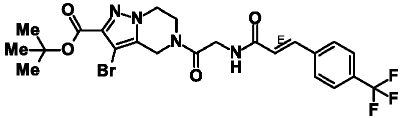 | MS (ESI) : 557.0/ 559.0 [M+H]+ |
| 254 (I-254) | 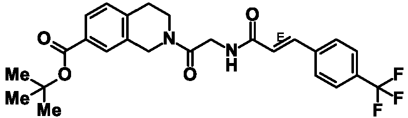 | MS (ESI) : 489.3 [M+H]+ |
| 255 (I-255) | 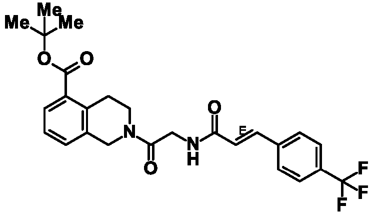 | MS (ESI) : 489.3 [M+H]+ |
| 256 (I-256) | 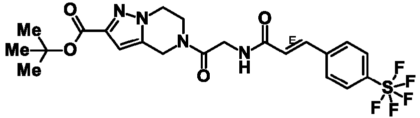 | MS (ESI) : 537.1 [M+H]+ |

**[Table 9-18]**

| Example No. (compound No.) | structural formula | instrument analysis data |
|---|---|---|
| 257 (I-257) | 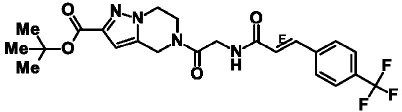 | MS (ESI) : 479.2 [M+H]+ |
| 258 (I-258) | 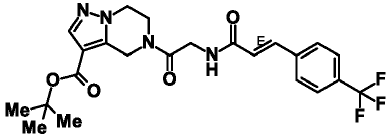 | MS (ESI) : 479.1 [M+H]+ |
| 259 (I-259) | 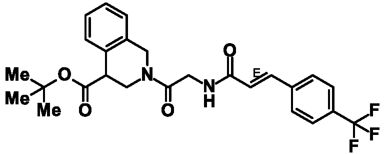 | MS (ESI) : 489.3 [M+H] + |
| 260 (I-260) | 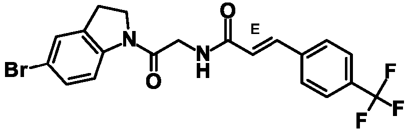 | MS (ESI) : 453.1/ 455.1 [M+H]+ |

### Example 261:

### Production of 5-fluoro-2-[2-[[(E)-3-[4-(trifluoromethyl)phenyl]prop-2-enoyl]amino]acetyl]-3,4-dihydro-1H-isoquinoline-6-carboxylic acid (compound (I-261))

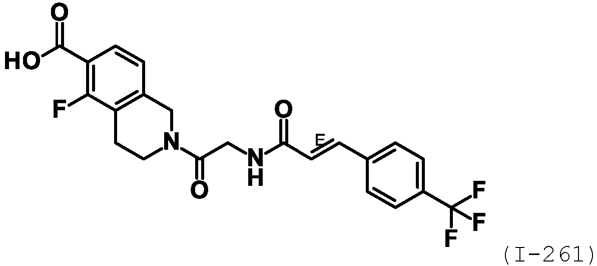

tert-Butyl 5-fluoro-2-[2-[[(E)-3-[4-(trifluoromethyl)phenyl]prop-2-enoyl]amino]acetyl]-3,4-dihydro-1H-isoquinoline-6-carboxylate (139.4 mg) was dissolved in hydrogen chloride-ethyl acetate solution (4 mol/l, 0.6 ml), and the mixture was stirred at room temperature for 3 hr. The reaction mixture was concentrated under reduced pressure, toluene was added thereto, and the mixture was concentrated again under reduced pressure. To the obtained residue was added diisopropyl ether, and the precipitate was collected by filtration and dried under reduced pressure to give the title compound (88.5 mg; yield 71.4%) as a colorless solid. MS (ESI) m/z : 451.2 [M+H]⁺

### Examples 262 to 275:

The compounds described in the following Table 10-1 to Table 10-3 were obtained by treating the corresponding starting compounds in the same manner as in Example 261.

**[Table 10-1]**

| Example No. (compound No.) | structural formula | instrument analysis data |
|---|---|---|
| 262 (I-262) | 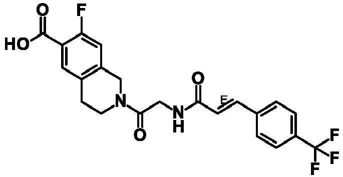 | MS (ESI) : 451.3 [M+H] + |
| 263 (I-263) | 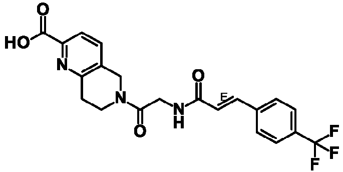 | MS (ESI) : 434.3 [M+H] + |
| 264 (I-264) | 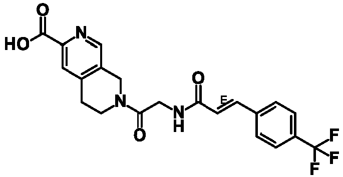 | MS (ESI) : 433.9 [M+H]+ |
| 265 (I-265) | 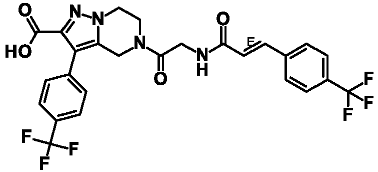 | MS (ESI) : 565.1 [M-H]- |
| 266 (I-266) | 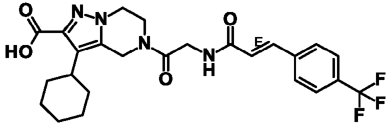 | MS (ESI) : 503.2 [M-H]- |
| 267 (I-267) | 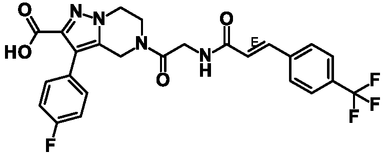 | MS(ESI) : 515.1 [M-H]- |

**[Table 10-2]**

| Example No. (compound No.) | structural formula | instrument analysis data |
|---|---|---|
| 268 (I-268) | 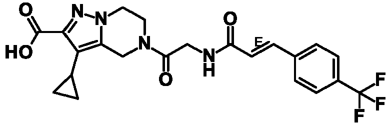 | MS (ESI) : 461.1 [M-H]- |
| 269 (I-269) | 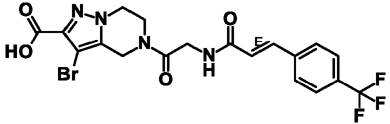 | MS (ESI) : 499.0/ 501.0 [M-H]- |
| 270 (I-270) | 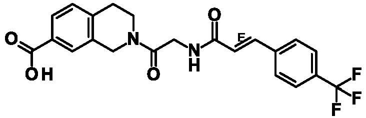 | MS (ESI) : 433.3 [M+H]+ |
| 271 (I-271) | 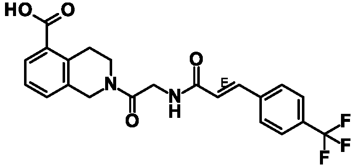 | MS(ESI) : 431.4 [M-H]- |
| 272 (I-272) | 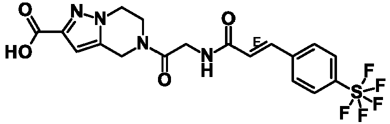 | MS (ESI) : 479.1 [M-H] - |

**[Table 10-3]**

| Example No. (compound No.) | structural formula | instrument analysis data |
|---|---|---|
| 273 (I-273) | 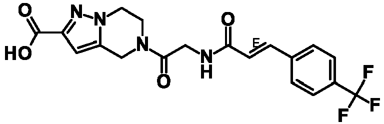 | MS (ESI) : 421.2 [M-H]- |
| 274 (I-274) | 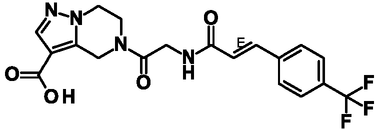 | MS (ESI) : 421.1 [M-H]- |
| 275 (I-275) | 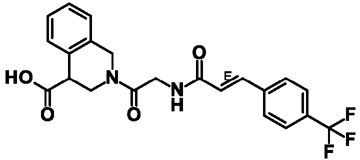 | MS(ESI) : 433.3 [M+H]+ |

### Example 276:

### Production of 2-[2-[[(E)-3-[4-(pentafluoro-λ6-sulfanyl)phenyl]prop-2-enoyl]amino]acetyl]-3,4-dihydro-1H-isoquinoline-6-carboxylic acid (compound (I-276))

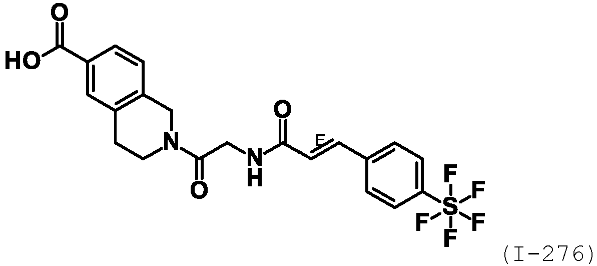

Methyl 2-[2-[[(E)-3-[4-(pentafluoro-λ6-sulfanyl)phenyl]prop-2-enoyl]amino]acetyl]-3,4-dihydro-1H-isoquinoline-6-carboxylate (147 mg) was dissolved in methanol (2 ml) and THF (2 ml) solution, aqueous lithium hydroxide solution (4 mol/l; 0.23 ml) was added thereto, and the mixture was stirred at room temperature for 3 hr. The reaction mixture was neutralized and concentrated under reduced pressure, and the obtained residue was purified by silica gel column chromatography (solvent:hexane/ethyl acetate =100/0 - 0/100) to give the title compound (28.6 mg; yield 22.1%) as a colorless solid.
MS (ESI)m/z : 491.2 [M+H]⁺

### Example 277:

The compound described in the following Table 11-1 was obtained by treating the corresponding starting compound in the same manner as in Example 276.

**[Table 11-1]**

| Example No. (compound No.) | structural formula | instrument analysis data |
|---|---|---|
| 277 (I-277) | 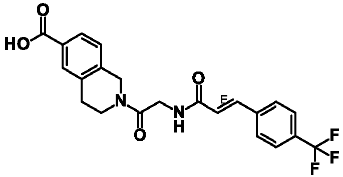 | MS (ESI) : 433.3 [M+H] + |

### Example 278:

### Production of N-(2-methylpropyl)-2-[2-[[(E)-3-[4-(trifluoromethyl)phenyl]prop-2-enoyl]amino]acetyl]-3,4-dihydro-1H-isoquinoline-4-carboxamide (compound (I-278))

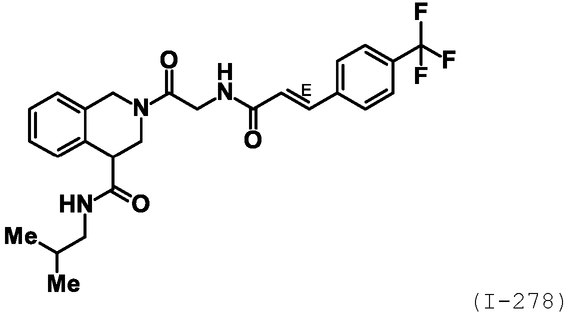

To a solution of N-(2-methylpropyl)-2-[2-[[(E)-3-[4-(trifluoromethyl)phenyl]prop-2-enoyl]amino]acetyl]-3,4-dihydro-1H-isoquinoline-4-carboxylic acid (50 mg) in DMF (1.5 ml) were added N,N-diisopropylethylamine (0.04 ml), HATU (62 mg), and 2-methylpropan-1-amine (17 mg), and the mixture was stirred at room temperature for 3 hr. To the reaction mixture was added water, and the mixture was extracted with ethyl acetate. The organic layer was washed successively with water and saturated brine, and concentrated under reduced pressure. The obtained residue was purified by silica gel column chromatography (solvent:hexane/ethyl acetate =100/0 - 0/100) to give the title compound (48.3 mg; yield 85.7%) as a colorless solid. MS (ESI)m/z : 488.4 [M+H]⁺

### Examples 279 to 281:

The compounds described in the following Table 12-1 were obtained by treating the corresponding starting compounds in the same manner as in Example 278.

**[Table 12-1]**

| Example No. (compound No.) | structural formula | instrument analysis data |
|---|---|---|
| 279 (I-279) | 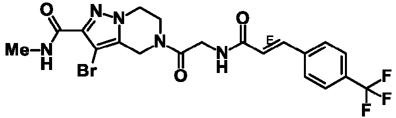 | MS(ESI):514.2/ 516.2 [M+H]+ |
| 280 (I-280) | 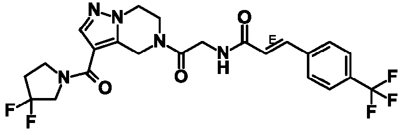 | MS(ESI):512.3 [M+H]+ |
| 281 (I-281) | 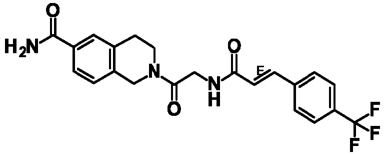 | MS (ESI) :432.3 [M+H]+ |

### Example 282:

### Production of (E)-N-[2-[2-(difluoromethyl)-6,7-dihydro-4H-pyrazolo[1,5-a]pyrazin-5-yl]-2-oxoethyl]-3-[4-(trifluoromethyl)phenyl]prop-2-enamide (compound (I-282))

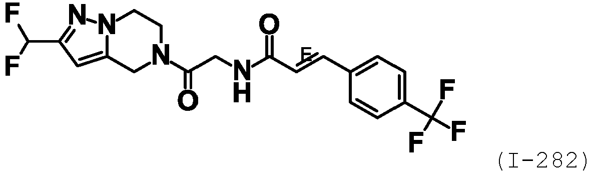

To a solution of (E)-N-[2-[2-(hydroxymethyl)-6,7-dihydro-4H-pyrazolo[1,5-a]pyrazin-5-yl]-2-oxo-ethyl]-3-[4-(trifluoromethyl)phenyl]prop-2-enamide (200 mg) obtained in Example 173 in dichloromethane (5 ml) was added Dess-Martin periodinane (312 mg), and the mixture was stirred at room temperature for 2 hr. The reaction mixture was partitioned by adding saturated aqueous sodium hydrogen carbonate solution, and the organic layer was washed with 10% aqueous sodium carbonate solution and saturated brine, sequentially, dried over sodium sulfate, and filtered. The solvent was evaporated under reduced pressure. To a solution of the obtained crude product in chloroform (5 ml) was added bis(2-methoxyethyl)aminosulfur trifluoride (0.3 ml) under ice-cooling, and the mixture was stirred at room temperature overnight. To the reaction mixture was added water, and the aqueous layer was extracted with ethyl acetate. The organic layer was washed with saturated brine, dried over sodium sulfate, and filtered. The solvent was evaporated under reduced pressure, and the obtained residue was purified by NH silica gel column chromatography (hexane/ethyl acetate=50/50 - 0/100). The fraction containing the objective compound was concentrated under reduced pressure to give the title compound (102 mg; yield 49%) as a white solid.
MS (ESI)m/z : 429.2 [M+H]⁺

### Example 283:

### Production of (6R)-5-[2-[[ (E)-3-[4-(trifluoromethyl)phenyl]prop-2-enoyl]amino]acetyl]-6,7-dihydro-4H-pyrazolo[1,5-a]pyrazine-6-carboxylic acid (compound (I-283))

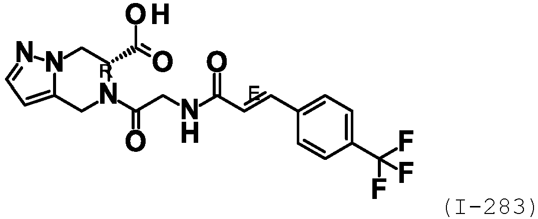

(1) To a solution of 1H-pyrazole-3-carbaldehyde (192 mg) in acetonitrile (10 ml) were added tert-butyl (2R)-3-hydroxy-2-(1H-pyrazol-3-ylmethylamino)propanoate (847 mg) and N,N-diisopropylethylamine (0.35 ml), and acetic acid (0.011 ml) and sodium triacetoxyborohydride (25 mg) were sequentially added, and the mixture was stirred at room temperature overnight. To the reaction mixture were added methanol and saturated aqueous sodium hydrogen carbonate solution, and the solvent was evaporated under reduced pressure. The obtained residue was purified by reversed-phase HPLC (10 mmol/1 aqueous ammonium carbonate solution/acetonitrile=90/10 - 60/40) to give tert-butyl (2R)-3-hydroxy-2-(1H-pyrazol-3-ylmethylamino)propanoate (350 mg; yield 73%) as a pale-yellow amorphous. MS (ESI)m/z : 242.1 [M+H]⁺
(2) To a solution of tert-butyl (2R)-3-hydroxy-2-(1H-pyrazol-3-ylmethylamino)propanoate (345 mg) obtained in (1) in acetonitrile (7.5 ml) were sequentially added benzaldehyde (152 mg), acetic acid (0.01 ml) and sodium triacetoxyborohydride (606 mg), and the mixture was stirred at room temperature overnight. To the reaction mixture were added methanol and saturated aqueous sodium hydrogen carbonate solution, and the solvent was evaporated under reduced pressure. The obtained residue was purified by reversed-phase HPLC (10 mmol/l aqueous ammonium carbonate solution/acetonitrile=60/40 - 30/70) to give tert-butyl (2R)-2-[benzyl(1H-pyrazol-3-ylmethyl)amino]-3-hydroxy-propanoate (364 mg; yield 77%) as a colorless amorphous. MS (ESI)m/z:332.2 [M+H]⁺
(3) To a solution of tert-butyl (2R)-2-[benzyl(1H-pyrazol-3-ylmethyl)amino]-3-hydroxy-propanoate (360 mg) obtained in (2) in dioxane (6 ml) was added cyanomethylenetributylphosphorane (393 mg), and the mixture was stirred at external temperature 110°C for 7 hr. To the reaction mixture was added toluene, and the solvent was evaporated under reduced pressure. The obtained residue was purified by reversed-phase HPLC (10 mmol/l aqueous ammonium carbonate solution/acetonitrile=50/50 - 20/80) to give tert-butyl (6R)-5-benzyl-6,7-dihydro-4H-pyrazolo[1,5-a]pyrazine-6-carboxylate (133 mg; yield 39%) as a yellow amorphous.
   MS (ESI)m/z : 314.2 [M+H]⁺
(4) To a solution of tert-butyl (6R)-5-benzyl-6,7-dihydro-4H-pyrazolo[1,5-a]pyrazine-6-carboxylate (130 mg) obtained in (3) in ethanol (5 ml) was added 10% palladium/carbon (393 mg), and the mixture was stirred under a hydrogen atmosphere at external temperature 60°C for 3 hr. The reaction mixture was purged with nitrogen, chloroform was added thereto, and the mixture was filtered through celite. The filtrate was evaporated under reduced pressure to give tert-butyl (6R)-4,5,6,7-tetrahydropyrazolo[1,5-a]pyrazine-6-carboxylate (85 mg; yield 93%) as a colorless amorphous.
   MS (ESI)m/z : 224.2 [M+H]⁺
(5) To a solution of tert-butyl (6R)-4,5,6,7-tetrahydropyrazolo[1,5-a]pyrazine-6-carboxylate (55 mg) obtained in (4) in dichloromethane (1.2 ml) was added a solution of 1N 2-azidoacetyl chloride in 2-methoxy-2-methylpropane (0.25 ml), and the mixture was stirred at room temperature for 1 hr. To the reaction mixture was added saturated aqueous ammonium chloride solution, and the mixture was extracted with chloroform. The solvent was evaporated under reduced pressure, and the obtained residue was purified by reversed-phase HPLC (10 mmol/l aqueous ammonium carbonate solution/acetonitrile=70/30 - 40/60) to give tert-butyl (6R)-5-(2-azidoacetyl)-6,7-dihydro-4H-pyrazolo[1,5-a]pyrazine-6-carboxylate (72 mg; yield 95%) as a yellow amorphous.
   MS (ESI)m/z : 307.1 [M+H]⁺
(6) To a mixed solution of tert-butyl (6R)-5-(2-azidoacetyl)-6,7-dihydro-4H-pyrazolo[1,5-a]pyrazine-6-carboxylate (72 mg) obtained in (5) in THF (1 ml) and water (0.05 ml) was added triphenyl phosphine (93 mg), and the mixture was stirred at external temperature 50°C for 2 hr. To the reaction mixture was added toluene, and the solvent was evaporated under reduced pressure to give a crude product. To the obtained crude product was added DMF (1.2 ml), and (E)-3-[4-(trifluoromethyl)phenyl]prop-2-enoic acid (51 mg), HATU (98 mg), and N,N-diisopropylethylamine (0.13 ml) were added, and the mixture was stirred at room temperature for 1 hr. The reaction mixture was evaporated under reduced pressure, and the obtained residue was purified by reversed-phase HPLC (10 mmol/l aqueous ammonium carbonate solution/acetonitrile=60/40 - 30/70) to give tert-butyl (6R)-5-[2-[[ (E)-3-[4-(trifluoromethyl)phenyl]prop-2-enoyl]amino]acetyl]-6,7-dihydro-4H-pyrazolo[1,5-a]pyrazine-6-carboxylate (46.3 mg; yield 41%) as a colorless amorphous. MS (ESI)m/z : 479.2 [M+H]⁺
(7) To a solution of tert-butyl (6R)-5-[2-[[ (E)-3-[4-(trifluoromethyl)phenyl]prop-2-enoyl]amino]acetyl]-6,7-dihydro-4H-pyrazolo[1,5-a]pyrazine-6-carboxylate (40 mg) obtained in (6) in dichloromethane (1 ml) was added trifluoroacetic acid (0.1 ml), and the mixture was stirred at room temperature for 2 hr and was stirred at external temperature 50°C for 4 hr. Trifluoroacetic acid (0.1 ml) was added thereto, and the mixture was stirred at external temperature 50°C for 2 hr. Trifluoroacetic acid (0.1 ml) was further added thereto, and the mixture was stirred at external temperature 50°C for 2 hr. Trifluoroacetic acid (0.1 ml) was further added thereto again, and the mixture was stirred at external temperature 50°C for 2 hr. The reaction mixture was evaporated under reduced pressure, toluene was added, and the mixture was evaporated under reduced pressure. The obtained crude product was crystallized by adding diethyl ether, and the crystalline compound was collected by filtration to give the title compound (20 mg; yield 57%) as a colorless amorphous.
   MS (ESI)m/z : 423.0 [M+H]⁺

### Experimental Example 1:

### hB0AT1 inhibitory test

### <Experimental method>

A test compound dissolved in dimethyl sulfoxide (DMSO) was added to a 96 well plate for solid phase radioactivity measurement containing a scintillator at the bottom of the well (final concentration of DMSO 0.5%). DMSO alone as a control and DMSO containing N-(4-bromophenyl)-3,5-dichloro-2-hydroxybenzamide (final concentration 10 µM) for the measurement of B0AT1 non-specific uptake were similarly added at 0.5 µL per well. Human B0AT1 stable expression CHO cells suspended in a buffer (buffer containing 96 mM sodium chloride, 2 mM potassium chloride, 1.8 mM calcium chloride, 1 mM magnesium chloride, 0.01% bovine serum albumin, and 10 mM 2-[4-(2-hydroxyethyl)-1-piperazinyl]ethanesulfonic acid) were added by 90 µL to 75000 cells/well. After standing at ordinary temperature for 30 min or longer, the cells were subjected to a phenylalanine uptake experiment. A buffer containing L-phenylalanine and [3,4,5-3H]-L-phenylalanine was added by 10 µL per well (final concentration of phenylalanine 0.25 mM), and the radioactivity of the plate bottom surface at ordinary temperature was measured with a scintillation counter over time, and the radioactivity value after 100 to 200 min was analyzed. A value obtained by subtracting the B0AT1 non-specific uptake from the control uptake was taken as 100%, and the concentration necessary for each test compound to achieve 50% inhibition (IC₅₀ value) was determined by nonlinear regression using a logistic model.

The results are shown in the following Table 13-1 to Table 13-8.

**[Table 13-1]**

| Example No. | hB0AT1 IC₅₀[nM] |
|---|---|
| I-1 | 107 |
| I-2 | 53 |
| I-3 | 40 |
| I-4 | 285 |
| I-5 | 262 |
| I-6 | 24 |
| I-9 | 1593 |
| I-10 | 803 |
| I-11 | 282 |
| I-12 | 163 |
| I-13 | 111 |
| I-14 | 815 |
| I-15 | 228 |
| I-16 | 1747 |
| I-17 | 195 |
| I-18 | 1659 |
| I-20 | 270 |
| I-21 | 65 |
| I-22 | 214 |
| I-23 | 112 |
| I-24 | 58 |
| I-25 | 81 |
| I-26 | 1435 |
| I-27 | 720 |
| I-28 | 978 |
| I-30 | 1968 |
| I-31 | 106 |
| I-32 | 212 |
| I-33 | 319 |
| I-34 | 21 |
| I-35 | 51 |

**[Table 13-2]**

| Example No. | hB0AT1 IC₅₀[nM] |
|---|---|
| II-36 | 15 |
| I-37 | 91 |
| I-38 | 46 |
| I-39 | 29 |
| I-40 | 29 |
| I-41 | 833 |
| I-42 | 73 |
| I-43 | 80 |
| I-44 | 261 |
| I-45 | 24 |
| I-46 | 181 |
| I-47 | 550 |
| I-48 | 536 |
| I-49 | 775 |
| I-50 | 81 |
| I-51 | 386 |
| I-52 | 56 |
| I-53 | 54 |
| I-54 | 438 |
| I-55 | 122 |
| I-56 | 67 |
| I-57 | 96 |
| I-58 | 353 |
| I-59 | 503 |
| I-60 | 918 |
| I-61 | 81 |
| I-62 | 77 |
| I-63 | 164 |
| I-64 | 195 |
| I-65 | 70 |
| I-66 | 122 |

**[Table 13-3]**

| Example No. | hB0AT1 IC₅₀ [nM] |
|---|---|
| I-67 | 141 |
| I-68 | 58 |
| I-69 | 1468 |
| I-70 | 136 |
| I-71 | 1335 |
| I-72 | 317 |
| I-73 | 228 |
| I-74 | 1308 |
| I-75 | 99 |
| I-76 | 53 |
| I-77 | 70 |
| I-78 | 56 |
| I-79 | 51 |
| I-80 | 42 |
| I-81 | 53 |
| I-82 | 64 |
| I-83 | 78 |
| I-84 | 54 |
| I-85 | 186 |
| I-86 | 23 |
| I-87 | 124 |
| I-89 | 94 |
| I-90 | 23 |
| I-91 | 36 |
| I-92 | 20 |
| I-93 | 31 |
| I-94 | 40 |
| I-95 | 39 |
| I-96 | 10 |
| I-97 | 397 |
| I-98 | 78 |

**[Table 13-4]**

| Example No. | hB0AT1 IC₅₀[nM] |
|---|---|
| I-99 | 64 |
| I-100 | 79 |
| I-103 | 31 |
| I-104 | 2026 |
| I-106 | 276 |
| I-107 | 1161 |
| I-108 | 104 |
| I-109 | 267 |
| I-110 | 98 |
| I-111 | 471 |
| I-112 | 56 |
| I-113 | 178 |
| I-114 | 925 |
| I-116 | 39 |
| I-117 | 199 |
| I-118 | 137 |
| I-119 | 30 |
| I-121 | 81 |
| I-122 | 640 |
| I-123 | 175 |
| I-125 | 1616 |
| I-126 | 38 |
| I-127 | 88 |
| I-128 | 678 |
| I-129 | 66 |
| I-130 | 486 |
| I-131 | 1931 |
| I-133 | 69 |
| I-134 | 1235 |
| I-136 | 87 |
| I-138 | 406 |

**[Table 13-5]**

| Example No. | HB0AT1 IC₅₀ [nM] |
|---|---|
| I-139 | 133 |
| I-140 | 328 |
| I-141 | 1722 |
| I-143 | 550 |
| I-144 | 261 |
| I-145 | 1695 |
| I-146 | 93 |
| I-147 | 298 |
| I-148 | 259 |
| I-149 | 170 |

**[Table 13-6]**

| compound No. | hB0AT1 IC₅₀ [nM] |
|---|---|
| I-172 | 44 |
| I-173 | 30 |
| I-174 | 48 |
| I-175 | 71 |
| I-176 | 185 |
| I-177 | 87 |
| I-178 | 60 |
| I-179 | 60 |
| I-180 | 20 |
| I-181 | 60 |
| I-182 | 168 |
| I-183 | 80 |
| I-184 | 47 |
| I-185 | 31 |
| I-186 | 10 |
| I-187 | 26 |
| I-188 | 34 |
| I-189 | 35 |

**[Table 13-7]**

| compound No. | hB0AT1 IC₅₀ [nM] |
|---|---|
| I-190 | 21 |
| I-191 | 31 |
| I-192 | 30 |
| I-193 | 50 |
| I-194 | 140 |
| I-195 | 54 |
| I-196 | 17 |
| I-197 | 25 |
| I-198 | 49 |
| I-199 | 101 |
| I-200 | 41 |
| I-201 | 32 |
| I-202 | 22 |
| I-203 | 45 |
| I-204 | 819 |
| I-205 | 88 |
| I-206 | 77 |
| I-207 | 324 |
| I-208 | 50 |
| I-209 | 32 |
| I-210 | 165 |
| I-211 | 61 |
| I-212 | 43 |
| I-213 | 108 |
| I-214 | 728 |
| I-215 | 473 |
| I-216 | 161 |
| I-217 | 114 |
| I-218 | 355 |
| I-219 | 262 |
| I-220 | 43 |
| I-221 | 44 |

**[Table 13-8]**

| compound No. | hB0AT1 IC₅₀ [nM] |
|---|---|
| I-222 | 33 |
| I-223 | 41 |
| I-224 | 44 |
| I-225 | 44 |
| I-226 | 67 |
| I-236 | 34 |
| I-260 | 24 |
| I-261 | 223 |
| I-262 | 435 |
| I-263 | 200 |
| I-264 | 55 |
| I-265 | 66 |
| I-266 | 133 |
| I-267 | 83 |
| I-268 | 79 |
| I-269 | 145 |
| I-270 | 168 |
| I-271 | 699 |
| I-272 | 141 |
| I-273 | 125 |
| I-274 | 531 |
| I-276 | 87 |
| I-277 | 143 |
| I-278 | 87 |
| I-279 | 71 |
| I-280 | 49 |
| I-281 | 21 |
| I-282 | 26 |
| I-283 | 361 |

The efficacy of the B0AT1 inhibitor found in the present invention in vivo can be measured, for example, as follows.

### Experimental Example 2:

### Mouse urinary phenylalanine excretion evaluation test

### (1) Preparation of compound and phenylalanine administration solution

The test compound was weighed, and 200 µL of 0.5% carboxymethylcellulose sodium salt (Sigma-Aldrich), a saline solution (for intraperitoneal administration), or an aqueous solution (for oral administration) was added. Using a mixer mill (Retsch), the mixture was pulverized, suspended, and adjusted to the optimum concentration. Thereafter, sonication was performed for 10 min using an ultrasonic disintegrator (Nihon seiki). An administration solution was prepared on the day of test compound administration. L-phenylalanine (Sigma-Aldrich) was dissolved in saline (Otsuka Fresh Food Injection, Otsuka Pharmaceutical Factory, Inc.) by sonication and adjusted to 25 mg/ml. An administration solution was prepared on the day of phenylalanine administration.

### (2) Administration of compound and collection of urine sample

Male C57BL/6J (Charles River Japan, Inc.) weighing 20-30 g (8-11 weeks old) were used as test animals. Until 2 days prior to the test, they were group-reared in PC cages. Two days prior to the test, the general conditions of the test animals were confirmed to be normal, and the body weight was measured. The test animals were kept singly in stainless steel metabolic cages for mouse (Shinano Seisakusho), and fed ad libitum with tap water and feed. In the morning of the day of test compound administration, the body weight of the animals was measured, and assigned to groups using the grouping program GPR3 ver.3.0.2 so that the body weight on the test day and the body weight change from 2 days before the test were homogeneous among the groups by a simulation method. The test compounds were administered intraperitoneally or orally, and the test animals were housed in clean metabolic cages. Thirty minutes after administration of the test compound, phenylalanine was intraperitoneally administered at 500 mg/20 ml/kg. Urine was collected 4 hr, 6 hr, or 24 hr after administration of the test compound, and the urine weight was measured. When urine was collected 4 hr and 6 hr after administration, the mouse was restrained and the urine in the bladder was urinated. Contaminants and urine were separated by centrifugation, and the supernatant was collected. Using urinalysis paper Lifestix (Siemens Healthcare Diagnostics), glucose, occult blood, and leukocyte were measured. The collected urine was stored at - 20°C or below and used for phenylalanine concentration measurement.

### (3) Measurement of urinary phenylalanine by LC-MS/MS

To a urine sample (10 µL) was added 190 µL of IS solution (d5-phenylalanine dissolved in acetonitrile/methanol=7/3 at a concentration of 1 µg/mL) and, after mixing, centrifuged supernatant (3 µL) was analyzed by LC-MS/MS. As a calibration standard, 10 µl of a standard solution containing an IS solution and 180 µl of an IS solution were added to 10 µl of physiological saline, mixed, and centrifuged. The supernatant (3 µl) was analyzed by LC-MS/MS under the following conditions.
<measurement conditions>
MS: Quattro Premier XE
LC: ACQUITY UPLC
column: ACQUITY UPLC HSS C18 Column, 100Å, 1.8 µm, 2.1 mm × 100 mm
mobile phase A: 0.025% aqueous heptafluorobutyric acid solution mobile phase B: acetonitrile
gradient (B%): 2-2-98-98-2 (0.00-0.50-3.50-4.25-5.00 min) column temperature: 50°C
flow rate: 0.40 mL/min

As shown in Tables 13-1 to 13-8, it was confirmed that compound (I) of the present invention or a salt thereof has superior inhibitory activity against B0AT1.

These results suggest that compound (I) of the present invention or a salt thereof exhibits prophylactic and/or therapeutic effects on amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric academia, and the like.

### [INDUSTRIAL APPLICABILITY]

The compound of the present invention (I) or a pharmaceutically acceptable salt thereof has a superior inhibitory activity against B0AT1, and therefore, a pharmaceutical composition containing the compound can be used for the treatment and/or prevention of diseases whose symptoms can be relieved by a B0AT1 inhibitory action, specifically, for example, amino acid metabolism disorders such as phenylketonuria, hypertyrosinemia (types 1-3), hypermethioninemia, maple syrup urine disease, homocystinuria, nonketotic hyperglycinemia, propionic acidemia, methylmalonic acidemia, isovaleric acidemia, and the like. Since these amino acid metabolism disorders are designated intractable diseases that require long-term medical treatments including a very strict dietary therapy (amino acid-restricted diet) for life, the compound (I) of the present invention or a pharmaceutically acceptable salt thereof can provide a novel and effective prophylactic and/or therapeutic drug.

This application is based on a patent application No. 2022-011030 filed in Japan (filing date: January 27, 2022), the contents of which are incorporated in full herein.

## Claims

1. A B0AT1 inhibitor consisting of a compound represented by the formula (I): 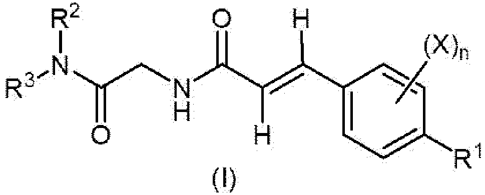 wherein
R¹ is a halogen atom, an optionally substituted C₁₋₆ alkyl group,
an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X in the number of n are each independently a fluorine atom or
a chlorine atom;
n is an integer of 0 to 2; and
R² is a C₁₋₆ alkyl group optionally substituted by substituent(s) selected from substituent group a, and R³ is a C₁₋₆ alkyl group optionally substituted by substituent(s) selected from substituent group a or a C₃₋₆ cycloalkyl group optionally substituted by substituent(s) selected from substituent group b, or R² and R³ are bonded to each other to form, together with a nitrogen atom bonded thereto, a nitrogen-containing non-aromatic heterocyclic group optionally substituted by substituent(s) selected from substituent group b
(substituent group a):
halogen atom;
hydroxy group;
cyano group;
carboxy group;
C₁₋₆ alkoxy group optionally substituted by halogen atom(s);
C₁₋₆ alkylsulfonyl group optionally substituted by halogen atom(s);
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
di-C₁₋₆ alkylamino group;
C₃₋₈ cycloalkyl group optionally substituted by 1 to 3 substituents selected from substituent group c;
C₆₋₁₄ aryl group optionally substituted by 1 to 3 substituents selected from substituent group c;
nitrogen-containing aromatic heterocyclic group optionally substituted by 1 to 3 substituents selected from substituent group c; and
non-aromatic heterocyclic group optionally substituted by 1 to 3 substituents selected from substituent group b
(substituent group b):
halogen atom;
hydroxy group;
cyano group;
carboxy group;
oxo group;
thioxo group;
amino group optionally substituted by 1 or 2 substituents selected from the group consisting of a C₁₋₆ alkyl group, a C₁₋₆ alkyl-carbonyl group, and a C₁₋₆ alkoxy-carbonyl group;
C₁₋₆ alkyl group optionally substituted by substituent(s) selected from the group consisting of a halogen atom, a hydroxy group, and a C₁₋₆ alkoxy group;
C₁₋₆ alkoxy group optionally substituted by halogen atom(s);
C₁₋₆ alkylsulfonyl group optionally substituted by halogen atom(s);
C₁₋₆ alkyl-carbonyl group optionally substituted by halogen atom(s);
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
trisubstituted silyl group;
trisubstituted silyloxy group;
C₃₋₈ cycloalkyl group optionally substituted by 1 to 3 substituents selected from substituent group c;
C₆₋₁₄ aryl group optionally substituted by 1 to 3 substituents selected from substituent group c; and
nitrogen-containing aromatic heterocyclic group optionally substituted by 1 to 3 substituents selected from substituent group c
(substituent group c):
halogen atom;
hydroxy group;
cyano group;
carboxy group;
amino group optionally substituted by 1 or 2 substituents selected from the group consisting of a C₁₋₆ alkyl group and a C₁₋₆ alkoxy-carbonyl group;
C₁₋₆ alkyl group optionally substituted by halogen atom(s);
C₁₋₆ alkoxy group optionally substituted by halogen atom(s);
C₁₋₆ alkylsulfonyl group optionally substituted by halogen atom(s);
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
C₆₋₁₄ aryl group optionally substituted by 1 to 3 substituents selected from the group consisting of a halogen atom, a C₁₋₆ alkyl group, and a C₁₋₆ alkoxy group; and
nitrogen-containing aromatic heterocyclic group optionally substituted by 1 to 3 substituents selected from the group consisting of a halogen atom, a C₁₋₆ alkyl group, and a C₁₋₆ alkoxy group,
or a pharmaceutically acceptable salt thereof.

2. The B0AT1 inhibitor according to claim 1, wherein, in the formula (I),
R¹ is a C₂₋₆ alkyl group, a halo C₁₋₆ alkyl group, a C₃₋₆ cycloalkyl group, a C₂₋₆ alkoxy group, a halo C₂₋₆ alkoxy group,
a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or
6-membered nitrogen-containing aromatic heterocyclic group optionally substituted by a halogen atom or a C₁₋₆ alkyl group,
and
n is 0.

3. The B0AT1 inhibitor according to claim 1 or 2, wherein, in the formula (I), R¹ is a halo C₁₋₄ alkyl group.

4. The B0AT1 inhibitor according to any one of claims 1 to 3, wherein, in the formula (I),
R² and R³ are bonded to each other to form, together with a nitrogen atom bonded thereto, a 3- to 10-membered monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b.

5. The B0AT1 inhibitor according to any one of claims 1 to 3, wherein, in the formula (I),
R² and R³ are bonded to each other to form, together with a nitrogen atom bonded thereto, a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b.

6. The B0AT1 inhibitor according to any one of claims 1 to 3, wherein, in the formula (I),
R² is a C₁₋₄ alkyl group substituted by 1 to 3 substituents selected from the aforementioned substituent group a, and
R³ is a C₁₋₄ alkyl group optionally substituted by 1 to 3 substituents selected from the aforementioned substituent group a.

7. The B0AT1 inhibitor according to any one of claims 1 to 3, wherein, in the formula (I),
R² and R³ are bonded to each other to form, together with a nitrogen atom bonded thereto, a group represented by the following formula: 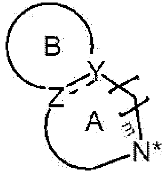
wherein Y and Z are each independently a carbon atom or a nitrogen atom; 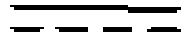
is a single bond or a double bond; ring A is a 5- to 8-membered non-aromatic heterocycle; ring B is a 5- or 6-membered, non-aromatic heterocycle or aromatic heterocycle, or a benzene ring; m is an integer of 0 to 3; and * is a binding site with the carbonyl group.

8. A pharmaceutical composition comprising the B0AT1 inhibitor according to any one of claims 1 to 7, and a pharmaceutically acceptable carrier, for preventing and/or treating diseases whose symptoms can be alleviated by a B0AT1 inhibitory action.

9. The pharmaceutical composition according to claim 8, wherein the disease whose symptoms can be alleviated by the B0AT1 inhibitory action is an amino acid metabolism disorder.

10. The pharmaceutical composition according to claim 9, wherein the amino acid metabolism disorder is phenylketonuria, hypertyrosinemia (types 1-3), hypermethioninemia, maple syrup urine disease, homocystinuria, nonketotic hyperglycinemia, propionic acidemia, methylmalonic academia, or isovaleric acidemia.

11. The pharmaceutical composition according to claim 9, wherein the amino acid metabolism disorder is phenylketonuria.

12. A compound represented by the formula (I'): 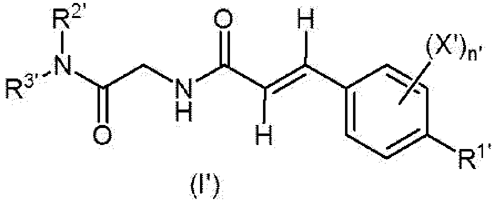 wherein
R¹' is a C₂₋₆ alkyl group, a halo C₁₋₆ alkyl group, a C₃₋₆ cycloalkyl group, a C₂₋₆ alkoxy group, a halo C₂₋₆ alkoxy group,
a C₃₋₆ cycloalkyloxy group, a C₃₋₆ cycloalkyl-C₁₋₄ alkoxy group, a C₂₋₆ alkylsulfanyl group, a halo C₁₋₆ alkylsulfanyl group, a C₃₋₆ cycloalkylsulfanyl group, a pentafluorosulfanyl group, a C₆₋₁₄ aryl group optionally substituted by a halogen atom, or a 5- or
6-membered nitrogen-containing aromatic heterocyclic group optionally substituted by a halogen atom or a C₁₋₆ alkyl group;
X' in the number of n' are each independently a fluorine atom or a chlorine atom;
n' is an integer of 0 to 2; and
R²' is a C₁₋₄ alkyl group substituted by substituent(s) selected from substituent group a, and
R³' is a C₁₋₄ alkyl group optionally substituted by substituent(s) selected from substituent group a or a C₃₋₆ cycloalkyl group optionally substituted by substituent(s) selected from substituent group b, or
R^{2'} and R^{3'} are bonded to each other to form, together with a nitrogen atom bonded thereto, a nitrogen-containing non-aromatic heterocyclic group optionally substituted by substituent(s) selected from substituent group b
(substituent group a):
halogen atom;
hydroxy group;
cyano group;
carboxy group;
C₁₋₆ alkoxy group optionally substituted by halogen atom(s);
C₁₋₆ alkylsulfonyl group optionally substituted by halogen atom(s) ;
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
di-C₁₋₆ alkylamino group;
C₃₋₈ cycloalkyl group optionally substituted by 1 to 3 substituents selected from substituent group c;
C₆₋₁₄ aryl group optionally substituted by 1 to 3 substituents selected from substituent group c;
nitrogen-containing aromatic heterocyclic group optionally substituted by 1 to 3 substituents selected from substituent group c; and
non-aromatic heterocyclic group optionally substituted by 1 to 3 substituents selected from substituent group b
(substituent group b):
halogen atom;
hydroxy group;
cyano group;
carboxy group;
oxo group;
thioxo group;
amino group optionally substituted by 1 or 2 substituents selected from the group consisting of a C₁₋₆ alkyl group, a C₁₋₆ alkyl-carbonyl group, and a C₁₋₆ alkoxy-carbonyl group;
C₁₋₆ alkyl group optionally substituted by substituent(s) selected from the group consisting of a halogen atom, a hydroxy group, and a C₁₋₆ alkoxy group;
C₁₋₆ alkoxy group optionally substituted by halogen atom(s);
C₁₋₆ alkylsulfonyl group optionally substituted by halogen atom(s);
C₁₋₆ alkyl-carbonyl group optionally substituted by halogen atom(s);
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
trisubstituted silyl group;
trisubstituted silyloxy group;
C₃₋₈ cycloalkyl group optionally substituted by 1 to 3 substituents selected from substituent group c;
C₆₋₁₄ aryl group optionally substituted by 1 to 3 substituents selected from substituent group c; and
nitrogen-containing aromatic heterocyclic group optionally substituted by 1 to 3 substituents selected from substituent group c
(substituent group c):
halogen atom;
hydroxy group;
cyano group;
carboxy group;
amino group optionally substituted by 1 or 2 substituents selected from the group consisting of a C₁₋₆ alkyl group and a C₁₋₆ alkoxy-carbonyl group;
C₁₋₆ alkyl group optionally substituted by halogen atom(s);
C₁₋₆ alkoxy group optionally substituted by halogen atom(s);
C₁₋₆ alkylsulfonyl group optionally substituted by halogen atom(s);
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
C₆₋₁₄ aryl group optionally substituted by 1 to 3 substituents selected from the group consisting of a halogen atom, a C₁₋₆ alkyl group, and a C₁₋₆ alkoxy group; and
nitrogen-containing aromatic heterocyclic group optionally substituted by 1 to 3 substituents selected from the group consisting of a halogen atom, a C₁₋₆ alkyl group, and a C₁₋₆ alkoxy group.]
or a salt thereof, excluding compounds represented by the following formulas: 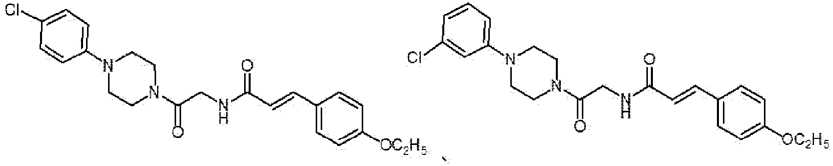 and

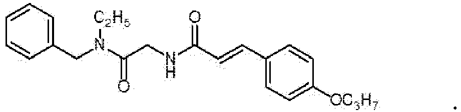

13. The compound according to claim 12, wherein, in the formula (I'), R¹' is a halo C₁₋₄ alkyl group, and n' is 0, or a salt thereof.

14. The compound according to claim 12 or 13, wherein, in the formula (I'),
R²' and R³' are bonded to each other to form, together with a nitrogen atom bonded thereto, a 3- to 10-membered monocyclic nitrogen-containing non-aromatic heterocyclic group, a 6- to 10-membered bridged nitrogen-containing non-aromatic heterocyclic group, a 6- to 12-membered spirocyclic nitrogen-containing non-aromatic heterocyclic group, or a 9- to 14-membered fused nitrogen-containing non-aromatic heterocyclic group, each of which is optionally substituted by 1 to 3 substituents selected from the aforementioned substituent group b, or a salt thereof.

15. The compound according to claim 12 or 13, wherein, in the formula (I'), R²' and R³' are bonded to each other to form, together with a nitrogen atom bonded thereto, a pyrrolidinyl group, a piperidyl group, a piperazinyl group, a morpholinyl group, a thiomorpholinyl group, a 3,8-diazabicyclo[3.2.1]octyl group, a diazepanyl group, a 5,6,7,8-tetrahydro-[1,2,4]triazolo[4,3-a]pyrazinyl group, a 5,6,7,8-tetrahydro-[1,2,4]triazolo[1,5-a]pyrazinyl group, a 4,5,6,7-tetrahydropyrazolo[1,5-a]pyrazinyl group, a 1,2,3,4-tetrahydroisoquinolyl group, a 5,6,7,8-tetrahydro-1,6-naphthyridinyl group, a 1,2,3,4-tetrahydro-2,6-naphthyridinyl group, a 1,2,3,4-tetrahydro-2,7-naphthyridinyl group, or a 2,6-diazaspiro[3.3]heptyl group, each of which is optionally substituted by 1 to 3 substituents selected from the aforementioned substituent group b, or a salt thereof.

16. A compound represented by the formula (I"): 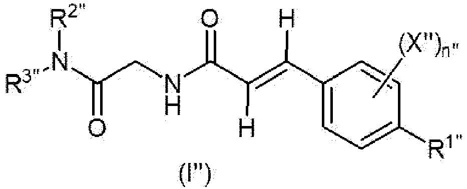 wherein
R¹" is a halogen atom, an optionally substituted C₁₋₆ alkyl group, an optionally substituted C₃₋₈ cycloalkyl group, an optionally substituted C₁₋₆ alkoxy group, an optionally substituted C₃₋₈ cycloalkyloxy group, an optionally substituted C₁₋₆ alkylsulfanyl group, an optionally substituted C₃₋₈ cycloalkylsulfanyl group, a pentafluorosulfanyl group, an optionally substituted C₆₋₁₄ aryl group, or an optionally substituted 5- or 6-membered aromatic heterocyclic group;
X'' in the number of n'' are each independently a fluorine atom or a chlorine atom;
n'' is an integer of 0 to 2; and
R²" is a C₁₋₄ alkyl group substituted by a group selected from the group consisting of
(i) a 5- or 6-membered monocyclic nitrogen-containing aromatic heterocyclic group optionally substituted by substituent(s) selected from substituent group c, and
(ii) a 5- or 6-membered monocyclic nitrogen-containing non-aromatic heterocyclic group optionally substituted by substituent(s) selected from substituent group b, and optionally further substituted by substituent(s) selected from substituent group a, and R³" is a C₁₋₄ alkyl group substituted by substituent(s) selected from substituent group a, or
R^{2"} and R^{3"} are bonded to each other to form, together with a nitrogen atom bonded thereto, a fused nitrogen-containing non-aromatic heterocyclic group (excluding a tetrahydroquinolyl group and a tetrahydroisoquinolyl group) optionally substituted by substituent(s) selected from substituent group b
(substituent group a):
halogen atom;
hydroxy group;
cyano group;
carboxy group;
C₁₋₆ alkoxy group optionally substituted by halogen atom(s);
C₁₋₆ alkylsulfonyl group optionally substituted by halogen atom(s) ;
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
di-C₁₋₆ alkylamino group;
C₃₋₈ cycloalkyl group optionally substituted by 1 to 3 substituents selected from substituent group c;
C₆₋₁₄ aryl group optionally substituted by 1 to 3 substituents selected from substituent group c;
nitrogen-containing aromatic heterocyclic group optionally substituted by 1 to 3 substituents selected from substituent group c; and
non-aromatic heterocyclic group optionally substituted by 1 to 3 substituents selected from substituent group b
(substituent group b):
halogen atom;
hydroxy group;
cyano group;
carboxy group;
oxo group;
thioxo group;
amino group optionally substituted by 1 or 2 substituents selected from the group consisting of a C₁₋₆ alkyl group, a C₁₋₆ alkyl-carbonyl group, and a C₁₋₆ alkoxy-carbonyl group;
C₁₋₆ alkyl group optionally substituted by substituent(s) selected from the group consisting of a halogen atom, a hydroxy group, and a C₁₋₆ alkoxy group;
C₁₋₆ alkoxy group optionally substituted by halogen atom(s);
C₁₋₆ alkylsulfonyl group optionally substituted by halogen atom(s);
C₁₋₆ alkyl-carbonyl group optionally substituted by halogen atom(s);
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
aminosulfonyl group substituted by one substituent selected from the group consisting of a C₁₋₆ alkyl group, a C₃₋₈ cycloalkyl group, and a non-aromatic heterocyclic group, each of which is optionally substituted by 1 to 3 substituents selected from substituent group c;
trisubstituted silyl group;
trisubstituted silyloxy group;
C₃₋₈ cycloalkyl group optionally substituted by 1 to 3 substituents selected from substituent group c;
C₆₋₁₄ aryl group optionally substituted by 1 to 3 substituents selected from substituent group c; and
nitrogen-containing aromatic heterocyclic group optionally substituted by 1 to 3 substituents selected from substituent group c
(substituent group c):
halogen atom;
hydroxy group;
cyano group;
carboxy group;
amino group optionally substituted by 1 or 2 substituents selected from the group consisting of a C₁₋₆ alkyl group and a C₁₋₆ alkoxy-carbonyl group;
C₁₋₆ alkyl group optionally substituted by halogen atom(s);
C₁₋₆ alkoxy group optionally substituted by halogen atom(s);
C₁₋₆ alkylsulfonyl group optionally substituted by halogen atom(s);
C₁₋₆ alkyl-carbonyl group;
C₁₋₆ alkoxy-carbonyl group;
carbamoyl group optionally substituted by 1 or 2 C₁₋₆ alkyl groups optionally substituted by substituent(s) selected from the group consisting of a hydroxy group, a di-C₁₋₆ alkylamino group, and a C₁₋₆ alkoxy group;
C₆₋₁₄ aryl group optionally substituted by 1 to 3 substituents selected from the group consisting of a halogen atom, a C₁₋₆ alkyl group, and a C₁₋₆ alkoxy group; and
nitrogen-containing aromatic heterocyclic group optionally substituted by 1 to 3 substituents selected from the group consisting of a halogen atom, a C₁₋₆ alkyl group, and a C₁₋₆ alkoxy group,
or a salt thereof.

17. The compound according to claim 16, wherein, in the formula (I"),
R²" and R³'' are bonded to each other to form, together with a nitrogen atom bonded thereto, a fused nitrogen-containing non-aromatic heterocyclic group represented by the following formula: 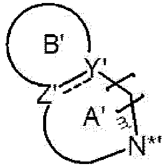
wherein Y' and Z' are each independently a carbon atom or a nitrogen atom; 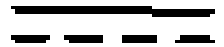
is a single bond or a double bond; ring A' is a 5- to 8-membered non-aromatic heterocycle; ring B' is a 5- or 6-membered, non-aromatic heterocycle or aromatic heterocycle; m' is an integer of 0 to 3; and *' is a binding site with the carbonyl group,
and optionally substituted by 1 to 3 substituents selected from the aforementioned substituent group b, or a salt thereof.

18. A pharmaceutical composition comprising the compound according to any one of claims 12 to 17 or a salt thereof, and a pharmaceutically acceptable carrier.

19. The pharmaceutical composition according to claim 18, which is for use in the treatment and/or prophylaxis of an amino acid metabolism disorder.

20. The pharmaceutical composition according to claim 18, which is for use in the treatment and/or prophylaxis of a disease selected from the group consisting of phenylketonuria, hypertyrosinemia (types 1-3), hypermethioninemia, maple syrup urine disease, homocystinuria, nonketotic hyperglycinemia, propionic acidemia, methylmalonic acidemia, and isovaleric acidemia.

21. The pharmaceutical composition according to claim 18, which is for use in the treatment and/or prophylaxis of phenylketonuria.
